# EUROPEAN PATENT SPECIFICATION

(11) **EP 0 550 687 B1**
(45) Date of publication and mention of the grant of the patent: **09.06.1999**
(21) Application number: 91919358.1
(22) Date of filing: 30.09.1991
(51) Int. Cl.: C12N 15/54, C12N 9/12, C12N 1/21

(54) **5' TO 3' EXONUCLEASE MUTATIONS OF THERMOSTABLE DNA POLYMERASES**
MUTATIONEN IN DER 5'-3'-EXONUKLEASEAKTIVITÄT THERMOSTABILER DNA-POLYMERASEN
MUTATIONS D'ADN-POLYMERASES THERMOSTABLES EN 5' A 3' EXONUCLEASE

(30) Priority: 28.09.1990 US 590213; 28.09.1990 US 590466; 28.09.1990 US 590490
(43) Date of publication of application: 14.07.1993
(60) Divisional application: 98115951.0
(73) Proprietor: F. HOFFMANN-LA ROCHE AG, 4002 Basel (CH)
(72) Inventor: GELFAND, David, H., Oakland, CA 94611 (US); ABRAMSON, Richard, D., Oakland, CA 94618 (US)
(74) Representative: Kolb, Bernd, Dr.
(86) International application number: US9107035
(87) International publication number: WO9206200

(56) References cited:
- WO-A-91/02090
- WO-A-91/09944
- WO-A-91/09950
- The FASEB Journal, volume 5, no. 4, March 1991, (Bethesda, MD, US) R.D. Abramson et al.: "Characterization of the 5'-3'exonuclease activity of thermus aquaticus DNA polymerase", page A437, abstract no. 386, see the abstract
- The Journal of Biological Chemistry, volume 264, no. 11, 15 April 1989, Am. Soc. for Biochemist. and Molecular Biology, Inc. (US) F.C: Lawyer et al.: "Isolation, characterization, and expression in Eschericia coli of the DNA polymerase gene from thermus aquaticus", pages 6427-6437, see figure 2; page 6432, lines 25-30 (cited in the application)
- Cell, volume 59, no. 1, 6 October 1989, Cell Press (MA, US) A. Bernad et al.: "A conserved 3'-5' exonuclease active site in prokarytic and eukarytic DNA polymerases", pages 219-228, see page 224, lines 6-10, line 21 - page 225, line 14; figure 5
- Proc. Natl. Acad. Sci., volume 86, no. 12, June 1989, Biochemistry (US) M.C. Leavitt et al.: "T5 DNA polymerase: structural-functonal relationships to other DNA polymerases", pages 4465-4469, see figure 4; page 4468, lines 13-35
- Chemical Abstracts, volume 93, no. 5, 4 August 1980 (Columbus, Ohio, US) A.S. Kaledin et al.: "Isolation and properties of DNA polymerase from extremal thermophylic bacteria Thermus aquaticus YT-1", see page 377, abstract 40169p, & Biokhimiya (Moscow) 1980, 45(4), 644-51
- Chemical Abstracts, volume 85, no. 21, 22 November 1976, (Columbus, Ohio, US) A. Chien et al.: "Deoxyribonucleic acid polymerase from the extreme thermophile Thermus aquaticus", see page 180, abstract 155559t, & J. Bacteriol. 1976, 127(3), 550-7

### SEQUENCE LISTING

(1) GENERAL INFORMATION:
   (i) APPLICANT: Gelfand, David H.
      Abramson, Richard D.
   (ii) TITLE OF INVENTION: 5' TO 3' EXONUCLEASE MUTATIONS OF THERMOSTABLE DNA POLYMERASES
   (iii) NUMBER OF SEQUENCES: 38
   (iv) CORRESPONDENCE ADDRESS:
      (A) ADDRESSEE: Cetus Corporation
      (B) STREET: 1400 Fifty-third Street
      (C) CITY: Emeryville
      (D) STATE: California
      (F) ZIP: 94608
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: WordPerfect 5.0
   (vi) CURRENT APPLICATION DATA:
      (A) APPLICATION NUMBER: WO
      (B) FILING DATE:
      (C) CLASSIFICATION:
   (vii) PRIOR APPLICATION DATA:
      (A) APPLICATION NUMBER: US 590,490
      (B) FILING DATE: 28-SEP-1990
   (vii) PRIOR APPLICATION DATA:
      (A) APPLICATION NUMBER: US 590,466
      (B) FILING DATE: 28-SEP-1990
   (vii) PRIOR APPLICATION DATA:
      (A) APPLICATION NUMBER: US 590,213
      (B) FILING DATE: 28-SEP-1990
   (vii) PRIOR APPLICATION DATA:
      (A) APPLICATION NUMBER: US 523,394
      (B) FILING DATE: 15-MAY-1990
   (vii) PRIOR APPLICATION DATA:
      (A) APPLICATION NUMBER: US 143,441
      (B) FILING DATE: 12-JAN-1988
   (vii) PRIOR APPLICATION DATA:
      (A) APPLICATION NUMBER: US 063,509
      (B) FILING DATE: 17-JUN-1987
   (vii) PRIOR APPLICATION DATA:
      (A) APPLICATION NUMBER: US 899,241
      (B) FILING DATE: 22-AUC-1986
   (vii) PRIOR APPLICATION DATA:
      (A) APPLICATION NUMBER: US 746,121
      (B) FILING DATE: 15-AUG-1991
   (vii) PRIOR APPLICATION DATA:
      (A) APPLICATION NUMBER: WO PCT/US90/07641
      (B) FILING DATE: 21-DEC-1990
   (vii) PRIOR APPLICATION DATA:
      (A) APPLICATION NUMBER: US 585,471
      (B) FILING DATE: 20-SEP-1990
   (vii) PRIOR APPLICATION DATA:
      (A) APPLICATION NUMBER: US 455,611
      (B) FILING DATE: 22-DEC-1989
   (vii) PRIOR APPLICATION DATA:
      (A) APPLICATION NUMBER: US 609,157
      (B) FILING DATE: 02-NOV-1990
   (vii) PRIOR APPLICATION DATA:
      (A) APPLICATION NUMBER: US 557,517
      (B) FILING DATE: 24-JUL-1990
   (viii) ATTORNEY/AGENT INFORMATION:
      (A) NAME: Sias Ph.D, Stacey R.
      (B) REGISTRATION NUMBER: 32,630
      (C) REFERENCE/DOCKET NUMBER: Case No. 2580
   (ix) TELECOMMUNICATION INFORMATION:
      (A) TELEPHONE: 415-420-3300
(2) INFORMATION FOR SEQ ID NO:1:
   (i) SEQUENCE CHARACTERISTICS:
      (A) LENGTH: 2499 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Thermus aquaticus
   (ix) FEATURE:
      (A) NAME/KEY: CDS
      (B) LOCATION: 1..2496
   (xi) SEQUENCE DESCRIPTION: SEQ ID NO:1:
   ATG AGG GGG ATG CTG CCC CTC TTT GAG CCC AAG GGC CGG GTC CTC CTG 48
   GTG GAC GGC CAC CAC CTG GCC TAC CGC ACC TTC CAC GCC CTG AAG GGC 96
   CTC ACC ACC AGC CGG GGG GAG CCG GTG CAG GCG GTC TAC GGC TTC GCC 144
   AAG AGC CTC CTC AAG GCC CTC AAG GAG GAC GGG GAC GCG GTG ATC GTG 192
   GTC TTT GAC GCC AAG GCC CCC TCC TTC CGC CAC GAG GCC TAC GGG GGG 240
   TAC AAG GCG GGC CGG GCC CCC ACG CCG GAG GAC TTT CCC CGG CAA CTC 288
   GCC CTC ATC AAG GAG CTG GTG GAC CTC CTG GGG CTG GCG CGC CTC GAG 336
   GTC CCG GGC TAC GAG GCG GAC GAC GTC CTG GCC AGC CTG GCC AAG AAG 384
   GCG GAA AAG GAG GGC TAC GAG GTC CGC ATC CTC ACC GCC GAC AAA GAC 432
   CTT TAC CAG CTC CTT TCC GAC CGC ATC CAC GTC CTC CAC CCC GAG GGG 480
   TAC CTC ATC ACC CCG GCC TGG CTT TGG GAA AAG TAC GGC CTG AGG CCC 528
   GAC CAG TGG GCC GAC TAC CGG GCC CTG ACC GGG GAC GAG TCC GAC AAC 576
   CTT CCC GGG GTC AAG GGC ATC GGG GAG AAG ACG GCG AGG AAG CTT CTG 624
   GAG GAG TGG GGG AGC CTG GAA GCC CTC CTC AAG AAC CTG GAC CGG CTG 672
   AAG CCC GCC ATC CGG GAG AAG ATC CTG GCC CAC ATG GAC GAT CTG AAG 720
   CTC TCC TGG GAC CTG GCC AAG GTG CGC ACC GAC CTG CCC CTG GAG GTG 768
   GAC TTC GCC AAA AGG CGG GAG CCC GAC CGG GAG AGG CTT AGG GCC TTT 816
   CTG GAG AGG CTT GAG TTT GGC AGC CTC CTC CAC GAG TTC GGC CTT CTG 864
   GAA AGC CCC AAG GCC CTG GAG GAG GCC CCC TGG CCC CCG CCG GAA GGG 912
   GCC TTC GTG GGC TTT GTG CTT TCC CGC AAG GAG CCC ATG TGG GCC GAT 960
   CTT CTG GCC CTG GCC GCC GCC AGG GGG GGC CGG GTC CAC CGG GCC CCC 1008
   GAG CCT TAT AAA GCC CTC AGG GAC CTG AAG GAG GCG CGG GGG CTT CTC        1056
   GCC AAA GAC CTG AGC GTT CTG GCC CTG AGG GAA GGC CTT GGC CTC CCG        1104
   CCC GGC GAC GAC CCC ATG CTC CTC GCC TAC CTC CTG GAC CCT TCC AAC        1152
   ACC ACC CCC GAG GGG GTG GCC CGG CGC TAC GGC GGG GAG TGG ACG GAG        1200
   GAG GCG GGG GAG CGG GCC GCC CTT TCC GAG AGG CTC TTC GCC AAC CTG        1248
   TGG GGG AGG CTT GAG GGG GAG GAG AGG CTC CTT TGG CTT TAC CGG GAG        1296
   GTG GAG AGG CCC CTT TCC GCT GTC CTG GCC CAC ATG GAG GCC ACG GGG        1344
   GTG CGC CTG GAC GTG GCC TAT CTC AGG GCC TTG TCC CTG GAG GTG GCC        1392
   GAG GAG ATC GCC CGC CTC GAG GCC GAG GTC TTC CGC CTG GCC GGC CAC        1440
   CCC TTC AAC CTC AAC TCC CGG GAC CAG CTG GAA AGG GTC CTC TTT GAC        1488
   GAG CTA GGG CTT CCC GCC ATC GGC AAG ACG GAG AAG ACC GGC AAG CGC        1536
   TCC ACC AGC GCC GCC GTC CTG GAG GCC CTC CGC GAG GCC CAC CCC ATC 1584
   GTG GAG AAG ATC CTG CAG TAC CGG GAG CTC ACC AAG CTG AAG AGC ACC 1632
   TAC ATT GAC CCC TTG CCG GAC CTC ATC CAC CCC AGG ACG GGC CGC CTC 1680
   CAC ACC CGC TTC AAC CAG ACG GCC ACG GCC ACG GGC AGG CTA AGT AGC 1728
   TCC GAT CCC AAC CTC CAG AAC ATC CCC GTC CGC ACC CCG CTT GGG CAG 1776
   AGG ATC CGC CGG GCC TTC ATC GCC GAG GAG GGG TGG CTA TTG GTG GCC 1824
   CTG GAC TAT AGC CAG ATA GAG CTC AGG GTG CTG GCC CAC CTC TCC GGC 1872
   GAC GAG AAC CTG ATC CGG GTC TTC CAG GAG GGG CGG GAC ATC CAC ACG 1920
   GAG ACC GCC AGC TGG ATG TTC GGC GTC CCC CGG GAG GCC GTG GAC CCC 1968
   CTG ATG CGC CGG GCG GCC AAG ACC ATC AAC TTC GGG GTC CTC TAC GGC 2016
   ATG TCG GCC CAC CGC CTC TCC CAG GAG CTA GCC ATC CCT TAC GAG GAG 2064
   GCC CAG GCC TTC ATT GAG CGC TAC TTT CAG AGC TTC CCC AAG GTG CGG        2112
   GCC TGG ATT GAG AAG ACC CTG GAG GAG GGC AGG AGG CGG GGG TAC GTG        2160
   GAG ACC CTC TTC GGC CGC CGC CGC TAC GTG CCA GAC CTA GAG GCC CGG        2208
   GTG AAG AGC GTG CGG GAG GCG GCC GAG CGC ATG GCC TTC AAC ATG CCC        2256
   GTC CAG GGC ACC GCC GCC GAC CTC ATG AAG CTG GCT ATG GTG AAG CTC        2304
   TTC CCC AGG CTG GAG GAA ATG GGG GCC AGG ATG CTC CTT CAG GTC CAC        2352
   GAC GAG CTG GTC CTC GAG GCC CCA AAA GAG AGG GCG GAG GCC GTG GCC        2400
   CGG CTG GCC AAG GAG GTC ATG GAG GGG GTG TAT CCC CTG GCC GTG CCC        2448
   CTG GAG GTG GAG GTG GGG ATA GGG GAG GAC TGG CTC TCC GCC AAG GAG        2496
   TGA                                                                    2499
(2) INFORMATION FOR SEQ ID NO:2:
   (i) SEQUENCE CHARACTERISTICS:
      (A) LENGTH: 832 amino acids
      (B) TYPE: amino acid
      (D) TOPOLOGY: linear
   (ii) MOLECULE TYPE: protein
   (xi) SEQUENCE DESCRIPTION: SEQ ID NO:2:
(2) INFORMATION FOR SEQ ID NO:3:
   (i) SEQUENCE CHARACTERISTICS:
      (A) LENGTH: 2682 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Thermotoga maritima
   (ix) FEATURE:
      (A) NAME/KEY: CDS
      (B) LOCATION: 1..2679
   (xi) SEQUENCE DESCRIPTION: SEQ ID NO:3:
   ATG GCG AGA CTA TTT CTC TTT GAT GGA ACT GCT CTG GCC TAC AGA GCG          48
   Met Ala Arg Leu Phe Leu Phe Asp Gly Thr Ala Leu Ala Tyr Arg Ala
     1               5                  10                  15
   TAC TAT GCG CTC GAT AGA TCG CTT TCT ACT TCC ACC GGC ATT CCC ACA          96
   Tyr Tyr Ala Leu Asp Arg Ser Leu Ser Thr Ser Thr Gly Ile Pro Thr
                    20                  25                  30
   AAC GCC ACA TAC GGT GTG GCG AGG ATG CTG GTG AGA TTC ATC AAA GAC         144
   Asn Ala Thr Tyr Gly Val Ala Arg Met Leu Val Arg Phe Ile Lys Asp
                35                  40                  45
   CAT ATC ATT GTC GGA AAA GAC TAC GTT GCT GTG GCT TTC GAC AAA AAA         192
   His Ile Ile Val Gly Lys Asp Tyr Val Ala Val Ala Phe Asp Lys Lys
        50                  55                  60
   GCT GCC ACC TTC AGA CAC AAG CTC CTC GAG ACT TAC AAG GCT CAA AGA         240
   Ala Ala Thr Phe Arg His Lys Leu Leu Glu Thr Tyr Lys Ala Gln Arg
    65                  70                  75                  80
   CCA AAG ACT CCG GAT CTC CTG ATT CAG CAG CTT CCG TAC ATA AAG AAG         288
   Pro Lys Thr Pro Asp Leu Leu Ile Gln Gln Leu Pro Tyr Ile Lys Lys
                    85                  90                  95
   CTG GTC GAA GCC CTT GGA ATG AAA GTG CTG GAG GTA GAA GGA TAC GAA         336
   Leu Val Glu Ala Leu Gly Met Lys Val Leu Glu Val Glu Gly Tyr Glu
                   100                 105                 110
   GCG GAC GAT ATA ATT GCC ACT CTG GCT GTG AAG GGG CTT CCG CTT TTT      384
   Ala Asp Asp Ile Ile Ala Thr Leu Ala Val Lys Gly Leu Pro Leu Phe
           115                 120                 125
   GAT GAA ATA TTC ATA GTG ACC GGA GAT AAA GAC ATG CTT CAG CTT GTG      432
   Asp Glu Ile Phe Ile Val Thr Gly Asp Lys Asp Met Leu Gln Leu Val
       130                 135                 140
   AAC GAA AAG ATC AAG GTG TGG CGA ATC GTA AAA GGG ATA TCC GAT CTG      480
   Asn Glu Lys Ile Lys Val Trp Arg Ile Val Lys Gly Ile Ser Asp Leu
   145                 150                 155                 160
   GAA CTT TAC GAT GCG CAG AAG GTG AAG GAA AAA TAC GGT GTT GAA CCC      528
   Glu Leu Tyr Asp Ala Gln Lys Val Lys Glu Lys Tyr Gly Val Glu Pro
                   165                 170                 175
   CAG CAG ATC CCG GAT CTT CTG GCT CTA ACC GGA GAT GAA ATA GAC AAC      576
   Gln Gln Ile Pro Asp Leu Leu Ala Leu Thr Gly Asp Glu Ile Asp Asn
               180                 185                 190
   ATC CCC GGT GTA ACT GGG ATA GGT GAA AAG ACT GCT GTT CAG CTT CTA      624
   Ile Pro Gly Val Thr Gly Ile Gly Glu Lys Thr Ala Val Gln Leu Leu
           195                 200                 205
   GAG AAG TAC AAA GAC CTC GAA GAC ATA CTG AAT CAT GTT CGC GAA CTT      672
   Glu Lys Tyr Lys Asp Leu Glu Asp Ile Leu Asn His Val Arg Glu Leu
       210                 215                 220
   CCT CAA AAG GTG AGA AAA GCC CTG CTT CGA GAC AGA GAA AAC GCC ATT      720
   Pro Gln Lys Val Arg Lys Ala Leu Leu Arg Asp Arg Glu Asn Ala Ile
   225                 230                 235                 240
   CTC AGC AAA AAG CTG GCG ATT CTG GAA ACA AAC GTT CCC ATT GAA ATA      768
   Leu Ser Lys Lys Leu Ala Ile Leu Glu Thr Asn Val Pro Ile Glu Ile
                   245                 250                 255
   AAC TGG GAA GAA CTT CGC TAC CAG GGC TAC GAC AGA GAG AAA CTC TTA      816
   Asn Trp Glu Glu Leu Arg Tyr Gln Gly Tyr Asp Arg Glu Lys Leu Leu
               260                 265                 270
   CCA CTT TTG AAA GAA CTG GAA TTC GCA TCC ATC ATG AAG GAA CTT CAA      864
   Pro Leu Leu Lys Glu Leu Glu Phe Ala Ser Ile Met Lys Glu Leu Gln
           275                 280                 285
   CTG TAC GAA GAG TCC GAA CCC GTT GGA TAC AGA ATA GTG AAA GAC CTA 912
   Leu Tyr Glu Glu Ser Glu Pro Val Gly Tyr Arg Ile Val Lys Asp Leu
   290 295 300
   GTG GAA TTT GAA AAA CTC ATA GAG AAA CTG AGA GAA TCC CCT TCG TTC 960
   Val Glu Phe Glu Lys Leu Ile Glu Lys Leu Arg Glu Ser Pro Ser Phe
   305 310 315 320
   GCC ATA GAT CTT GAG ACG TCT TCC CTC GAT CCT TTC GAC TGC GAC ATT 1008
   Ala Ile Asp Leu Glu Thr Ser Ser Leu Asp Pro Phe Asp Cys Asp Ile
   325 330 335
   GTC GGT ATC TCT GTG TCT TTC AAA CCA AAG GAA GCG TAC TAC ATA CCA 1056
   Val Gly Ile Ser Val Ser Phe Lys Pro Lys Glu Ala Tyr Tyr Ile Pro
   340 345 350
   CTC CAT CAT AGA AAC GCC CAG AAC CTG GAC GAA AAA GAG GTT CTG AAA 1104
   Leu His His Arg Asn Ala Gln Asn Leu Asp Glu Lys Glu Val Leu Lys
   355 360 365
   AAG CTC AAA GAA ATT CTG GAG GAC CCC GGA GCA AAG ATC GTT GGT CAG     1152
   Lys Leu Lys Glu Ile Leu Glu Asp Pro Gly Ala Lys Ile Val Gly Gln
       370                 375                 380
   AAT TTG AAA TTC GAT TAC AAG GTG TTG ATG GTG AAG GGT GTT GAA CCT 1200
   Asn Leu Lys Phe Asp Tyr Lys Val Leu Met Val Lys Gly Val Glu Pro
   385 390 395 400
   GTT CCT CCT TAC TTC GAC ACG ATG ATA GCG GCT TAC CTT CTT GAG CCG 1248
   Val Pro Pro Tyr Phe Asp Thr Met Ile Ala Ala Tyr Leu Leu Glu Pro
   405 410 415
   AAC GAA AAG AAG TTC AAT CTG GAC GAT CTC GCA TTG AAA TTT CTT GGA 1296
   Asn Glu Lys Lys Phe Asn Leu Asp Asp Leu Ala Leu Lys Phe Leu Gly
   420 425 430
   TAC AAA ATG ACA TCT TAC CAA GAG CTC ATG TCC TTC TCT TTT CCG CTG 1344
   Tyr Lys Met Thr Ser Tyr Gln Glu Leu Met Ser Phe Ser Phe Pro Leu
   435 440 445
   TTT GGT TTC AGT TTT GCC GAT GTT CCT GTA GAA AAA GCA GCG AAC TAC 1392
   Phe Gly Phe Ser Phe Ala Asp Val Pro Val Glu Lys Ala Ala Asn Tyr
   450 455 460
   TCC TGT GAA GAT GCA GAC ATC ACC TAC AGA CTT TAC AAG ACC CTG AGC 1440
   Ser Cys Glu Asp Ala Asp Ile Thr Tyr Arg Leu Tyr Lys Thr Leu Ser
   465 470 475 480
   TTA AAA CTC CAC GAG GCA GAT CTG GAA AAC GTG TTC TAC AAG ATA GAA 1488
   Leu Lys Leu His Glu Ala Asp Leu Glu Asn Val Phe Tyr Lys Ile Glu
   485 490 495
   ATG CCC CTT GTG AAC GTG CTT GCA CGG ATG GAA CTG AAC GGT GTG TAT 1536
   Met Pro Leu Val Asn Val Leu Ala Arg Met Glu Leu Asn Gly Val Tyr
   500 505 510
   GTG GAC ACA GAG TTC CTG AAG AAA CTC TCA GAA GAG TAC GGA AAA AAA 1584
   Val Asp Thr Glu Phe Leu Lys Lys Leu Ser Glu Glu Tyr Gly Lys Lys
   515 520 525
   CTC GAA GAA CTG GCA GAG GAA ATA TAC AGG ATA GCT GGA GAG CCG TTC 1632
   Leu Glu Glu Leu Ala Glu Glu Ile Tyr Arg Ile Ala Gly Glu Pro Phe
   530 535 540
   AAC ATA AAC TCA CCG AAG CAG GTT TCA AGG ATC CTT TTT GAA AAA CTC 1680
   Asn Ile Asn Ser Pro Lys Gln Val Ser Arg Ile Leu Phe Glu Lys Leu
   545 550 555 560
   GGC ATA AAA CCA CGT GGT AAA ACG ACG AAA ACG GGA GAC TAT TCA ACA 1728
   Gly Ile Lys Pro Arg Gly Lys Thr Thr Lys Thr Gly Asp Tyr Ser Thr
   565 570 575
   CGC ATA GAA GTC CTC GAG GAA CTT GCC GGT GAA CAC GAA ATC ATT CCT 1776
   Arg Ile Glu Val Leu Glu Glu Leu Ala Gly Glu His Glu Ile Ile Pro
   580 585 590
   CTG ATT CTT GAA TAC AGA AAG ATA CAG AAA TTG AAA TCA ACC TAC ATA 1824
   Leu Ile Leu Glu Tyr Arg Lys Ile Gln Lys Leu Lys Ser Thr Tyr Ile
   595 600 605
   GAC GCT CTT CCC AAG ATG GTC AAC CCA AAG ACC GGA AGG ATT CAT GCT 1872
   Asp Ala Leu Pro Lys Met Val Asn Pro Lys Thr Gly Arg Ile His Ala
   610 615 620
   TCT TTC AAT CAA ACG GGG ACT GCC ACT GGA AGA CTT AGC AGC AGC GAT 1920
   Ser Phe Asn Gln Thr Gly Thr Ala Thr Gly Arg Leu Ser Ser Ser Asp
   625 630 635 640
   CCC AAT CTT CAG AAC CTC CCG ACG AAA AGT GAA GAG GGA AAA GAA ATC 1968
   Pro Asn Leu Gln Asn Leu Pro Thr Lys Ser Glu Glu Gly Lys Glu Ile
   645 650 655
   AGG AAA GCG ATA GTT CCT CAG GAT CCA AAC TGG TGG ATC GTC AGT GCC 2016
   Arg Lys Ala Ile Val Pro Gln Asp Pro Asn Trp Trp Ile Val Ser Ala
   660 665 670
   GAC TAC TCC CAA ATA GAA CTG AGG ATC CTC GCC CAT CTC AGT GGT GAT 2064
   Asp Tyr Ser Gln Ile Glu Leu Arg Ile Leu Ala His Leu Ser Gly Asp
   675 680 685
   GAG AAT CTT TTG AGG GCA TTC GAA GAG GGC ATC GAC GTC CAC ACT CTA 2112
   Glu Asn Leu Leu Arg Ala Phe Glu Glu Gly Ile Asp Val His Thr Leu
   690 695 700
   ACA GCT TCC AGA ATA TTC AAC GTG AAA CCC GAA GAA GTA ACC GAA GAA 2160
   Thr Ala Ser Arg Ile Phe Asn Val Lys Pro Glu Glu Val Thr Glu Glu
   705 710 715 720
   ATG CGC CGC GCT GGT AAA ATG GTT AAT TTT TCC ATC ATA TAC GGT GTA 2208
   Met Arg Arg Ala Gly Lys Met Val Asn Phe Ser Ile Ile Tyr Gly Val
   725 730 735
   ACA CCT TAC GGT CTG TCT GTG AGG CTT GGA GTA CCT GTG AAA GAA GCA 2256
   Thr Pro Tyr Gly Leu Ser Val Arg Leu Gly Val Pro Val Lys Glu Ala
   740 745 750
   GAA AAG ATG ATC GTC AAC TAC TTC GTC CTC TAC CCA AAG GTG CGC GAT 2304
   Glu Lys Met Ile Val Asn Tyr Phe Val Leu Tyr Pro Lys Val Arg Asp
   755 760 765
   TAC ATT CAG AGG GTC GTA TCG GAA GCG AAA GAA AAA GGC TAT GTT AGA 2352
   Tyr Ile Gln Arg Val Val Ser Glu Ala Lys Glu Lys Gly Tyr Val Arg
   770 775 780
   ACG CTG TTT GGA AGA AAA AGA GAC ATA CCA CAG CTC ATG GCC CGG GAC      2400
   Thr Leu Phe Gly Arg Lys Arg Asp Ile Pro Gln Leu Met Ala Arg Asp
   785                 790                 795                 800
   AGG AAC ACA CAG GCT GAA GGA GAA CGA ATT GCC ATA AAC ACT CCC ATA 2448
   Arg Asn Thr Gln Ala Glu Gly Glu Arg Ile Ala Ile Asn Thr Pro Ile
   805 810 815
   CAG GGT ACA GCA GCG GAT ATA ATA AAG CTG GCT ATG ATA GAA ATA GAC        2496
   Gln Gly Thr Ala Ala Asp Ile Ile Lys Leu Ala Met Ile Glu Ile Asp
               820                 825                 830
   AGG GAA CTG AAA GAA AGA AAA ATG AGA TCG AAG ATG ATC ATA CAG GTC        2544
   Arg Glu Leu Lys Glu Arg Lys Met Arg Ser Lys Met Ile Ile Gln Val
           835                 840                 845
   CAC GAC GAA CTG GTT TTT GAA GTG CCC AAT GAG GAA AAG GAC GCG CTC        2592
   His Asp Glu Leu Val Phe Glu Val Pro Asn Glu Glu Lys Asp Ala Leu
       850                 855                 860
   GTC GAG CTG GTG AAA GAC AGA ATG ACG AAT GTG GTA AAG CTT TCA GTG        2640
   Val Glu Leu Val Lys Asp Arg Met Thr Asn Val Val Lys Leu Ser Val
   865                 870                 875                 880
   CCG CTC GAA GTG GAT GTA ACC ATC GGC AAA ACA TGG TCG TGA                2682
   Pro Leu Glu Val Asp Val Thr Ile Gly Lys Thr Trp Ser
                   885                 890
(2) INFORMATION FOR SEQ ID NO:4:
   (i) SEQUENCE CHARACTERISTICS:
      (A) LENGTH: 893 amino acids
      (B) TYPE: amino acid
      (D) TOPOLOGY: linear
   (ii) MOLECULE TYPE: protein
   (xi) SEQUENCE DESCRIPTION: SEQ ID NO:4:
   Met Ala Arg Leu Phe Leu Phe Asp Gly Thr Ala Leu Ala Tyr Arg Ala
   1               5                   10                  15
   Tyr Tyr Ala Leu Asp Arg Ser Leu Ser Thr Ser Thr Gly Ile Pro Thr
               20                  25                  30
   Asn Ala Thr Tyr Gly Val Ala Arg Met Leu Val Arg Phe Ile Lys Asp
           35                  40                  45
   His Ile Ile Val Gly Lys Asp Tyr Val Ala Val Ala Phe Asp Lys Lys
       50                  55                  60
   Ala Ala Thr Phe Arg His Lys Leu Leu Glu Thr Tyr Lys Ala Gln Arg
   65                  70                  75                  80
   Pro Lys Thr Pro Asp Leu Leu Ile Gln Gln Leu Pro Tyr Ile Lys Lys
                   85                  90                  95
   Leu Val Glu Ala Leu Gly Met Lys Val Leu Glu Val Glu Gly Tyr Glu
   100 105 110
   Ala Asp Asp Ile Ile Ala Thr Leu Ala Val Lys Gly Leu Pro Leu Phe
   115 120 125
   Asp Glu Ile Phe Ile Val Thr Gly Asp Lys Asp Met Leu Gln Leu Val
   130 135 140
   Asn Glu Lys Ile Lys Val Trp Arg Ile Val Lys Gly Ile Ser Asp Leu
   145 150 155 160
   Glu Leu Tyr Asp Ala Gln Lys Val Lys Glu Lys Tyr Gly Val Glu Pro
   165 170 175
   Gln Gln Ile Pro Asp Leu Leu Ala Leu Thr Gly Asp Glu Ile Asp Asn
   180 185 190
   Ile Pro Gly Val Thr Gly Ile Gly Glu Lys Thr Ala Val Gln Leu Leu
   195 200 205
   Glu Lys Tyr Lys Asp Leu Glu Asp Ile Leu Asn His Val Arg Glu Leu
   210 215 220
   Pro Gln Lys Val Arg Lys Ala Leu Leu Arg Asp Arg Glu Asn Ala Ile
   225 230 235 240
   Leu Ser Lys Lys Leu Ala Ile Leu Glu Thr Asn Val Pro Ile Glu Ile
   245 250 255
   Asn Trp Glu Glu Leu Arg Tyr Gln Gly Tyr Asp Arg Glu Lys Leu Leu
   260 265 270
   Pro Leu Leu Lys Glu Leu Glu Phe Ala Ser Ile Met Lys Glu Leu Gln
   275 280 285
   Leu Tyr Glu Glu Ser Glu Pro Val Gly Tyr Arg Ile Val Lys Asp Leu
   290 295 300
   Val Glu Phe Glu Lys Leu Ile Glu Lys Leu Arg Glu Ser Pro Ser Phe
   305 310 315 320
   Ala Ile Asp Leu Glu Thr Ser Ser Leu Asp Pro Phe Asp Cys Asp Ile
   325 330 335
   Val Gly Ile Ser Val Ser Phe Lys Pro Lys Glu Ala Tyr Tyr Ile Pro
   340 345 350
   Leu His His Arg Asn Ala Gln Asn Leu Asp Glu Lys Glu Val Leu Lys
   355 360 365
   Lys Leu Lys Glu Ile Leu Glu Asp Pro Gly Ala Lys Ile Val Gly Gln
   370 375 380
   Asn Leu Lys Phe Asp Tyr Lys Val Leu Met Val Lys Gly Val Glu Pro
   385 390 395 400
   Val Pro Pro Tyr Phe Asp Thr Met Ile Ala Ala Tyr Leu Leu Glu Pro
                   405                 410                 415
   Asn Glu Lys Lys Phe Asn Leu Asp Asp Leu Ala Leu Lys Phe Leu Gly
               420                 425                 430
   Tyr Lys Met Thr Ser Tyr Gln Glu Leu Met Ser Phe Ser Phe Pro Leu
           435                 440                 445
   Phe Gly Phe Ser Phe Ala Asp Val Pro Val Glu Lys Ala Ala Asn Tyr
       450                 455                 460
   Ser Cys Glu Asp Ala Asp Ile Thr Tyr Arg Leu Tyr Lys Thr Leu Ser
   465                 470                 475                 480
   Leu Lys Leu His Glu Ala Asp Leu Glu Asn Val Phe Tyr Lys Ile Glu
                   485                 490                 495
   Met Pro Leu Val Asn Val Leu Ala Arg Met Glu Leu Asn Gly Val Tyr
               500                 505                 510
   Val Asp Thr Glu Phe Leu Lys Lys Leu Ser Glu Glu Tyr Gly Lys Lys
           515                 520                 525
   Leu Glu Glu Leu Ala Glu Glu Ile Tyr Arg Ile Ala Gly Glu Pro Phe
       530                 535                 540
   Asn Ile Asn Ser Pro Lys Gln Val Ser Arg Ile Leu Phe Glu Lys Leu
   545                 550                 555                 560
   Gly Ile Lys Pro Arg Gly Lys Thr Thr Lys Thr Gly Asp Tyr Ser Thr
                   565                 570                 575
   Arg Ile Glu Val Leu Glu Glu Leu Ala Gly Glu His Glu Ile Ile Pro
               580                 585                 590
   Leu Ile Leu Glu Tyr Arg Lys Ile Gln Lys Leu Lys Ser Thr Tyr Ile
           595                 600                 605
   Asp Ala Leu Pro Lys Met Val Asn Pro Lys Thr Gly Arg Ile His Ala
       610                 615                 620
   Ser Phe Asn Gln Thr Gly Thr Ala Thr Gly Arg Leu Ser Ser Ser Asp
   625                 630                 635                 640
   Pro Asn Leu Gln Asn Leu Pro Thr Lys Ser Glu Glu Gly Lys Glu Ile
                   645                 650                 655
   Arg Lys Ala Ile Val Pro Gln Asp Pro Asn Trp Trp Ile Val Ser Ala
               660                 665                 670
   Asp Tyr Ser Gln Ile Glu Leu Arg Ile Leu Ala His Leu Ser Gly Asp
           675                 680                 685
   Glu Asn Leu Leu Arg Ala Phe Glu Glu Gly Ile Asp Val His Thr Leu
       690                 695                 700
   Thr Ala Ser Arg Ile Phe Asn Val Lys Pro Glu Glu Val Thr Glu Glu
   705 710 715 720
   Met Arg Arg Ala Gly Lys Met Val Asn Phe Ser Ile Ile Tyr Gly Val
   725 730 735
   Thr Pro Tyr Gly Leu Ser Val Arg Leu Gly Val Pro Val Lys Glu Ala
   740 745 750
   Glu Lys Met Ile Val Asn Tyr Phe Val Leu Tyr Pro Lys Val Arg Asp
   755 760 765
   Tyr Ile Gln Arg Val Val Ser Glu Ala Lys Glu Lys Gly Tyr Val Arg
   770 775 780
   Thr Leu Phe Gly Arg Lys Arg Asp Ile Pro Gln Leu Met Ala Arg Asp
   785 790 795 800
   Arg Asn Thr Gln Ala Glu Gly Glu Arg Ile Ala Ile Asn Thr Pro Ile
   805 810 815
   Gln Gly Thr Ala Ala Asp Ile Ile Lys Leu Ala Met Ile Glu Ile Asp
   820 825 830
   Arg Glu Leu Lys Glu Arg Lys Met Arg Ser Lys Met Ile Ile Gln Val
   835 840 845
   His Asp Glu Leu Val Phe Glu Val Pro Asn Glu Glu Lys Asp Ala Leu
   850 855 860
   Val Glu Leu Val Lys Asp Arg Met Thr Asn Val Val Lys Leu Ser Val
   865 870 875 880
   Pro Leu Glu Val Asp Val Thr Ile Gly Lys Thr Trp Ser
   885 890
(2) INFORMATION FOR SEQ ID NO:5:
   (i) SEQUENCE CHARACTERISTICS:
      (A) LENGTH: 2493 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Thermus species sps17
   (ix) FEATURE:
      (A) NAME/KEY: CDS
      (B) LOCATION: 1..2490
   (xi) SEQUENCE DESCRIPTION: SEQ ID NO:5:
   ATG CTG CCC CTC TTT GAG CCC AAG GGC CGG GTC CTC CTG GTG GAC GGC 48
   Met Leu Pro Leu Phe Glu Pro Lys Gly Arg Val Leu Leu Val Asp Gly
   1 5 10 15
   CAC CAC CTG GCC TAC CGC ACC TTT TTC GCC CTC AAG GGC CTC ACC ACC 96
   His His Leu Ala Tyr Arg Thr Phe Phe Ala Leu Lys Gly Leu Thr Thr
   20 25 30
   AGC CGG GGC GAG CCC GTG CAG GCG GTT TAT GGC TTC GCC AAA AGC CTC 144
   Ser Arg Gly Glu Pro Val Gln Ala Val Tyr Gly Phe Ala Lys Ser Leu
   35 40 45
   CTC AAG GCC CTG AAG GAG GAT GGG GAG GTG GCC ATC GTG GTC TTT GAC 192
   Leu Lys Ala Leu Lys Glu Asp Gly Glu Val Ala Ile Val Val Phe Asp
   50 55 60
   GCC AAG GCC CCC TCC TTC CGC CAC GAG GCC TAC GAG GCC TAC AAG GCG 240
   Ala Lys Ala Pro Ser Phe Arg His Glu Ala Tyr Glu Ala Tyr Lys Ala
   65 70 75 80
   GGC CGG GCC CCC ACC CCG GAG GAC TTT CCC CGG CAG CTC GCC CTC ATC 288
   Gly Arg Ala Pro Thr Pro Glu Asp Phe Pro Arg Gln Leu Ala Leu Ile
   85 90 95
   AAG GAG CTG GTG GAC CTT TTG GGC CTC GTG CGC CTT GAG GTC CCG GGC 336
   Lys Glu Leu Val Asp Leu Leu Gly Leu Val Arg Leu Glu Val Pro Gly
   100 105 110
   TTT GAG GCG GAC GAT GTC GTC GCC ACC CTG GCC AAG AAG GCA GAA AGG 384
   Phe Glu Ala Asp Asp Val Leu Ala Thr Leu Ala Lys Lys Ala Glu Arg
   115 120 125
   GAG GGG TAC GAG GTG CGC ATC CTG AGC GCG GAC CGC GAC CTC TAC CAG 432
   Glu Gly Tyr Glu Val Arg Ile Leu Ser Ala Asp Arg Asp Leu Tyr Gln
   130 135 140
   CTC CTT TCC GAC CGG ATC CAC CTC CTC CAC CCC GAG GGG GAG GTC CTG 480
   Leu Leu Ser Asp Arg Ile His Leu Leu His Pro Glu Gly Glu Val Leu
   145 150 155 160
   ACC CCC GGG TGG CTC CAG GAG CGC TAC GGC CTC TCC CCG GAG AGG TGG 528
   Thr Pro Gly Trp Leu Gln Glu Arg Tyr Gly Leu Ser Pro Glu Arg Trp
   165 170 175
   GTG GAG TAC CGG GCC CTG GTG GGG GAC CCT TCG GAC AAC CTC CCC GGG 576
   Val Glu Tyr Arg Ala Leu Val Gly Asp Pro Ser Asp Asn Leu Pro Gly
   180 185 190
   GTG CCC GGC ATC GGG GAG AAG ACC GCC CTG AAG CTC CTG AAG GAG TGG 624
   Val Pro Gly Ile Gly Glu Lys Thr Ala Leu Lys Leu Leu Lys Glu Trp
   195 200 205
   GGT AGC CTG GAA GCG ATT CTA AAG AAC CTG GAC CAG GTG AAG CCG GAA 672
   Gly Ser Leu Glu Ala Ile Leu Lys Asn Leu Asp Gln Val Lys Pro Glu
   210 215 220
   AGG GTG CGG GAG GCC ATC CGG AAT AAC CTG GAT AAG CTC CAG ATG TCC 720
   Arg Val Arg Glu Ala Ile Arg Asn Asn Leu Asp Lys Leu Gln Met Ser
   225 230 235 240
   CTG GAG CTT TCC CGC CTC CGC ACC GAC CTC CCC CTG GAG GTG GAC TTC 768
   Leu Glu Leu Ser Arg Leu Arg Thr Asp Leu Pro Leu Glu Val Asp Phe
   245 250 255
   GCC AAG AGG CGG GAG CCC GAC TGG GAG GGG CTT AAG GCC TTT TTG GAG 816
   Ala Lys Arg Arg Glu Pro Asp Trp Glu Gly Leu Lys Ala Phe Leu Glu
   260 265 270
   CGG CTT GAG TTC GGA AGC CTC CTC CAC GAG TTC GGC CTT CTG GAG GCC 864
   Arg Leu Glu Phe Gly Ser Leu Leu His Glu Phe Gly Leu Leu Glu Ala
   275 280 285
   CCC AAG GAG GCG GAG GAG GCC CCC TGG CCC CCG CCT GGA GGG GCC TTT 912
   Pro Lys Glu Ala Glu Glu Ala Pro Trp Pro Pro Pro Gly Gly Ala Phe
   290 295 300
   TTG GGC TTC CTC CTC TCC CGC CCC GAG CCC ATG TGG GCG GAG CTT TTG 960
   Leu Gly Phe Leu Leu Ser Arg Pro Glu Pro Met Trp Ala Glu Leu Leu
   305 310 315 320
   GCC CTG GCG GGG GCC AAG GAG GGG CGG GTC CAT CGG GCG GAA GAC CCC 1008
   Ala Leu Ala Gly Ala Lys Glu Gly Arg Val His Arg Ala Glu Asp Pro
   325 330 335
   GTG GGG GCC CTA AAG GAC CTG AAG GAG ATC CGG GGC CTC CTC GCC AAG 1056
   Val Gly Ala Leu Lys Asp Leu Lys Glu Ile Arg Gly Leu Leu Ala Lys
   340 345 350
   GAC CTC TCG GTC CTG GCC CTG AGG GAG GGC CGG GAG ATC CCG CCG GGG 1104
   Asp Leu Ser Val Leu Ala Leu Arg Glu Gly Arg Glu Ile Pro Pro Gly
   355 360 365
   GAC GAC CCC ATG CTC CTC GCC TAC CTC CTG GAC CCG GGG AAC ACC AAC 1152
   Asp Asp Pro Met Leu Leu Ala Tyr Leu Leu Asp Pro Gly Asn Thr Asn
   370 375 380
   CCC GAG GGG GTG GCC CGG CGG TAC GGG GGG GAG TGG AAG GAG GAC GCC 1200
   Pro Glu Gly Val Ala Arg Arg Tyr Gly Gly Glu Trp Lys Glu Asp Ala
   385 390 395 400
   GCC GCC CGG GCC CTC CTT TCG GAA AGG CTC TGG CAG GCC CTT TAC CCC 1248
   Ala Ala Arg Ala Leu Leu Ser Glu Arg Leu Trp Gln Ala Leu Tyr Pro
   405 410 415
   CGG GTG GCG GAG GAG GAA AGG CTC CTT TGG CTC TAC CGG GAG GTG GAG 1296
   Arg Val Ala Glu Glu Glu Arg Leu Leu Trp Leu Tyr Arg Glu Val Glu
   420 425 430
   CGG CCC CTC GCC CAG GTC CTC GCC CAC ATG GAG GCC ACG GGG GTG CGG 1344
   Arg Pro Leu Ala Gln Val Leu Ala His Met Glu Ala Thr Gly Val Arg
   435 440 445
   CTG GAT GTG CCC TAC CTG GAG GCC CTT TCC CAG GAG GTG GCC TTT GAG 1392
   Leu Asp Val Pro Tyr Leu Glu Ala Leu Ser Gln Glu Val Ala Phe Glu
   450 455 460
   CTG GAG CGC CTC GAG GCC GAG GTC CAC CGC CTG GCG GGC CAC CCC TTC 1440
   Leu Glu Arg Leu Glu Ala Glu Val His Arg Leu Ala Gly His Pro Phe
   465 470 475 480
   AAC CTG AAC TCT AGG GAC CAG CTG GAG CGG GTC CTC TTT GAC GAG CTC 1488
   Asn Leu Asn Ser Arg Asp Gln Leu Glu Arg Val Leu Phe Asp Glu Leu
   485 490 495
   GGC CTA CCC CCC ATC GGC AAG ACG GAG AAG ACG GGC AAG CGC TCC ACC 1536
   Gly Leu Pro Pro Ile Gly Lys Thr Glu Lys Thr Gly Lys Arg Ser Thr
   500 505 510
   AGC GCC GCC GTC CTG GAG CTC TTA AGG GAG GCC CAC CCC ATC GTG GGG 1584
   Ser Ala Ala Val Leu Glu Leu Leu Arg Glu Ala His Pro Ile Val Gly
   515 520 525
   CGG ATC CTG GAG TAC CGG GAG CTC ATG AAG CTC AAG AGC ACC TAC ATA 1632
   Arg Ile Leu Glu Tyr Arg Glu Leu Met Lys Leu Lys Ser Thr Tyr Ile
   530 535 540
   GAC CCC CTC CCC AGG CTG GTC CAC CCC AAA ACC GGC CGG CTC CAC ACC 1680
   Asp Pro Leu Pro Arg Leu Val His Pro Lys Thr Gly Arg Leu His Thr
   545 550 555 560
   CGC TTC AAC CAG ACG GCC ACC GCC ACG GGC CGC CTC TCC AGC TCC GAC 1728
   Arg Phe Asn Gln Thr Ala Thr Ala Thr Gly Arg Leu Ser Ser Ser Asp
   565 570 575
   CCC AAC CTG CAG AAC ATC CCC GTG CGC ACC CCC TTA GGC CAG CGC ATC 1776
   Pro Asn Leu Gln Asn Ile Pro Val Arg Thr Pro Leu Gly Gln Arg Ile
   580 585 590
   CGC AAG GCC TTC ATT GCC GAG GAG GGC CAT CTC CTG GTG GCC CTG GAC 1824
   Arg Lys Ala Phe Ile Ala Glu Glu Gly His Leu Leu Val Ala Leu Asp
   595 600 605
   TAT AGC CAG ATC GAG CTC CGG GTC CTC GCC CAC CTC TCG GGG GAC GAG 1872
   Tyr Ser Gln Ile Glu Leu Arg Val Leu Ala His Leu Ser Gly Asp Glu
   610 615 620
   AAC CTC ATC CGG GTC TTC CGG GAA GGG AAG GAC ATC CAC ACC GAG ACC 1920
   Asn Leu Ile Arg Val Phe Arg Glu Gly Lys Asp Ile His Thr Glu Thr
   625 630 635 640
   GCC GCC TGG ATG TTC GGC GTG CCC CCC GAG GGG GTG GAC GGG GCC ATG 1968
   Ala Ala Trp Met Phe Gly Val Pro Pro Glu Gly Val Asp Gly Ala Met
   645 650 655
   CGC CGG GCG GCC AAG ACG GTG AAC TTC GGG GTG CTC TAC GGG ATG TCC 2016
   Arg Arg Ala Ala Lys Thr Val Asn Phe Gly Val Leu Tyr Gly Met Ser
   660 665 670
   GCC CAC CGC CTC TCC CAG GAG CTC TCC ATC CCC TAC GAG GAG GCG GCG 2064
   Ala His Arg Leu Ser Gln Glu Leu Ser Ile Pro Tyr Glu Glu Ala Ala
   675 680 685
   GCC TTC ATC GAG CGC TAC TTC CAG AGC TTC CCC AAG GTG CGG GCC TGG      2112
   Ala Phe Ile Glu Arg Tyr Phe Gln Ser Phe Pro Lys Val Arg Ala Trp
       690                 695                 700
   ATC GCC AAA ACC TTG GAG GAG GGG CGG AAG AAG GGC TAC GTG GAG ACC      2160
   Ile Ala Lys Thr Leu Glu Glu Gly Arg Lys Lys Gly Tyr Val Glu Thr
   705                 710                 715                 720
   CTC TTC GGC CGC CGC CGC TAC GTG CCC GAC CTC AAC GCC CGG GTG AAG      2208
   Leu Phe Gly Arg Arg Arg Tyr Val Pro Asp Leu Asn Ala Arg Val Lys
                   725                 730                 735
   AGC GTG CGG GAG GCG GCG GAG CGC ATG GCC TTC AAC ATG CCC GTG CAG      2256
   Ser Val Arg Glu Ala Ala Glu Arg Met Ala Phe Asn Met Pro Val Gln
               740                 745                 750
   GGC ACC GCC GCG GAC CTC ATG AAG CTG GCC ATG GTG AAG CTC TTC CCC      2304
   Gly Thr Ala Ala Asp Leu Met Lys Leu Ala Met Val Lys Leu Phe Pro
           755                 760                 765
   AGG CTC AGG CCC TTG GGC GTT CGC ATC CTC CTC CAG GTG CAC GAC GAG      2352
   Arg Leu Arg Pro Leu Gly Val Arg Ile Leu Leu Gln Val His Asp Glu
       770                 775                 780
   CTG GTC TTG GAG GCC CCA AAG GCG CGG GCG GAG GAG GCC GCC CAG TTG      2400
   Leu Val Leu Glu Ala Pro Lys Ala Arg Ala Glu Glu Ala Ala Gln Leu
   785                 790                 795                 800
   GCC AAG GAG ACC ATG GAA GGG GTT TAC CCC CTC TCC GTC CCC CTG GAG      2448
   Ala Lys Glu Thr Met Glu Gly Val Tyr Pro Leu Ser Val Pro Leu Glu
                   805                 810                 815
   GTG GAG GTG GGG ATG GGG GAG GAC TGG CTT TCC GCC AAG GCC              2490
   Val Glu Val Gly Met Gly Glu Asp Trp Leu Ser Ala Lys Ala
               820                 825                 830
   TAG                                                                  2493
(2) INFORMATION FOR SEQ ID NO:6:
   (i) SEQUENCE CHARACTERISTICS:
      (A) LENGTH: 830 amino acids
      (B) TYPE: amino acid
      (D) TOPOLOGY: linear
   (ii) MOLECULE TYPE: protein
   (xi) SEQUENCE DESCRIPTION: SEQ ID NO:6:
   Met Leu Pro Leu Phe Glu Pro Lys Gly Arg Val Leu Leu Val Asp Gly
     1               5                  10                  15
   His His Leu Ala Tyr Arg Thr Phe Phe Ala Leu Lys Gly Leu Thr Thr
                20                  25                  30
   Ser Arg Gly Glu Pro Val Gln Ala Val Tyr Gly Phe Ala Lys Ser Leu
            35                  40                  45
   Leu Lys Ala Leu Lys Glu Asp Gly Glu Val Ala Ile Val Val Phe Asp
        50                  55                  60
   Ala Lys Ala Pro Ser Phe Arg His Glu Ala Tyr Glu Ala Tyr Lys Ala
    65                  70                  75                  80
   Gly Arg Ala Pro Thr Pro Glu Asp Phe Pro Arg Gln Leu Ala Leu Ile
                    85                  90                  95
   Lys Glu Leu Val Asp Leu Leu Gly Leu Val Arg Leu Glu Val Pro Gly
               100                 105                 110
   Phe Glu Ala Asp Asp Val Leu Ala Thr Leu Ala Lys Lys Ala Glu Arg
           115                 120                 125
   Glu Gly Tyr Glu Val Arg Ile Leu Ser Ala Asp Arg Asp Leu Tyr Gln
       130                 135                 140
   Leu Leu Ser Asp Arg Ile His Leu Leu His Pro Glu Gly Glu Val Leu
   145                 150                 155                 160
   Thr Pro Gly Trp Leu Gln Glu Arg Tyr Gly Leu Ser Pro Glu Arg Trp
                   165                 170                 175
   Val Glu Tyr Arg Ala Leu Val Gly Asp Pro Ser Asp Asn Leu Pro Gly
               180                 185                 190
   Val Pro Gly Ile Gly Glu Lys Thr Ala Leu Lys Leu Leu Lys Glu Trp
           195                 200                 205
   Gly Ser Leu Glu Ala Ile Leu Lys Asn Leu Asp Gln Val Lys Pro Glu
       210                 215                 220
   Arg Val Arg Glu Ala Ile Arg Asn Asn Leu Asp Lys Leu Gln Met Ser
   225                 230                 235                 240
   Leu Glu Leu Ser Arg Leu Arg Thr Asp Leu Pro Leu Glu Val Asp Phe
                   245                 250                 255
   Ala Lys Arg Arg Glu Pro Asp Trp Glu Gly Leu Lys Ala Phe Leu Glu
               260                 265                 270
   Arg Leu Glu Phe Gly Ser Leu Leu His Glu Phe Gly Leu Leu Glu Ala
           275                 280                 285
   Pro Lys Glu Ala Glu Glu Ala Pro Trp Pro Pro Pro Gly Gly Ala Phe
       290                 295                 300
   Leu Gly Phe Leu Leu Ser Arg Pro Glu Pro Met Trp Ala Glu Leu Leu
   305                 310                 315                 320
   Ala Leu Ala Gly Ala Lys Glu Gly Arg Val His Arg Ala Glu Asp Pro
                   325                 330                 335
   Val Gly Ala Leu Lys Asp Leu Lys Glu Ile Arg Gly Leu Leu Ala Lys
               340                 345                 350
   Asp Leu Ser Val Leu Ala Leu Arg Glu Gly Arg Glu Ile Pro Pro Gly
           355                 360                 365
   Asp Asp Pro Met Leu Leu Ala Tyr Leu Leu Asp Pro Gly Asn Thr Asn
       370                 375                 380
   Pro Glu Gly Val Ala Arg Arg Tyr Gly Gly Glu Trp Lys Glu Asp Ala
   385                 390                 395                 400
   Ala Ala Arg Ala Leu Leu Ser Glu Arg Leu Trp Gln Ala Leu Tyr Pro
                   405                 410                 415
   Arg Val Ala Glu Glu Glu Arg Leu Leu Trp Leu Tyr Arg Glu Val Glu
               420                 425                 430
   Arg Pro Leu Ala Gln Val Leu Ala His Met Glu Ala Thr Gly Val Arg
           435                 440                 445
   Leu Asp Val Pro Tyr Leu Glu Ala Leu Ser Gln Glu Val Ala Phe Glu
       450                 455                 460
   Leu Glu Arg Leu Glu Ala Glu Val His Arg Leu Ala Gly His Pro Phe
   465                 470                 475                 480
   Asn Leu Asn Ser Arg Asp Gln Leu Glu Arg Val Leu Phe Asp Glu Leu
                   485                 490                 495
   Gly Leu Pro Pro Ile Gly Lys Thr Glu Lys Thr Gly Lys Arg Ser Thr
               500                 505                 510
   Ser Ala Ala Val Leu Glu Leu Leu Arg Glu Ala His Pro Ile Val Gly
           515                 520                 525
   Arg Ile Leu Glu Tyr Arg Glu Leu Met Lys Leu Lys Ser Thr Tyr Ile
       530                 535                 540
   Asp Pro Leu Pro Arg Leu Val His Pro Lys Thr Gly Arg Leu His Thr
   545                 550                 555                 560
   Arg Phe Asn Gln Thr Ala Thr Ala Thr Gly Arg Leu Ser Ser Ser Asp
                   565                 570                 575
   Pro Asn Leu Gln Asn Ile Pro Val Arg Thr Pro Leu Gly Gln Arg Ile
   580 585 590
   Arg Lys Ala Phe Ile Ala Glu Glu Gly His Leu Leu Val Ala Leu Asp
   595 600 605
   Tyr Ser Gln Ile Glu Leu Arg Val Leu Ala His Leu Ser Gly Asp Glu
   610 615 620
   Asn Leu Ile Arg Val Phe Arg Glu Gly Lys Asp Ile His Thr Glu Thr
   625 630 635 640
   Ala Ala Trp Met Phe Gly Val Pro Pro Glu Gly Val Asp Gly Ala Met
   645 650 655
   Arg Arg Ala Ala Lys Thr Val Asn Phe Gly Val Leu Tyr Gly Met Ser
   660 665 670
   Ala His Arg Leu Ser Gln Glu Leu Ser Ile Pro Tyr Glu Glu Ala Ala
   675 680 685
   Ala Phe Ile Glu Arg Tyr Phe Gln Ser Phe Pro Lys Val Arg Ala Trp
   690 695 700
   Ile Ala Lys Thr Leu Glu Glu Gly Arg Lys Lys Gly Tyr Val Glu Thr
   705 710 715 720
   Leu Phe Gly Arg Arg Arg Tyr Val Pro Asp Leu Asn Ala Arg Val Lys
   725 730 735
   Ser Val Arg Glu Ala Ala Glu Arg Met Ala Phe Asn Met Pro Val Gln
   740 745 750
   Gly Thr Ala Ala Asp Leu Met Lys Leu Ala Met Val Lys Leu Phe Pro
   755 760 765
   Arg Leu Arg Pro Leu Gly Val Arg Ile Leu Leu Gln Val His Asp Glu
   770 775 780
   Leu Val Leu Glu Ala Pro Lys Ala Arg Ala Glu Glu Ala Ala Gln Leu
   785 790 795 800
   Ala Lys Glu Thr Met Glu Gly Val Tyr Pro Leu Ser Val Pro Leu Glu
   805 810 815
   Val Glu Val Gly Met Gly Glu Asp Trp Leu Ser Ala Lys Ala
   820 825 830
(2) INFORMATION FOR SEQ ID NO:7:
   (i) SEQUENCE CHARACTERISTICS:
      (A) LENGTH: 2505 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Thermus species Z05
   (ix) FEATURE:
      (A) NAME/KEY: CDS
      (B) LOCATION: 1..2502
   (xi) SEQUENCE DESCRIPTION: SEQ ID NO:7:
   ATG AAG GCG ATG CTT CCG CTC TTT GAA CCC AAA GGC CGG GTT CTC CTG      48
   Met Lys Ala Met Leu Pro Leu Phe Glu Pro Lys Gly Arg Val Leu Leu
     1               5                  10                  15
   GTG GAC GGC CAC CAC CTG GCC TAC CGC ACC TTC TTC GCC CTA AAG GGC      96
   Val Asp Gly His His Leu Ala Tyr Arg Thr Phe Phe Ala Leu Lys Gly
                    20                  25                  30
   CTC ACC ACG AGC CGG GGC GAA CCG GTG CAG GCG GTT TAC GGC TTC GCC     144
   Leu Thr Thr Ser Arg Gly Glu Pro Val Gln Ala Val Tyr Gly Phe Ala
                35                  40                  45
   AAG AGC CTC CTC AAG GCC CTG AAG GAG GAC GGG TAC AAG GCC GTC TTC     192
   Lys Ser Leu Leu Lys Ala Leu Lys Glu Asp Gly Tyr Lys Ala Val Phe
            50                  55                  60
   GTG GTC TTT GAC GCC AAG GCC CCT TCC TTC CGC CAC GAG GCC TAC GAG     240
   Val Val Phe Asp Ala Lys Ala Pro Ser Phe Arg His Glu Ala Tyr Glu
    65                  70                  75                  80
   GCC TAC AAG GCA GGC CGC GCC CCG ACC CCC GAG GAC TTC CCC CGG CAG     288
   Ala Tyr Lys Ala Gly Arg Ala Pro Thr Pro Glu Asp Phe Pro Arg Gln
                    85                  90                  95
   CTC GCC CTC ATC AAG GAG CTG GTG GAC CTC CTG GGG TTT ACT CGC CTC     336
   Leu Ala Leu Ile Lys Glu Leu Val Asp Leu Leu Gly Phe Thr Arg Leu
                   100                 105                 110
   GAG GTT CCG GGC TTT GAG GCG GAC GAC GTC CTC GCC ACC CTG GCC AAG 384
   Glu Val Pro Gly Phe Glu Ala Asp Asp Val Leu Ala Thr Leu Ala Lys
   115 120 125
   AAG GCG GAA AGG GAG GGG TAC GAG GTG CGC ATC CTC ACC GCC GAC CGG 432
   Lys Ala Glu Arg Glu Gly Tyr Glu Val Arg Ile Leu Thr Ala Asp Arg
   130 135 140
   GAC CTT TAC CAG CTC GTC TCC GAC CGC GTC CCC GTC CTC CAC CCC GAG 480
   Asp Leu Tyr Gln Leu Val Ser Asp Arg Val Ala Val Leu His Pro Glu
   145 150 155 160
   GGC CAC CTC ATC ACC CCG GAG TGG CTT TGG GAG AAG TAC GGC CTT AAG 528
   Gly His Leu Ile Thr Pro Glu Trp Leu Trp Glu Lys Tyr Gly Leu Lys
   165 170 175
   CCG GAG CAG TGG GTG GAC TTC CGC GCC CTC GTG GGG GAC CCC TCC GAC 576
   Pro Glu Gln Trp Val Asp Phe Arg Ala Leu Val Gly Asp Pro Ser Asp
   180 185 190
   AAC CTC CCC GGG GTC AAG GGC ATC GGG GAG AAG ACC GCC CTC AAG CTC 624
   Asn Leu Pro Gly Val Lys Gly Ile Gly Glu Lys Thr Ala Leu Lys Leu
   195 200 205
   CTC AAG GAG TGG GGA AGC CTG GAA AAT ATC CTC AAG AAC CTG GAC CGG 672
   Leu Lys Glu Trp Gly Ser Leu Glu Asn Ile Leu Lys Asn Leu Asp Arg
   210 215 220
   GTG AAG CCG GAA AGC GTC CGG GAA AGG ATC AAG GCC CAC CTG GAA GAC 720
   Val Lys Pro Glu Ser Val Arg Glu Arg Ile Lys Ala His Leu Glu Asp
   225 230 235 240
   CTT AAG CTC TCC TTG GAG CTT TCC CGG GTG CGC TCG GAC CTC CCC CTG 768
   Leu Lys Leu Ser Leu Glu Leu Ser Arg Val Arg Ser Asp Leu Pro Leu
   245 250 255
   GAG GTG GAC TTC GCC CGG AGG CGG GAG CCT GAC CGG GAA GGG CTT CGG 816
   Glu Val Asp Phe Ala Arg Arg Arg Glu Pro Asp Arg Glu Gly Leu Arg
   260 265 270
   GCC TTT TTG GAG CGC TTG GAG TTC GGC AGC CTC CTC CAC GAG TTC GGC 864
   Ala Phe Leu Glu Arg Leu Glu Phe Gly Ser Leu Leu His Glu Phe Gly
   275 280 285
   CTC CTC GAG GCC CCC GCC CCC CTG GAG GAG GCC CCC TGG CCC CCG CCG 912
   Leu Leu Glu Ala Pro Ala Pro Leu Glu Glu Ala Pro Trp Pro Pro Pro
   290 295 300
   GAA GGG GCC TTC GTG GGC TTC GTC CTC TCC CGC CCC GAG CCC ATG TGG 960
   Glu Gly Ala Phe Val Gly Phe Val Leu Ser Arg Pro Glu Pro Met Trp
   305 310 315 320
   GCG GAG CTT AAA GCC CTG GCC GCC TGC AAG GAG GGC CGG GTG CAC CGG 1008
   Ala Glu Leu Lys Ala Leu Ala Ala Cys Lys Glu Gly Arg Val His Arg
   325 330 335
   GCA AAG GAC CCC TTG GCG GGG CTA AAG GAC CTC AAG GAG GTC CGA GGC 1056
   Ala Lys Asp Pro Leu Ala Gly Leu Lys Asp Leu Lys Glu Val Arg Gly
   340 345 350
   CTC CTC GCC AAG GAC CTC GCC GTT TTG GCC CTT CGC GAG GGG CTG GAC 1104
   Leu Leu Ala Lys Asp Leu Ala Val Leu Ala Leu Arg Glu Gly Leu Asp
   355 360 365
   CTC GCG CCT TCG GAC GAC CCC ATG CTC CTC GCC TAC CTC CTG GAC CCC 1152
   Leu Ala Pro Ser Asp Asp Pro Met Leu Leu Ala Tyr Leu Leu Asp Pro
   370 375 380
   TCC AAC ACC ACC CCC GAG GGG GTG GCC CGG CGC TAC GGG GGG GAG TGG 1200
   Ser Asn Thr Thr Pro Glu Gly Val Ala Arg Arg Tyr Gly Gly Glu Trp
   385 390 395 400
   ACG GAG GAC GCC GCC CAC CGG GCC CTC CTC GCC GAG CGG CTC CAG CAA 1248
   Thr Glu Asp Ala Ala His Arg Ala Leu Leu Ala Glu Arg Leu Gln Gln
   405 410 415
   AAC CTC TTG GAA CGC CTC AAG GGA GAG GAA AAG CTC CTT TGG CTC TAC 1296
   Asn Leu Leu Glu Arg Leu Lys Gly Glu Glu Lys Leu Leu Trp Leu Tyr
   420 425 430
   CAA GAG GTG GAA AAG CCC CTC TCC CGG GTC CTG GCC CAC ATG GAG GCC 1344
   Gln Glu Val Glu Lys Pro Leu Ser Arg Val Leu Ala His Met Glu Ala
   435 440 445
   ACC GGG GTA AGG CTG GAC GTG GCC TAT CTA AAG GCC CTT TCC CTG GAG 1392
   Thr Gly Val Arg Leu Asp Val Ala Tyr Leu Lys Ala Leu Ser Leu Glu
   450 455 460
   CTT GCG GAG GAG ATT CGC CGC CTC GAG GAG GAG GTC TTC CGC CTG GCG        1440
   Leu Ala Glu Glu Ile Arg Arg Leu Glu Glu Glu Val Phe Arg Leu Ala
   465                 470                 475                 480
   GGC CAC CCC TTC AAC CTG AAC TCC CGT GAC CAG CTA GAG CGG GTG CTC        1488
   Gly His Pro Phe Asn Leu Asn Ser Arg Asp Gln Leu Glu Arg Val Leu
                   485                 490                 495
   TTT GAC GAG CTT AGG CTT CCC GCC CTG GGC AAG ACG CAA AAG ACG GGG        1536
   Phe Asp Glu Leu Arg Leu Pro Ala Leu Gly Lys Thr Gln Lys Thr Gly
               500                 505                 510
   AAG CGC TCC ACC AGC GCC GCG GTG CTG GAG GCC CTC AGG GAG GCC CAC        1584
   Lys Arg Ser Thr Ser Ala Ala Val Leu Glu Ala Leu Arg Glu Ala His
           515                 520                 525
   CCC ATC GTG GAG AAG ATC CTC CAG CAC CGG GAG CTC ACC AAG CTC AAG        1632
   Pro Ile Val Glu Lys Ile Leu Gln His Arg Glu Leu Thr Lys Leu Lys
       530                 535                 540
   AAC ACC TAC GTG GAC CCC CTC CCG GGC CTC GTC CAC CCG AGG ACG GGC        1680
   Asn Thr Tyr Val Asp Pro Leu Pro Gly Leu Val His Pro Arg Thr Gly
   545                 550                 555                 560
   CGC CTC CAC ACC CGC TTC AAC CAG ACA GCC ACG GCC ACG GGA AGG CTC        1728
   Arg Leu His Thr Arg Phe Asn Gln Thr Ala Thr Ala Thr Gly Arg Leu
                   565                 570                 575
   TCT AGC TCC GAC CCC AAC CTG CAG AAC ATC CCC ATC CGC ACC CCC TTG        1776
   Ser Ser Ser Asp Pro Asn Leu Gln Asn Ile Pro Ile Arg Thr Pro Leu
               580                 585                 590
   GGC CAG AGG ATC CGC CGG GCC TTC GTG GCC GAG GCG GGA TGG GCG TTG        1824
   Gly Gln Arg Ile Arg Arg Ala Phe Val Ala Glu Ala Gly Trp Ala Leu
           595                 600                 605
   GTG GCC CTG GAC TAT AGC CAG ATA GAG CTC CGG GTC CTC GCC CAC CTC        1872
   Val Ala Leu Asp Tyr Ser Gln Ile Glu Leu Arg Val Leu Ala His Leu
       610                 615                 620
   TCC GGG GAC GAG AAC CTG ATC AGG GTC TTC CAG GAG GGG AAG GAC ATC        1920
   Ser Gly Asp Glu Asn Leu Ile Arg Val Phe Gln Glu Gly Lys Asp Ile
   625                 630                 635                 640
   CAC ACC CAG ACC GCA AGC TGG ATG TTC GGC GTC TCC CCG GAG GCC GTG 1968
   His Thr Gln Thr Ala Ser Trp Met Phe Gly Val Ser Pro Glu Ala Val
   645 650 655
   GAC CCC CTG ATG CGC CGG GCG GCC AAG ACG GTG AAC TTC GGC GTC CTC 2016
   Asp Pro Leu Met Arg Arg Ala Ala Lys Thr Val Asn Phe Gly Val Leu
   660 665 670
   TAC GGC ATG TCC GCC CAT AGG CTC TCC CAG GAG CTT GCC ATC CCC TAC 2064
   Tyr Gly Met Ser Ala His Arg Leu Ser Gln Glu Leu Ala Ile Pro Tyr
   675 680 685
   GAG GAG GCG GTG GCC TTT ATA GAG CGC TAC TTC CAA AGC TTC CCC AAG 2112
   Glu Glu Ala Val Ala Phe Ile Glu Arg Tyr Phe Gln Ser Phe Pro Lys
   690 695 700
   GTG CGG GCC TGG ATA GAA AAG ACC CTG GAG GAG GGG AGG AAG CGG GGC 2160
   Val Arg Ala Trp Ile Glu Lys Thr Leu Glu Glu Gly Arg Lys Arg Gly
   705 710 715 720
   TAC GTG GAA ACC CTC TTC GGA AGA AGG CGC TAC GTG CCC GAC CTC AAC 2208
   Tyr Val Glu Thr Leu Phe Gly Arg Arg Arg Tyr Val Pro Asp Leu Asn
   725 730 735
   GCC CGG GTG AAG AGC GTC AGG GAG GCC GCG GAG CGC ATG GCC TTC AAC 2256
   Ala Arg Val Lys Ser Val Arg Glu Ala Ala Glu Arg Met Ala Phe Asn
   740 745 750
   ATG CCC GTC CAG GGC ACC GCC GCC GAC CTC ATG AAG CTC GCC ATG GTG 2304
   Met Pro Val Gln Gly Thr Ala Ala Asp Leu Met Lys Leu Ala Met Val
   755 760 765
   AAG CTC TTC CCC CAC CTC CGG GAG ATG GGG GCC CGC ATG CTC CTC CAG 2352
   Lys Leu Phe Pro His Leu Arg Glu Met Gly Ala Arg Met Leu Leu Gln
   770 775 780
   GTC CAC GAC GAG CTC CTC CTG GAG GCC CCC CAA GCG CGG GCC GAG GAG 2400
   Val His Asp Glu Leu Leu Leu Glu Ala Pro Gln Ala Arg Ala Glu Glu
   785 790 795 800
   GTG GCG GCT TTG GCC AAG GAG GCC ATG GAG AAG GCC TAT CCC CTC GCC 2448
   Val Ala Ala Leu Ala Lys Glu Ala Met Glu Lys Ala Tyr Pro Leu Ala
   805 810 815
   GTG CCC CTG GAG GTG GAG GTG GGG ATC GGG GAG GAC TGG CTT TCC GCC 2496
   Val Pro Leu Glu Val Glu Val Gly Ile Gly Glu Asp Trp Leu Ser Ala
   820 825 830
   AAG GCC TGA 2505
   Lys Gly
(2) INFORMATION FOR SEQ ID NO:8:
   (i) SEQUENCE CHARACTERISTICS:
      (A) LENGTH: 834 amino acids
      (B) TYPE: amino acid
      (D) TOPOLOGY: linear
   (ii) MOLECULE TYPE: protein
   (xi) SEQUENCE DESCRIPTION: SEQ ID NO:8:
   Met Lys Ala Met Leu Pro Leu Phe Glu Pro Lys Gly Arg Val Leu Leu
   1 5 10 15
   Val Asp Gly His His Leu Ala Tyr Arg Thr Phe Phe Ala Leu Lys Gly
   20 25 30
   Leu Thr Thr Ser Arg Gly Glu Pro Val Gln Ala Val Tyr Gly Phe Ala
   35 40 45
   Lys Ser Leu Leu Lys Ala Leu Lys Glu Asp Gly Tyr Lys Ala Val Phe
   50 55 60
   Val Val Phe Asp Ala Lys Ala Pro Ser Phe Arg His Glu Ala Tyr Glu
   65 70 75 80
   Ala Tyr Lys Ala Gly Arg Ala Pro Thr Pro Glu Asp Phe Pro Arg Gln
   85 90 95
   Leu Ala Leu Ile Lys Glu Leu Val Asp Leu Leu Gly Phe Thr Arg Leu
   100 105 110
   Glu Val Pro Gly Phe Glu Ala Asp Asp Val Leu Ala Thr Leu Ala Lys
   115 120 125
   Lys Ala Glu Arg Glu Gly Tyr Glu Val Arg Ile Leu Thr Ala Asp Arg
   130 135 140
   Asp Leu Tyr Gln Leu Val Ser Asp Arg Val Ala Val Leu His Pro Glu
   145 150 155 160
   Gly His Leu Ile Thr Pro Glu Trp Leu Trp Glu Lys Tyr Gly Leu Lys
   165 170 175
   Pro Glu Gln Trp Val Asp Phe Arg Ala Leu Val Gly Asp Pro Ser Asp
   180 185 190
   Asn Leu Pro Gly Val Lys Gly Ile Gly Glu Lys Thr Ala Leu Lys Leu
   195 200 205
   Leu Lys Glu Trp Gly Ser Leu Glu Asn Ile Leu Lys Asn Leu Asp Arg
   210 215 220
   Val Lys Pro Glu Ser Val Arg Glu Arg Ile Lys Ala His Leu Glu Asp
   225 230 235 240
   Leu Lys Leu Ser Leu Glu Leu Ser Arg Val Arg Ser Asp Leu Pro Leu
   245 250 255
   Glu Val Asp Phe Ala Arg Arg Arg Glu Pro Asp Arg Glu Gly Leu Arg
   260 265 270
   Ala Phe Leu Glu Arg Leu Glu Phe Gly Ser Leu Leu His Glu Phe Gly
   275 280 285
   Leu Leu Glu Ala Pro Ala Pro Leu Glu Glu Ala Pro Trp Pro Pro Pro
   290 295 300
   Glu Gly Ala Phe Val Gly Phe Val Leu Ser Arg Pro Glu Pro Met Trp
   305 310 315 320
   Ala Glu Leu Lys Ala Leu Ala Ala Cys Lys Glu Gly Arg Val His Arg
   325 330 335
   Ala Lys Asp Pro Leu Ala Gly Leu Lys Asp Leu Lys Glu Val Arg Gly
   340 345 350
   Leu Leu Ala Lys Asp Leu Ala Val Leu Ala Leu Arg Glu Gly Leu Asp
   355 360 365
   Leu Ala Pro Ser Asp Asp Pro Met Leu Leu Ala Tyr Leu Leu Asp Pro
   370 375 380
   Ser Asn Thr Thr Pro Glu Gly Val Ala Arg Arg Tyr Gly Gly Glu Trp
   385 390 395 400
   Thr Glu Asp Ala Ala His Arg Ala Leu Leu Ala Glu Arg Leu Gln Gln
   405 410 415
   Asn Leu Leu Glu Arg Leu Lys Gly Glu Glu Lys Leu Leu Trp Leu Tyr
   420 425 430
   Gln Glu Val Glu Lys Pro Leu Ser Arg Val Leu Ala His Met Glu Ala
   435 440 445
   Thr Gly Val Arg Leu Asp Val Ala Tyr Leu Lys Ala Leu Ser Leu Glu
   450 455 460
   Leu Ala Glu Glu Ile Arg Arg Leu Glu Glu Glu Val Phe Arg Leu Ala
   465 470 475 480
   Gly His Pro Phe Asn Leu Asn Ser Arg Asp Gln Leu Glu Arg Val Leu
   485 490 495
   Phe Asp Glu Leu Arg Leu Pro Ala Leu Gly Lys Thr Gln Lys Thr Gly
   500 505 510
   Lys Arg Ser Thr Ser Ala Ala Val Leu Glu Ala Leu Arg Glu Ala His
   515 520 525
   Pro Ile Val Glu Lys Ile Leu Gln His Arg Glu Leu Thr Lys Leu Lys
   530 535 540
   Asn Thr Tyr Val Asp Pro Leu Pro Gly Leu Val His Pro Arg Thr Gly
   545 550 555 560
   Arg Leu His Thr Arg Phe Asn Gln Thr Ala Thr Ala Thr Gly Arg Leu
   565 570 575
   Ser Ser Ser Asp Pro Asn Leu Gln Asn Ile Pro Ile Arg Thr Pro Leu
   580 585 590
   Gly Gln Arg Ile Arg Arg Ala Phe Val Ala Glu Ala Gly Trp Ala Leu
   595 600 605
   Val Ala Leu Asp Tyr Ser Gln Ile Glu Leu Arg Val Leu Ala His Leu
   610 615 620
   Ser Gly Asp Glu Asn Leu Ile Arg Val Phe Gln Glu Gly Lys Asp Ile
   625 630 635 640
   His Thr Gln Thr Ala Ser Trp Met Phe Gly Val Ser Pro Glu Ala Val
   645 650 655
   Asp Pro Leu Met Arg Arg Ala Ala Lys Thr Val Asn Phe Gly Val Leu
   660 665 670
   Tyr Gly Met Ser Ala His Arg Leu Ser Gln Glu Leu Ala Ile Pro Tyr
   675 680 685
   Glu Glu Ala Val Ala Phe Ile Glu Arg Tyr Phe Gln Ser Phe Pro Lys
   690 695 700
   Val Arg Ala Trp Ile Glu Lys Thr Leu Glu Glu Gly Arg Lys Arg Gly
   705 710 715 720
   Tyr Val Glu Thr Leu Phe Gly Arg Arg Arg Tyr Val Pro Asp Leu Asn
   725 730 735
   Ala Arg Val Lys Ser Val Arg Glu Ala Ala Glu Arg Met Ala Phe Asn
   740 745 750
   Met Pro Val Gln Gly Thr Ala Ala Asp Leu Met Lys Leu Ala Met Val
   755 760 765
   Lys Leu Phe Pro His Leu Arg Glu Met Gly Ala Arg Met Leu Leu Gln
   770 775 780
   Val His Asp Glu Leu Leu Leu Glu Ala Pro Gln Ala Arg Ala Glu Glu
   785                 790                 795                 800
   Val Ala Ala Leu Ala Lys Glu Ala Met Glu Lys Ala Tyr Pro Leu Ala
                   805                 810                 815
   Val Pro Leu Glu Val Glu Val Gly Ile Gly Glu Asp Trp Leu Ser Ala
               820                 825                 830
   Lys Gly
(2) INFORMATION FOR SEQ ID NO:9:
   (i) SEQUENCE CHARACTERISTICS:
      (A) LENGTH: 2505 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Thermus thermophilus
   (ix) FEATURE:
      (A) NAME/KEY: CDS
      (B) LOCATION: 1..2502
   (xi) SEQUENCE DESCRIPTION: SEQ ID NO:9:
   ATG GAG GCG ATG CTT CCG CTC TTT GAA CCC AAA GGC CGG GTC CTC CTG       48
   Met Glu Ala Met Leu Pro Leu Phe Glu Pro Lys Gly Arg Val Leu Leu
     1               5                  10                  15
   GTG GAC GGC CAC CAC CTG GCC TAC CGC ACC TTC TTC GCC CTG AAG GGC       96
   Val Asp Gly His His Leu Ala Tyr Arg Thr Phe Phe Ala Leu Lys Gly
                20                  25                  30
   CTC ACC ACG AGC CGG GGC GAA CCG GTG CAG GCG GTC TAC GGC TTC GCC      144
   Leu Thr Thr Ser Arg Gly Glu Pro Val Gln Ala Val Tyr Gly Phe Ala
            35                  40                  45
   AAG AGC CTC CTC AAG GCC CTG AAG GAG GAC GGG TAC AAG GCC GTC TTC      192
   Lys Ser Leu Leu Lys Ala Leu Lys Glu Asp Gly Tyr Lys Ala Val Phe
        50                  55                  60
   GTG GTC TTT GAC GCC AAG GCC CCC TCC TTC CGC CAC GAG GCC TAC GAG        240
   Val Val Phe Asp Ala Lys Ala Pro Ser Phe Arg His Glu Ala Tyr Glu
    65                  70                  75                  80
   GCC TAC AAG GCG GGG AGG GCC CCG ACC CCC GAG GAC TTC CCC CGG CAG        288
   Ala Tyr Lys Ala Gly Arg Ala Pro Thr Pro Glu Asp Phe Pro Arg Gln
                        85                  90                  95
   CTC GCC CTC ATC AAG GAG CTG GTG GAC CTC CTG GGG TTT ACC CGC CTC        336
   Leu Ala Leu Ile Lys Glu Leu Val Asp Leu Leu Gly Phe Thr Arg Leu
               100                 105                 110
   GAG GTC CCC GGC TAC GAG GCG GAC GAC GTT CTC GCC ACC CTG GCC AAG        384
   Glu Val Pro Gly Tyr Glu Ala Asp Asp Val Leu Ala Thr Leu Ala Lys
           115                 120                 125
   AAG GCG GAA AAG GAG GGG TAC GAG GTG CGC ATC CTC ACC GCC GAC CGC        432
   Lys Ala Glu Lys Glu Gly Tyr Glu Val Arg Ile Leu Thr Ala Asp Arg
       130                 135                 140
   GAC CTC TAC CAA CTC GTC TCC GAC CGC GTC GCC GTC CTC CAC CCC GAG        480
   Asp Leu Tyr Gln Leu Val Ser Asp Arg Val Ala Val Leu His Pro Glu
   145                 150                 155                 160
   GGC CAC CTC ATC ACC CCG GAG TGG CTT TGG GAG AAG TAC GGC CTC AGG        528
   Gly His Leu Ile Thr Pro Glu Trp Leu Trp Glu Lys Tyr Gly Leu Arg
                   165                 170                 175
   CCG GAG CAG TGG GTG GAC TTC CGC GCC CTC GTG GGG GAC CCC TCC GAC        576
   Pro Glu Gln Trp Val Asp Phe Arg Ala Leu Val Gly Asp Pro Ser Asp
               180                 185                 190
   AAC CTC CCC GGG GTC AAG GGC ATC GGG GAG AAG ACC GCC CTC AAG CTC        624
   Asn Leu Pro Gly Val Lys Gly Ile Gly Glu Lys Thr Ala Leu Lys Leu
           195                 200                 205
   CTC AAG GAG TGG GGA AGC CTG GAA AAC CTC CTC AAG AAC CTG GAC CGG        672
   Leu Lys Glu Trp Gly Ser Leu Glu Asn Leu Leu Lys Asn Leu Asp Arg
       210                 215                 220
   GTA AAG CCA GAA AAC GTC CGG GAG AAG ATC AAG GCC CAC CTG GAA GAC        720
   Val Lys Pro Glu Asn Val Arg Glu Lys Ile Lys Ala His Leu Glu Asp
   225                 230                 235                 240
   CTC AGG CTC TCC TTG GAG CTC TCC CGG GTG CGC ACC GAC CTC CCC CTG 768
   Leu Arg Leu Ser Leu Glu Leu Ser Arg Val Arg Thr Asp Leu Pro Leu
   245 250 255
   GAG GTG GAC CTC GCC CAG GGG CGG GAG CCC GAC CGG GAG GGG CTT AGG 816
   Glu Val Asp Leu Ala Gln Gly Arg Glu Pro Asp Arg Glu Gly Leu Arg
   260 265 270
   GCC TTC CTG GAG AGG CTG GAG TTC GGC AGC CTC CTC CAC GAG TTC GGC 864
   Ala Phe Leu Glu Arg Leu Glu Phe Gly Ser Leu Leu His Glu Phe Gly
   275 280 285
   CTC CTG GAG GCC CCC GCC CCC CTG GAG GAG GCC CCC TGG CCC CCG CCG 912
   Leu Leu Glu Ala Pro Ala Pro Leu Glu Glu Ala Pro Trp Pro Pro Pro
   290 295 300
   GAA GGG GCC TTC GTG GGC TTC GTC CTC TCC CGC CCC GAG CCC ATG TGG 960
   Glu Gly Ala Phe Val Gly Phe Val Leu Ser Arg Pro Glu Pro Met Trp
   305 310 315 320
   GCG GAG CTT AAA GCC CTG GCC GCC TGC AGG GAC GGC CGG GTG CAC CGG 1008
   Ala Glu Leu Lys Ala Leu Ala Ala Cys Arg Asp Gly Arg Val His Arg
   325 330 335
   GCA GCA GAC CCC TTG GCG GGG CTA AAG GAC CTC AAG GAG GTC CGG GGC 1056
   Ala Ala Asp Pro Leu Ala Gly Leu Lys Asp Leu Lys Glu Val Arg Gly
   340 345 350
   CTC CTC GCC AAG GAC CTC GCC GTC TTG GCC TCG AGG GAG GGG CTA GAC 1104
   Leu Leu Ala Lys Asp Leu Ala Val Leu Ala Ser Arg Glu Gly Leu Asp
   355 360 365
   CTC GTG CCC GGG GAC GAC CCC ATG CTC CTC GCC TAC CTC CTG GAC CCC 1152
   Leu Val Pro Gly Asp Asp Pro Met Leu Leu Ala Tyr Leu Leu Asp Pro
   370 375 380
   TCC AAC ACC ACC CCC GAG GGG GTG GCG CGG CGC TAC GGG GGG GAG TGG 1200
   Ser Asn Thr Thr Pro Glu Gly Val Ala Arg Arg Tyr Gly Gly Glu Trp
   385 390 395 400
   ACG GAG GAC GCC GCC CAC CGG GCC CTC CTC TCG GAG AGG CTC CAT CGG 1248
   Thr Glu Asp Ala Ala His Arg Ala Leu Leu Ser Glu Arg Leu His Arg
   405 410 415
   AAC CTC CTT AAG CGC CTC GAG GGG GAG GAG AAG CTC CTT TGG CTC TAC 1296
   Asn Leu Leu Lys Arg Leu Glu Gly Glu Glu Lys Leu Leu Trp Leu Tyr
   420 425 430
   CAC GAG GTG GAA AAG CCC CTC TCC CGG GTC CTG GCC CAC ATG GAG GCC 1344
   His Glu Val Glu Lys Pro Leu Ser Arg Val Leu Ala His Met Glu Ala
   435 440 445
   ACC GGG GTA CGG CTG GAC GTG GCC TAC CTT CAG GCC CTT TCC CTG GAG 1392
   Thr Gly Val Arg Leu Asp Val Ala Tyr Leu Gln Ala Leu Ser Leu Glu
   450 455 460
   CTT GCG GAG GAG ATC CGC CGC CTC GAG GAG GAG GTC TTC CGC TTG GCG 1440
   Leu Ala Glu Glu Ile Arg Arg Leu Glu Glu Glu Val Phe Arg Leu Ala
   465 470 475 480
   GGC CAC CCC TTC AAC CTC AAC TCC CGG GAC CAG CTG GAA AGG GTG CTC 1488
   Gly His Pro Phe Asn Leu Asn Ser Arg Asp Gln Leu Glu Arg Val Leu
   485 490 495
   TTT GAC GAG CTT AGG CTT CCC GCC TTG GGG AAG ACG CAA AAG ACA GGC 1536
   Phe Asp Glu Leu Arg Leu Pro Ala Leu Gly Lys Thr Gln Lys Thr Gly
   500 505 510
   AAG CGC TCC ACC AGC GCC GCG GTG CTG GAG GCC CTA CGG GAG GCC CAC 1584
   Lys Arg Ser Thr Ser Ala Ala Val Leu Glu Ala Leu Arg Glu Ala His
   515 520 525
   CCC ATC GTG GAG AAG ATC CTC CAG CAC CGG GAG CTC ACC AAG CTC AAG 1632
   Pro Ile Val Glu Lys Ile Leu Gln His Arg Glu Leu Thr Lys Leu Lys
   530 535 540
   AAC ACC TAC GTG GAC CCC CTC CCA AGC CTC GTC CAC CCG AGG ACG GGC 1680
   Asn Thr Tyr Val Asp Pro Leu Pro Ser Leu Val His Pro Arg Thr Gly
   545 550 555 560
   CGC CTC CAC ACC CGC TTC AAC CAG ACG GCC ACG GCC ACG GGG AGG CTT 1728
   Arg Leu His Thr Arg Phe Asn Gln Thr Ala Thr Ala Thr Gly Arg Leu
   565 570 575
   AGT AGC TCC GAC CCC AAC CTG CAG AAC ATC CCC GTC CGC ACC CCC TTG 1776
   Ser Ser Ser Asp Pro Asn Leu Gln Asn Ile Pro Val Arg Thr Pro Leu
   580 585 590
   GGC CAG AGG ATC CGC CGG GCC TTC GTG GCC GAG GCG GGT TGG GCG TTG 1824
   Gly Gln Arg Ile Arg Arg Ala Phe Val Ala Glu Ala Gly Trp Ala Leu
   595 600 605
   GTG GCC CTG GAC TAT AGC CAG ATA GAG CTC CGC GTC CTC GCC CAC CTC 1872
   Val Ala Leu Asp Tyr Ser Gln Ile Glu Leu Arg Val Leu Ala His Leu
   610 615 620
   TCC GGG GAC GAA AAC CTG ATC AGG GTC TTC CAG GAG GGG AAG GAC ATC 1920
   Ser Gly Asp Glu Asn Leu Ile Arg Val Phe Gln Glu Gly Lys Asp Ile
   625 630 635 640
   CAC ACC CAG ACC GCA AGC TGG ATG TTC GGC GTC CCC CCG GAG GCC GTG 1968
   His Thr Gln Thr Ala Ser Trp Met Phe Gly Val Pro Pro Glu Ala Val
   645 650 655
   GAC CCC CTG ATG CGC CGG GCG GCC AAG ACG GTG AAC TTC GGC GTC CTC 2016
   Asp Pro Leu Met Arg Arg Ala Ala Lys Thr Val Asn Phe Gly Val Leu
   660 665 670
   TAC GGC ATG TCC GCC CAT AGG CTC TCC CAG GAG CTT GCC ATC CCC TAC 2064
   Tyr Gly Met Ser Ala His Arg Leu Ser Gln Glu Leu Ala Ile Pro Tyr
   675 680 685
   GAG GAG GCG GTG GCC TTT ATA GAG CGC TAC TTC CAA AGC TTC CCC AAG 2112
   Glu Glu Ala Val Ala Phe Ile Glu Arg Tyr Phe Gln Ser Phe Pro Lys
   690 695 700
   GTG CGG GCC TGG ATA GAA AAG ACC CTG GAG GAG GGG AGG AAG CGG GGC 2160
   Val Arg Ala Trp Ile Glu Lys Thr Leu Glu Glu Gly Arg Lys Arg Gly
   705 710 715 720
   TAC GTG GAA ACC CTC TTC GGA AGA AGG CGC TAC GTG CCC GAC CTC AAC 2208
   Tyr Val Glu Thr Leu Phe Gly Arg Arg Arg Tyr Val Pro Asp Leu Asn
   725 730 735
   GCC CGG GTG AAG AGC GTC AGG GAG GCC GCG GAG CGC ATG GCC TTC AAC 2256
   Ala Arg Val Lys Ser Val Arg Glu Ala Ala Glu Arg Met Ala Phe Asn
   740 745 750
   ATG CCC GTC CAG GGC ACC GCC GCC GAC CTC ATG AAG CTC GCC ATG GTG 2304
   Met Pro Val Gln Gly Thr Ala Ala Asp Leu Met Lys Leu Ala Met Val
   755 760 765
   AAG CTC TTC CCC CGC CTC CGG GAG ATG GGG GCC CGC ATG CTC CTC CAG      2352
   Lys Leu Phe Pro Arg Leu Arg Glu Met Gly Ala Arg Met Leu Leu Gln
       770                 775                 780
   GTC CAC GAC GAG CTC CTC CTG GAG GCC CCC CAA GCG CGG GCC GAG GAG      2400
   Val His Asp Glu Leu Leu Leu Glu Ala Pro Gln Ala Arg Ala Glu Glu
   785                 790                 795                 800
   GTG GCG GCT TTG GCC AAG GAG GCC ATG GAG AAG GCC TAT CCC CTC GCC      2448
   Val Ala Ala Leu Ala Lys Glu Ala Met Glu Lys Ala Tyr Pro Leu Ala
                   805                 810                 815
   GTG CCC CTG GAG GTG GAG GTG GGG ATG GGG GAG GAC TGG CTT TCC GCC      2496
   Val Pro Leu Glu Val Glu Val Gly Met Gly Glu Asp Trp Leu Ser Ala
               820                 825                 830
   AAG GGT TAG                                                          2505
   Lys Gly
(2) INFORMATION FOR SEQ ID NO:10:
   (i) SEQUENCE CHARACTERISTICS:
      (A) LENGTH: 834 amino acids
      (B) TYPE: amino acid
      (D) TOPOLOGY: linear
   (ii) MOLECULE TYPE: protein
   (xi) SEQUENCE DESCRIPTION: SEQ ID NO:10:
   Met Glu Ala Met Leu Pro Leu Phe Glu Pro Lys Gly Arg Val Leu Leu
     1               5                  10                  15
   Val Asp Gly His His Leu Ala Tyr Arg Thr Phe Phe Ala Leu Lys Gly
               20                  25                  30
   Leu Thr Thr Ser Arg Gly Glu Pro Val Gln Ala Val Tyr Gly Phe Ala
           35                  40                  45
   Lys Ser Leu Leu Lys Ala Leu Lys Glu Asp Gly Tyr Lys Ala Val Phe
       50                  55                  60
   Val Val Phe Asp Ala Lys Ala Pro Ser Phe Arg His Glu Ala Tyr Glu
    65                  70                  75                  80
   Ala Tyr Lys Ala Gly Arg Ala Pro Thr Pro Glu Asp Phe Pro Arg Gln
                   85                  90                  95
   Leu Ala Leu Ile Lys Glu Leu Val Asp Leu Leu Gly Phe Thr Arg Leu
   100 105 110
   Glu Val Pro Gly Tyr Glu Ala Asp Asp Val Leu Ala Thr Leu Ala Lys
   115 120 125
   Lys Ala Glu Lys Glu Gly Tyr Glu Val Arg Ile Leu Thr Ala Asp Arg
   130 135 140
   Asp Leu Tyr Gln Leu Val Ser Asp Arg Val Ala Val Leu His Pro Glu
   145 150 155 160
   Gly His Leu Ile Thr Pro Glu Trp Leu Trp Glu Lys Tyr Gly Leu Arg
   165 170 175
   Pro Glu Gln Trp Val Asp Phe Arg Ala Leu Val Gly Asp Pro Ser Asp
   180 185 190
   Asn Leu Pro Gly Val Lys Gly Ile Gly Glu Lys Thr Ala Leu Lys Leu
   195 200 205
   Leu Lys Glu Trp Gly Ser Leu Glu Asn Leu Leu Lys Asn Leu Asp Arg
   210 215 220
   Val Lys Pro Glu Asn Val Arg Glu Lys Ile Lys Ala His Leu Glu Asp
   225 230 235 240
   Leu Arg Leu Ser Leu Glu Leu Ser Arg Val Arg Thr Asp Leu Pro Leu
   245 250 255
   Glu Val Asp Leu Ala Gln Gly Arg Glu Pro Asp Arg Glu Gly Leu Arg
   260 265 270
   Ala Phe Leu Glu Arg Leu Glu Phe Gly Ser Leu Leu His Glu Phe Gly
   275 280 285
   Leu Leu Glu Ala Pro Ala Pro Leu Glu Glu Ala Pro Trp Pro Pro Pro
   290 295 300
   Glu Gly Ala Phe Val Gly Phe Val Leu Ser Arg Pro Glu Pro Met Trp
   305 310 315 320
   Ala Glu Leu Lys Ala Leu Ala Ala Cys Arg Asp Gly Arg Val His Arg
   325 330 335
   Ala Ala Asp Pro Leu Ala Gly Leu Lys Asp Leu Lys Glu Val Arg Gly
   340 345 350
   Leu Leu Ala Lys Asp Leu Ala Val Leu Ala Ser Arg Glu Gly Leu Asp
   355 360 365
   Leu Val Pro Gly Asp Asp Pro Met Leu Leu Ala Tyr Leu Leu Asp Pro
   370 375 380
   Ser Asn Thr Thr Pro Glu Gly Val Ala Arg Arg Tyr Gly Gly Glu Trp
   385 390 395 400
   Thr Glu Asp Ala Ala His Arg Ala Leu Leu Ser Glu Arg Leu His Arg
   405 410 415
   Asn Leu Leu Lys Arg Leu Glu Gly Glu Glu Lys Leu Leu Trp Leu Tyr
   420 425 430
   His Glu Val Glu Lys Pro Leu Ser Arg Val Leu Ala His Met Glu Ala
   435 440 445
   Thr Gly Val Arg Leu Asp Val Ala Tyr Leu Gln Ala Leu Ser Leu Glu
   450 455 460
   Leu Ala Glu Glu Ile Arg Arg Leu Glu Glu Glu Val Phe Arg Leu Ala
   465 470 475 480
   Gly His Pro Phe Asn Leu Asn Ser Arg Asp Gln Leu Glu Arg Val Leu
   485 490 495
   Phe Asp Glu Leu Arg Leu Pro Ala Leu Gly Lys Thr Gln Lys Thr Gly
   500 505 510
   Lys Arg Ser Thr Ser Ala Ala Val Leu Glu Ala Leu Arg Glu Ala His
   515 520 525
   Pro Ile Val Glu Lys Ile Leu Gln His Arg Glu Leu Thr Lys Leu Lys
   530 535 540
   Asn Thr Tyr Val Asp Pro Leu Pro Ser Leu Val His Pro Arg Thr Gly
   545 550 555 560
   Arg Leu His Thr Arg Phe Asn Gln Thr Ala Thr Ala Thr Gly Arg Leu
   565 570 575
   Ser Ser Ser Asp Pro Asn Leu Gln Asn Ile Pro Val Arg Thr Pro Leu
   580 585 590
   Gly Gln Arg Ile Arg Arg Ala Phe Val Ala Glu Ala Gly Trp Ala Leu
   595 600 605
   Val Ala Leu Asp Tyr Ser Gln Ile Glu Leu Arg Val Leu Ala His Leu
   610 615 620
   Ser Gly Asp Glu Asn Leu Ile Arg Val Phe Gln Glu Gly Lys Asp Ile
   625 630 635 640
   His Thr Gln Thr Ala Ser Trp Met Phe Gly Val Pro Pro Glu Ala Val
   645 650 655
   Asp Pro Leu Met Arg Arg Ala Ala Lys Thr Val Asn Phe Gly Val Leu
   660 665 670
   Tyr Gly Met Ser Ala His Arg Leu Ser Gln Glu Leu Ala Ile Pro Tyr
   675 680 685
   Glu Glu Ala Val Ala Phe Ile Glu Arg Tyr Phe Gln Ser Phe Pro Lys
   690 695 700
   Val Arg Ala Trp Ile Glu Lys Thr Leu Glu Glu Gly Arg Lys Arg Gly
   705 710 715 720
   Tyr Val Glu Thr Leu Phe Gly Arg Arg Arg Tyr Val Pro Asp Leu Asn
   725 730 735
   Ala Arg Val Lys Ser Val Arg Glu Ala Ala Glu Arg Met Ala Phe Asn
   740 745 750
   Met Pro Val Gln Gly Thr Ala Ala Asp Leu Met Lys Leu Ala Met Val
   755 760 765
   Lys Leu Phe Pro Arg Leu Arg Glu Met Gly Ala Arg Met Leu Leu Gln
   770 775 780
   Val His Asp Glu Leu Leu Leu Glu Ala Pro Gln Ala Arg Ala Glu Glu
   785 790 795 800
   Val Ala Ala Leu Ala Lys Glu Ala Met Glu Lys Ala Tyr Pro Leu Ala
   805 810 815
   Val Pro Leu Glu Val Glu Val Gly Met Gly Glu Asp Trp Leu Ser Ala
   820 825 830
   Lys Gly
(2) INFORMATION FOR SEQ ID NO:11:
   (i) SEQUENCE CHARACTERISTICS:
      (A) LENGTH: 2679 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Thermosipho africanus
   (ix) FEATURE:
      (A) NAME/KEY: CDS
      (B) LOCATION: 1..2676
   (xi) SEQUENCE DESCRIPTION: SEQ ID NO:11:
   ATG GGA AAG ATG TTT CTA TTT GAT GGA ACT GGA TTA GTA TAC AGA GCA        48
   Met Gly Lys Met Phe Leu Phe Asp Gly Thr Gly Leu Val Tyr Arg Ala
     1               5                  10                  15
   TTT TAT GCT ATA GAT CAA TCT CTT CAA ACT TCG TCT GGT TTA CAC ACT        96
   Phe Tyr Ala Ile Asp Gln Ser Leu Gln Thr Ser Ser Gly Leu His Thr
                   20                  25                  30
   AAT GCT GTA TAC GGA CTT ACT AAA ATG CTT ATA AAA TTT TTA AAA GAA       144
   Asn Ala Val Tyr Gly Leu Thr Lys Met Leu Ile Lys Phe Leu Lys Glu
           35                  40                  45
   CAT ATC AGT ATT GGA AAA GAT GCT TGT GTT TTT GTT TTA GAT TCA AAA       192
   His Ile Ser Ile Gly Lys Asp Ala Cys Val Phe Val Leu Asp Ser Lys
       50                  55                  60
   GGT GGT AGC AAA AAA AGA AAG GAT ATT CTT GAA ACA TAT AAA GCA AAT       240
   Gly Gly Ser Lys Lys Arg Lys Asp Ile Leu Glu Thr Tyr Lys Ala Asn
   65                  70                  75                  80
   AGG CCA TCA ACG CCT GAT TTA CTT TTA GAG CAA ATT CCA TAT GTA GAA       288
   Arg Pro Ser Thr Pro Asp Leu Leu Leu Glu Gln Ile Pro Tyr Val Glu
                   85                  90                  95
   GAA CTT GTT GAT GCT CTT GGA ATA AAA GTT TTA AAA ATA GAA GGC TTT       336
   Glu Leu Val Asp Ala Leu Gly Ile Lys Val Leu Lys Ile Glu Gly Phe
               100                 105                 110
   GAA GCT GAT GAC ATT ATT GCT ACG CTT TCT AAA AAA TTT GAA AGT GAT       384
   Glu Ala Asp Asp Ile Ile Ala Thr Leu Ser Lys Lys Phe Glu Ser Asp
           115                 120                 125
   TTT GAA AAG GTA AAC ATA ATA ACT GGA GAT AAA GAT CTT TTA CAA CTT       432
   Phe Glu Lys Val Asn Ile Ile Thr Gly Asp Lys Asp Leu Leu Gln Leu
       130                 135                 140
   GTT TCT GAT AAG GTT TTT GTT TGG AGA GTA GAA AGA GGA ATA ACA GAT       480
   Val Ser Asp Lys Val Phe Val Trp Arg Val Glu Arg Gly Ile Thr Asp
   145                 150                 155                 160
   TTG GTA TTG TAC GAT AGA AAT AAA GTG ATT GAA AAA TAT GGA ATC TAC       528
   Leu Val Leu Tyr Asp Arg Asn Lys Val Ile Glu Lys Tyr Gly Ile Tyr
                   165                 170                 175
   CCA GAA CAA TTC AAA GAT TAT TTA TCT CTT GTC GGT GAT CAG ATT GAT       576
   Pro Glu Gln Phe Lys Asp Tyr Leu Ser Leu Val Gly Asp Gln Ile Asp
               180                 185                 190
   AAT ATC CCA GGA GTT AAA GGA ATA GGA AAG AAA ACA GCT GTT TCG CTT 624
   Asn Ile Pro Gly Val Lys Gly Ile Gly Lys Lys Thr Ala Val Ser Leu
   195 200 205
   TTG AAA AAA TAT AAT AGC TTG GAA AAT GTA TTA AAA AAT ATT AAC CTT 672
   Leu Lys Lys Tyr Asn Ser Leu Glu Asn Val Leu Lys Asn Ile Asn Leu
   210 215 220
   TTG ACG GAA AAA TTA AGA AGG CTT TTG GAA GAT TCA AAG GAA GAT TTG 720
   Leu Thr Glu Lys Leu Arg Arg Leu Leu Glu Asp Ser Lys Glu Asp Leu
   225 230 235 240
   CAA AAA AGT ATA GAA CTT GTG GAG TTG ATA TAT GAT GTA CCA ATG GAT 768
   Gln Lys Ser Ile Glu Leu Val Glu Leu Ile Tyr Asp Val Pro Met Asp
   245 250 255
   GTG GAA AAA GAT GAA ATA ATT TAT AGA GGG TAT AAT CCA GAT AAG CTT 816
   Val Glu Lys Asp Glu Ile Ile Tyr Arg Gly Tyr Asn Pro Asp Lys Leu
   260 265 270
   TTA AAG GTA TTA AAA AAG TAC GAA TTT TCA TCT ATA ATT AAG GAG TTA 864
   Leu Lys Val Leu Lys Lys Tyr Glu Phe Ser Ser Ile Ile Lys Glu Leu
   275 280 285
   AAT TTA CAA GAA AAA TTA GAA AAG GAA TAT ATA CTG GTA GAT AAT GAA 912
   Asn Leu Gln Glu Lys Leu Glu Lys Glu Tyr Ile Leu Val Asp Asn Glu
   290 295 300
   GAT AAA TTG AAA AAA CTT GCA GAA GAG ATA GAA AAA TAC AAA ACT TTT 960
   Asp Lys Leu Lys Lys Leu Ala Glu Glu Ile Glu Lys Tyr Lys Thr Phe
   305 310 315 320
   TCA ATT GAT ACG GAA ACA ACT TCA CTT GAT CCA TTT GAA GCT AAA CTG 1008
   Ser Ile Asp Thr Glu Thr Thr Ser Leu Asp Pro Phe Glu Ala Lys Leu
   325 330 335
   GTT GGG ATC TCT ATT TCC ACA ATG GAA GGG AAG GCG TAT TAT ATT CCG 1056
   Val Gly Ile Ser Ile Ser Thr Met Glu Gly Lys Ala Tyr Tyr Ile Pro
   340 345 350
   GTG TCT CAT TTT GGA GCT AAG AAT ATT TCC AAA AGT TTA ATA GAT AAA 1104
   Val Ser His Phe Gly Ala Lys Asn Ile Ser Lys Ser Leu Ile Asp Lys
   355 360 365
   TTT CTA AAA CAA ATT TTG CAA GAG AAG GAT TAT AAT ATC GTT GGT CAG 1152
   Phe Leu Lys Gln Ile Leu Gln Glu Lys Asp Tyr Asn Ile Val Gly Gln
   370 375 380
   AAT TTA AAA TTT GAC TAT GAG ATT TTT AAA AGC ATG GGT TTT TCT CCA 1200
   Asn Leu Lys Phe Asp Tyr Glu Ile Phe Lys Ser Met Gly Phe Ser Pro
   385 390 395 400
   AAT GTT CCG CAT TTT GAT ACG ATG ATT GCA GCC TAT CTT TTA AAT CCA 1248
   Asn Val Pro His Phe Asp Thr Met Ile Ala Ala Tyr Leu Leu Asn Pro
   405 410 415
   GAT GAA AAA CGT TTT AAT CTT GAA GAG CTA TCC TTA AAA TAT TTA GGT 1296
   Asp Glu Lys Arg Phe Asn Leu Glu Glu Leu Ser Leu Lys Tyr Leu Gly
   420 425 430
   TAT AAA ATG ATC TCG TTT GAT GAA TTA GTA AAT GAA AAT GTA CCA TTG 1344
   Tyr Lys Met Ile Ser Phe Asp Glu Leu Val Asn Glu Asn Val Pro Leu
   435 440 445
   TTT GGA AAT GAC TTT TCG TAT GTT CCA CTA GAA AGA GCC GTT GAG TAT 1392
   Phe Gly Asn Asp Phe Ser Tyr Val Pro Leu Glu Arg Ala Val Glu Tyr
   450 455 460
   TCC TGT GAA GAT GCC GAT GTG ACA TAC AGA ATA TTT AGA AAG CTT GGT 1440
   Ser Cys Glu Asp Ala Asp Val Thr Tyr Arg Ile Phe Arg Lys Leu Gly
   465 470 475 480
   AGG AAG ATA TAT GAA AAT GAG ATG GAA AAG TTG TTT TAC GAA ATT GAG 1488
   Arg Lys Ile Tyr Glu Asn Glu Met Glu Lys Leu Phe Tyr Glu Ile Glu
   485 490 495
   ATG CCC TTA ATT GAT GTT CTT TCA GAA ATG GAA CTA AAT GGA GTG TAT 1536
   Met Pro Leu Ile Asp Val Leu Ser Glu Met Glu Leu Asn Gly Val Tyr
   500 505 510
   TTT GAT GAG GAA TAT TTA AAA GAA TTA TCA AAA AAA TAT CAA GAA AAA 1584
   Phe Asp Glu Glu Tyr Leu Lys Glu Leu Ser Lys Lys Tyr Gln Glu Lys
   515 520 525
   ATG GAT GGA ATT AAG GAA AAA GTT TTT GAG ATA GCT GGT GAA ACT TTC 1632
   Met Asp Gly Ile Lys Glu Lys Val Phe Glu Ile Ala Gly Glu Thr Phe
   530 535 540
   AAT TTA AAC TCT TCA ACT CAA GTA GCA TAT ATA CTA TTT GAA AAA TTA 1680
   Asn Leu Asn Ser Ser Thr Gln Val Ala Tyr Ile Leu Phe Glu Lys Leu
   545 550 555 560
   AAT ATT GCT CCT TAC AAA AAA ACA GCG ACT GGT AAG TTT TCA ACT AAT 1728
   Asn Ile Ala Pro Tyr Lys Lys Thr Ala Thr Gly Lys Phe Ser Thr Asn
   565 570 575
   GCG GAA GTT TTA GAA GAA CTT TCA AAA GAA CAT GAA ATT GCA AAA TTG 1776
   Ala Glu Val Leu Glu Glu Leu Ser Lys Glu His Glu Ile Ala Lys Leu
   580 585 590
   TTG CTG GAG TAT CGA AAG TAT CAA AAA TTA AAA AGT ACA TAT ATT GAT 1824
   Leu Leu Glu Tyr Arg Lys Tyr Gln Lys Leu Lys Ser Thr Tyr Ile Asp
   595 600 605
   TCA ATA CCG TTA TCT ATT AAT CGA AAA ACA AAC AGG GTC CAT ACT ACT 1872
   Ser Ile Pro Leu Ser Ile Asn Arg Lys Thr Asn Arg Val His Thr Thr
   610 615 620
   TTT CAT CAA ACA GGA ACT TCT ACT GGA AGA TTA AGT AGT TCA AAT CCA 1920
   Phe His Gln Thr Gly Thr Ser Thr Gly Arg Leu Ser Ser Ser Asn Pro
   625 630 635 640
   AAT TTG CAA AAT CTT CCA ACA AGA AGC GAA GAA GGA AAA GAA ATA AGA 1968
   Asn Leu Gln Asn Leu Pro Thr Arg Ser Glu Glu Gly Lys Glu Ile Arg
   645 650 655
   AAA GCA GTA AGA CCT CAA AGA CAA GAT TGG TGG ATT TTA GGT GCT GAC 2016
   Lys Ala Val Arg Pro Gln Arg Gln Asp Trp Trp Ile Leu Gly Ala Asp
   660 665 670
   TAT TCT CAG ATA GAA CTA AGG GTT TTA GCG CAT GTA AGT AAA GAT GAA 2064
   Tyr Ser Gln Ile Glu Leu Arg Val Leu Ala His Val Ser Lys Asp Glu
   675 680 685
   AAT CTA CTT AAA GCA TTT AAA GAA GAT TTA GAT ATT CAT ACA ATT ACT 2112
   Asn Leu Leu Lys Ala Phe Lys Glu Asp Leu Asp Ile His Thr Ile Thr
   690 695 700
   GCT GCC AAA ATT TTT GGT GTT TCA GAG ATG TTT GTT AGT GAA CAA ATG 2160
   Ala Ala Lys Ile Phe Gly Val Ser Glu Met Phe Val Ser Glu Gln Met
   705 710 715 720
   AGA AGA GTT GGA AAG ATG GTA AAT TTT GCA ATT ATT TAT GGA GTT TCA     2208
   Arg Arg Val Gly Lys Met Val Asn Phe Ala Ile Ile Tyr Gly Val Ser
                   725                 730                 735
   CCT TAT GGT CTT TCA AAG AGA ATT GGT CTT AGT GTT TCA GAG ACT AAA     2256
   Pro Tyr Gly Leu Ser Lys Arg Ile Gly Leu Ser Val Ser Glu Thr Lys
               740                 745                 750
   AAA ATA ATA GAT AAC TAT TTT AGA TAC TAT AAA GGA GTT TTT GAA TAT     2304
   Lys Ile Ile Asp Asn Tyr Phe Arg Tyr Tyr Lys Gly Val Phe Glu Tyr
           755                 760                 765
   TTA AAA AGG ATG AAA GAT GAA GCA AGG AAA AAA GGT TAT GTT ACA ACG     2352
   Leu Lys Arg Met Lys Asp Glu Ala Arg Lys Lys Gly Tyr Val Thr Thr
       770                 775                 780
   CTT TTT GGA AGG CGC AGA TAT ATT CCA CAG TTA AGA TCG AAA AAT GGT     2400
   Leu Phe Gly Arg Arg Arg Tyr Ile Pro Gln Leu Arg Ser Lys Asn Gly
   785                 790                 795                 800
   AAT AGA GTT CAA GAA GGA GAA AGA ATA GCT GTA AAC ACT CCA ATT CAA     2448
   Asn Arg Val Gln Glu Gly Glu Arg Ile Ala Val Asn Thr Pro Ile Gln
                   805                 810                 815
   GGA ACA GCA GCT GAT ATA ATA AAG ATA GCT ATG ATT AAT ATT CAT AAT     2496
   Gly Thr Ala Ala Asp Ile Ile Lys Ile Ala Met Ile Asn Ile His Asn
               820                 825                 830
   AGA TTG AAG AAG GAA AAT CTA CGT TCA AAA ATG ATA TTG CAG GTT CAT     2544
   Arg Leu Lys Lys Glu Asn Leu Arg Ser Lys Met Ile Leu Gln Val His
           835                 840                 845
   GAC GAG TTA GTT TTT GAA GTG CCC GAT AAT GAA CTG GAG ATT GTA AAA     2592
   Asp Glu Leu Val Phe Glu Val Pro Asp Asn Glu Leu Glu Ile Val Lys
       850                 855                 860
   GAT TTA GTA AGA GAT GAG ATG GAA AAT GCA GTT AAG CTA GAC GTT CCT     2640
   Asp Leu Val Arg Asp Glu Met Glu Asn Ala Val Lys Leu Asp Val Pro
   865                 870                 875                 880
   TTA AAA GTA GAT GTT TAT TAT GGA AAA GAG TGG GAA TAA                 2679
   Leu Lys Val Asp Val Tyr Tyr Gly Lys Glu Trp Glu
                   885                 890
(2) INFORMATION FOR SEQ ID NO:12:
   (i) SEQUENCE CHARACTERISTICS:
      (A) LENGTH: 892 amino acids
      (B) TYPE: amino acid
      (D) TOPOLOGY: linear
   (ii) MOLECULE TYPE: protein
   (xi) SEQUENCE DESCRIPTION: SEQ ID NO:12:
   Met Gly Lys Met Phe Leu Phe Asp Gly Thr Gly Leu Val Tyr Arg Ala
   1 5 10 15
   Phe Tyr Ala Ile Asp Gln Ser Leu Gln Thr Ser Ser Gly Leu His Thr
   20 25 30
   Asn Ala Val Tyr Gly Leu Thr Lys Met Leu Ile Lys Phe Leu Lys Glu
   35 40 45
   His Ile Ser Ile Gly Lys Asp Ala Cys Val Phe Val Leu Asp Ser Lys
   50 55 60
   Gly Gly Ser Lys Lys Arg Lys Asp Ile Leu Glu Thr Tyr Lys Ala Asn
   65 70 75 80
   Arg Pro Ser Thr Pro Asp Leu Leu Leu Glu Gln Ile Pro Tyr Val Glu
   85 90 95
   Glu Leu Val Asp Ala Leu Gly Ile Lys Val Leu Lys Ile Glu Gly Phe
   100 105 110
   Glu Ala Asp Asp Ile Ile Ala Thr Leu Ser Lys Lys Phe Glu Ser Asp
   115 120 125
   Phe Glu Lys Val Asn Ile Ile Thr Gly Asp Lys Asp Leu Leu Gln Leu
   130 135 140
   Val Ser Asp Lys Val Phe Val Trp Arg Val Glu Arg Gly Ile Thr Asp
   145 150 155 160
   Leu Val Leu Tyr Asp Arg Asn Lys Val Ile Glu Lys Tyr Gly Ile Tyr
   165 170 175
   Pro Glu Gln Phe Lys Asp Tyr Leu Ser Leu Val Gly Asp Gln Ile Asp
   180 185 190
   Asn Ile Pro Gly Val Lys Gly Ile Gly Lys Lys Thr Ala Val Ser Leu
   195 200 205
   Leu Lys Lys Tyr Asn Ser Leu Glu Asn Val Leu Lys Asn Ile Asn Leu
   210 215 220
   Leu Thr Glu Lys Leu Arg Arg Leu Leu Glu Asp Ser Lys Glu Asp Leu
   225 230 235 240
   Gln Lys Ser Ile Glu Leu Val Glu Leu Ile Tyr Asp Val Pro Met Asp
   245 250 255
   Val Glu Lys Asp Glu Ile Ile Tyr Arg Gly Tyr Asn Pro Asp Lys Leu
   260 265 270
   Leu Lys Val Leu Lys Lys Tyr Glu Phe Ser Ser Ile Ile Lys Glu Leu
   275 280 285
   Asn Leu Gln Glu Lys Leu Glu Lys Glu Tyr Ile Leu Val Asp Asn Glu
   290 295 300
   Asp Lys Leu Lys Lys Leu Ala Glu Glu Ile Glu Lys Tyr Lys Thr Phe
   305 310 315 320
   Ser Ile Asp Thr Glu Thr Thr Ser Leu Asp Pro Phe Glu Ala Lys Leu
   325 330 335
   Val Gly Ile Ser Ile Ser Thr Met Glu Gly Lys Ala Tyr Tyr Ile Pro
   340 345 350
   Val Ser His Phe Gly Ala Lys Asn Ile Ser Lys Ser Leu Ile Asp Lys
   355 360 365
   Phe Leu Lys Gln Ile Leu Gln Glu Lys Asp Tyr Asn Ile Val Gly Gln
   370 375 380
   Asn Leu Lys Phe Asp Tyr Glu Ile Phe Lys Ser Met Gly Phe Ser Pro
   385 390 395 400
   Asn Val Pro His Phe Asp Thr Met Ile Ala Ala Tyr Leu Leu Asn Pro
   405 410 415
   Asp Glu Lys Arg Phe Asn Leu Glu Glu Leu Ser Leu Lys Tyr Leu Gly
   420 425 430
   Tyr Lys Met Ile Ser Phe Asp Glu Leu Val Asn Glu Asn Val Pro Leu
   435 440 445
   Phe Gly Asn Asp Phe Ser Tyr Val Pro Leu Glu Arg Ala Val Glu Tyr
   450 455 460
   Ser Cys Glu Asp Ala Asp Val Thr Tyr Arg Ile Phe Arg Lys Leu Gly
   465 470 475 480
   Arg Lys Ile Tyr Glu Asn Glu Met Glu Lys Leu Phe Tyr Glu Ile Glu
   485 490 495
   Met Pro Leu Ile Asp Val Leu Ser Glu Met Glu Leu Asn Gly Val Tyr
   500 505 510
   Phe Asp Glu Glu Tyr Leu Lys Glu Leu Ser Lys Lys Tyr Gln Glu Lys
   515 520 525
   Met Asp Gly Ile Lys Glu Lys Val Phe Glu Ile Ala Gly Glu Thr Phe
   530 535 540
   Asn Leu Asn Ser Ser Thr Gln Val Ala Tyr Ile Leu Phe Glu Lys Leu
   545 550 555 560
   Asn Ile Ala Pro Tyr Lys Lys Thr Ala Thr Gly Lys Phe Ser Thr Asn
   565 570 575
   Ala Glu Val Leu Glu Glu Leu Ser Lys Glu His Glu Ile Ala Lys Leu
   580 585 590
   Leu Leu Glu Tyr Arg Lys Tyr Gln Lys Leu Lys Ser Thr Tyr Ile Asp
   595 600 605
   Ser Ile Pro Leu Ser Ile Asn Arg Lys Thr Asn Arg Val His Thr Thr
   610 615 620
   Phe His Gln Thr Gly Thr Ser Thr Gly Arg Leu Ser Ser Ser Asn Pro
   625 630 635 640
   Asn Leu Gln Asn Leu Pro Thr Arg Ser Glu Glu Gly Lys Glu Ile Arg
   645 650 655
   Lys Ala Val Arg Pro Gln Arg Gln Asp Trp Trp Ile Leu Gly Ala Asp
   660 665 670
   Tyr Ser Gln Ile Glu Leu Arg Val Leu Ala His Val Ser Lys Asp Glu
   675 680 685
   Asn Leu Leu Lys Ala Phe Lys Glu Asp Leu Asp Ile His Thr Ile Thr
   690 695 700
   Ala Ala Lys Ile Phe Gly Val Ser Glu Met Phe Val Ser Glu Gln Met
   705 710 715 720
   Arg Arg Val Gly Lys Met Val Asn Phe Ala Ile Ile Tyr Gly Val Ser
   725 730 735
   Pro Tyr Gly Leu Ser Lys Arg Ile Gly Leu Ser Val Ser Glu Thr Lys
   740 745 750
   Lys Ile Ile Asp Asn Tyr Phe Arg Tyr Tyr Lys Gly Val Phe Glu Tyr
   755 760 765
   Leu Lys Arg Met Lys Asp Glu Ala Arg Lys Lys Gly Tyr Val Thr Thr
   770 775 780
   Leu Phe Gly Arg Arg Arg Tyr Ile Pro Gln Leu Arg Ser Lys Asn Gly
   785 790 795 800
   Asn Arg Val Gln Glu Gly Glu Arg Ile Ala Val Asn Thr Pro Ile Gln
   805 810 815
   Gly Thr Ala Ala Asp Ile Ile Lys Ile Ala Met Ile Asn Ile His Asn
   820 825 830
   Arg Leu Lys Lys Glu Asn Leu Arg Ser Lys Met Ile Leu Gln Val His
   835 840 845
   Asp Glu Leu Val Phe Glu Val Pro Asp Asn Glu Leu Glu Ile Val Lys
       850                 855                 860
   Asp Leu Val Arg Asp Glu Met Glu Asn Ala Val Lys Leu Asp Val Pro
   865                 870                 875                 880
   Leu Lys Val Asp Val Tyr Tyr Gly Lys Glu Trp Glu
                   885                 890
(2) INFORMATION FOR SEQ ID NO:13:
   (i) SEQUENCE CHARACTERISTICS:
      (A) LENGTH: 33 nucleotides
      (B) TYPE: nucleic acid
      (C) STRANDEDNESS: single
      (D) TOPOLOGY: linear
   (ii) MOLECULE TYPE: DNA probe BW33
   (iii) HYPOTHETICAL: NO
   (iv) ANTI-SENSE: NO
   (xi) SEQUENCE DESCRIPTION: SEQ ID NO:13:
   GATCGCTGCG CGTAACCACC ACACCCGCCG CGC                    33
(2) INFORMATION FOR SEQ ID NO:14:
   (i) SEQUENCE CHARACTERISTICS:
      (A) LENGTH: 30 nucleotides
      (B) TYPE: nucleic acid
      (C) STRANDEDNESS: single
      (D) TOPOLOGY: linear
   (ii) MOLECULE TYPE: DNA primer BW37
   (iii) HYPOTHETICAL: NO
   (iv) ANTI-SENSE: NO
   (xi) SEQUENCE DESCRIPTION: SEQ ID NO:14:
   GCGCTAGGGC GCTGGCAAGT GTAGCGGTCA                        30
(2) INFORMATION FOR SEQ ID NO:15:
   (i) SEQUENCE CHARACTERISTICS:
      (A) LENGTH: 4 amino acids
      (B) TYPE: amino acid
      (D) TOPOLOGY: linear
   (ii) MOLECULE TYPE: peptide
   (iii) HYPOTHETICAL: YES
   (iv) ANTI-SENSE: NO
   (ix) FEATURE:
      (A) NAME/KEY: Peptide
      (B) LOCATION: 1..4
      (D) OTHER INFORMATION: /label- Xaa /note- "Xaa - Val or Thr"
   (xi) SEQUENCE DESCRIPTION: SEQ ID NO:15:
   Ala Xaa Tyr Gly
   1
(2) INFORMATION FOR SEQ ID NO:21:
   (i) SEQUENCE CHARACTERISTICS:
      (A) LENGTH: 22 nucleotides
      (B) TYPE: nucleic acid
      (C) STRANDEDNESS: single
      (D) TOPOLOGY: linear
   (ii) MOLECULE TYPE: DNA primer MK61
   (iii) HYPOTHETICAL: NO
   (iv) ANTI-SENSE: NO
   (xi) SEQUENCE DESCRIPTION: SEQ ID NO:21:
   AGGACTACAA CTGCCACACA CC                                    22
(2) INFORMATION FOR SEQ ID NO:22:
   (i) SEQUENCE CHARACTERISTICS:
      (A) LENGTH: 38 nucleotides
      (B) TYPE: nucleic acid
      (C) STRANDEDNESS: single
      (D) TOPOLOGY: linear
   (ii) MOLECULE TYPE: DNA primer RA01
   (iii) HYPOTHETICAL: NO
   (iv) ANTI-SENSE: NO
   (xi) SEQUENCE DESCRIPTION: SEQ ID NO:22:
   CGAGGCGCGC CAGCCCCAGG AGATCTACCA GCTCCTTG 38
(2) INFORMATION FOR SEQ ID NO:30:
   (i) SEQUENCE CHARACTERISTICS:
      (A) LENGTH: 31 nucleotides
      (B) TYPE: nucleic acid
      (C) STRANDEDNESS: single
      (D) TOPOLOGY: linear
   (ii) MOLECULE TYPE: DNA primer FL66
   (iii) HYPOTHETICAL: NO
   (iv) ANTI-SENSE: NO
   (xi) SEQUENCE DESCRIPTION: SEQ ID NO:30:
   CTATGCCATG GATAGATCGC TTTCTACTTC C 31
(2) INFORMATION FOR SEQ ID NO:31:
   (i) SEQUENCE CHARACTERISTICS:
      (A) LENGTH: 31 nucleotides
      (B) TYPE: nucleic acid
      (C) STRANDEDNESS: single
      (D) TOPOLOGY: linear
   (ii) MOLECULE TYPE: DNA primer FL67
   (iii) HYPOTHETICAL: NO
   (iv) ANTI-SENSE: NO
   (xi) SEQUENCE DESCRIPTION: SEQ ID NO:31:
   CAAGCCCATG GAAACTTACA AGGCTCAAAG A 31

## Description

### Background of the Invention

### Field of the Invention

The present invention relates to thermostable DNA polymerases which have been altered or mutated such that a different level of 5' to 3' exonuclease activity is exhibited from that which is exhibited by the native enzyme. The present invention also relates to means for isolating and producing such altered polymerases. Thermostable DNA polymerases are useful in many recombinant DNA techniques, especially nucleic acid amplification by the polymerase chain reaction (PCR) self-sustained sequence replication (3SR), and high temperature DNA sequencing.

### Background Art

Extensive research has been conducted on the isolation of DNA polymerases from mesophilic microorganisms such as E. coli. See, for example, Bessman et al., 1957, J. Biol. Chem. 223:171-177 and Buttin and Kornberg, 1966, J. Biol. Chem. 241:5419-5427.

Somewhat less investigation has been made on the isolation and purification of DNA polymerases from thermophiles such as Thermus aguaticus, Thermus thermophilus, Thermotoga maritima, Thermus species sps 17, Thermus species Z05 and Thermosipho africanus. The use of thermostable enzymes to amplify existing nucleic acid sequences in amounts that are large compared to the amount initially present was described in United States Patent Nos. 4,683,195 and 4,683,202, which describe the PCR process. Primers, template, nucleoside triphosphates, the appropriate buffer and reaction conditions, and polymerase are used in the PCR process, which involves denaturation of target DNA, hybridization of primers, and synthesis of complementary strands. The extension product of each primer becomes a template for the production of the 5 desired nucleic acid sequence. The two patents disclose that, if the polymerase employed is a thermostable enzyme, then polymerase need not be added after every denaturation step, because heat will not destroy the polymerase activity.

United States Patent No. 4,889,818, European Patent Publication No. 258,017 and PCT Publication No. 89/06691, all describe the isolation and recombinant expression of an ∼94 kDa thermostable DNA polymerase from Thermus aquaticus and the use of that polymerase in PCR. Although T. aquaticus DNA polymerase is especially preferred for use in PCR and other recombinant DNA techniques, there remains a need for other thermostable polymerases.
WO 91/02090 (Applicant: Promega Corp.), which is a prior right only, describes a thermostable DNA polymerase purified from *Thermus aquaticus.* This polymerase is a 80 or 85 kDa degradation product of the intact polymerase (molecular weight of 94 kDa) and is said to have substantially no 5'-3' exonuclease activity. No sequence data is provided on this polymerase.
WO 91/09950 (Applicant: Cetus Corp.), which is a prior right only, describes the purification of *Thermus thermophilus* polymerase and the recombinant expression of the gene encoding said polymerase in *E. coli* including Tth polymerase fragments lacking the amino terminal one third of the native enzyme.
WO 91/09944, which is a prior right only, relates to methods for the replication and amplification of RNA sequences by thermoactive DNA polymerases. On page 16, lines 14 and 15 it is suggested that changing Tth amino acid number 46 from glycine to aspartic acid may have an effect on the 5'-> 3' exonuclease activity, thus providing a novel enzyme.
Lawyer et al. have described in J. Biol. Chem. 264, 6427-6437 (1989) the isolation, characterization and expression of the DNA polymerase gene from *Thermus aquaticus* in *E. coli.* The cloning of expression vectors comprising a DNA sequence encoding a Taq polymerase fragment lacking the N-terminus of the native enzyme is also disclosed.
Leavitt et al. have described in Proc. Natl. Acad. Sci. USA 86, 4465-4468 (1989) the structural-functional relationship of T5 DNA polymerase to other DNA polymerases, such as T7 polymerase and *E.coli* polymerase I.
In Cell 59, 219-228 (1989) Bernad et al. have reported on a conserved 3 ' to 5' exonuclease active site in prokaryotic and eukaryotic DNA polymerases.
Kaledin et al. have reported in Chemical Abstract 93, No. 40169p (1989) on the purification of a thermostable DNA polymerase enzyme from *Thermus aquaticus* having a molecular weight of about 60 - 62'000 daltons. No sequence data is provided for this polymerase.
Chien et al. have reported in Chemical Abstract 85, No. 155559t (1976) on the purification of a thermostable DNA polymerase enzyme from *Thermus aquaticus .* The molecular weight of this enzyme is reported to be 68'000 as determined by sucrose gradient centrifugation and 63'000 as determined by gel filtration. No sequence data is provided for this polymerase.

### Summary of the Invention

In addressing the need for other thermostable polymerases, the present inventors found that some thermostable DNA polymerases such as that isolated from Thermus aquaticus (Taq) display a 5' to 3' exonuclease or structure-dependent single-stranded endonuclease (SDSSE) activity. As is explained in greater detail below, such 5' to 3' exonuclease activity is undesirable in an enzyme to be used in PCR, because it may limit the amount of product produced and contribute to the plateau phenomenon in the normally exponential accumulation of product. Furthermore, the presence of 5' to 3' nuclease activity in a thermostable DNA polymerase may contribute to an impaired ability to efficiently generate long PCR products greater than or equal to 10 kb particularly for G+C-rich targets. In DNA sequencing applications and cycle sequencing applitions, the presence of 5' to 3' nuclease activity may contribute to reduction in desired band intensities and/or generation of spurious or background bands. Finally, the absence of 5' to 3' nuclease activity may facilitate higher sensitivity allelic discrimination in a combined polymerase ligase chain reaction (PLCR) assay.

However, an enhanced or greater amount of 5' to 3' exonuclease activity in a thermostable DNA polymerase may be desirable in such an enzyme which is used in a homogeneous assay system for the concurrent amplification and detection of a target nucleic acid sequence. Generally, an enhanced 5' to 3' exonuclease activity is defined an enhanced rate of exonuclease cleavage or an enhanced rate of nick-translation synthesis or by the displacement of a larger nucleotide fragment before cleavage of the fragment.

Accordingly, the present invention was developed to meet the needs of the prior art by providing thermostable DNA polymerases which exhibit altered 5' to 3' exonuclease activity. Depending on the purpose for which the thermostable DNA polymerase will be used, the 5' to 3' exonuclease activity of the polymerase may be altered such that a range of 5' to 3' exonuclease activity may be expressed. This range of 5' to 3' exonuclease activity extends from an enhanced activity to a complete lack of activity. Although enhanced activity is useful in certain PCR applications, e. g. a homogeneous assay, as little 5' to 3' exonuclease activity as possible is desired in thermostable DNA polymerases utilized in most other PCR applications.

It was also found that both site directed mutagenesis as well as deletion mutagenesis may result in the desired altered 5' to 3' exonuclease activity in the thermostable DNA polymerases of the present invention. Some mutations which alter the exonuclease activity have been shown to alter the processivity of the DNA polymerase. In many applications (e.g. amplification of moderate sized targets in the presence of a large amount of high complexity genomic DNA) reduced processivity may simplify the optimization of PCRs and contribute to enhanced specificity at high enzyme concentration. Some mutations which eliminate 5' to 3' exonuclease activity do not reduce and may enhance the processivity of the thermostable DNA polymerase and accordingly, these mutant enzymes may be preferred in other applications (e.g. generation of long PCR products). Some mutations which eliminate the 5' to 3' exonuclease activity simultaneously enhance, relative to the wild type, the thermoresistance of the mutant thermostable polymerase, and thus, these mutant enzymes find additional utility in the amplification of G+C-rich or otherwise difficult to denature targets.

Particular common regions or domains of thermostable DNA polymerase genomes have been identified as preferred sites for mutagenesis to affect the enzyme's 5' to 3' exonuclease. These domains can be isolated and inserted into a thermostable DNA polymerase having none or little natural 5' to 3' exonuclease activity to enhance its activity. Thus, methods of preparing chimeric thermostable DNA polymerases with altered 5' to 3' exonuclease are also encompassed by the present invention.

### Detailed Description of the Invention

The present invention provides DNA sequences and expression vectors that encode thermostable DNA polymerases which have been mutated to alter the expression of 5' to 3' exonuclease. To facilitate understanding of the invention, a number of terms are defined below.

The terms "cell", "cell line", and "cell culture" can be used interchangeably and all such designations include progeny. Thus, the words "transformants" or "transformed cells" include the primary transformed cell and cultures derived from that cell without regard to the number of transfers. All progeny may not be precisely identical in DNA content, due to deliberate or inadvertent mutations. Mutant progeny that have the same functionality as screened for in the originally transformed cell are included in the definition of transformants.

The term "control sequences" refers to DNA sequences necessary for the expression of an operably linked coding sequence in a particular host organism. The control sequences that are suitable for procaryotes, for example, include a promoter, optionally an operator sequence, a ribosome binding site, and possibly other sequences. Eucaryotic cells are known to utilize promoters, polyadenylation signals, and enhancers.

The term "expression system" refers to DNA sequences containing a desired coding sequence and control sequences in operable linkage, so that hosts transformed with these sequences are capable of producing the encoded proteins. To effect transformation, the expression system may be included on a vector; however, the relevant DNA may also be integrated into the host chromosome.

The term "gene" refers to a DNA sequence that comprises control and coding sequences necessary for the production of a recoverable bioactive polypeptide or precursor. The polypeptide can be encoded by a full length coding sequence or by any portion of the coding sequence so long as the enzymatic activity is retained.

The term "operably linked" refers to the positioning of the coding sequence such that control sequences will function to drive expression of the protein encoded by the coding sequence. Thus, a coding sequence "operably linked" to control sequences refers to a configuration wherein the coding sequences can be expressed under the direction of a control sequence.

The term "mixture" as it relates to mixtures containing thermostable polymerases refers to a collection of materials which includes a desired thermostable polymerase but which can also include other proteins. If the desired thermostable polymerase is derived from recombinant host cells, the other proteins will ordinarily be those associated with the host. Where the host is bacterial, the contaminating proteins will, of course, be bacterial proteins.

The term "non-ionic polymeric detergents" refers to surface-active agents that have no ionic charge and that are characterized for purposes of this invention, by an ability to stabilize thermostable polymerase enzymes at a pH range of from about 3.5 to about 9.5, preferably from 4 to 8.5.

The term "oligonucleotide" as used herein is defined as a molecule comprised of two or more deoxyribonucleotides or ribonucleotides, preferably more than three, and usually more than ten. The exact size will depend on many factors, which in turn depends on the ultimate function or use of the oligonucleotide. The oligonucleotide may be derived synthetically or by cloning.

The term "primer" as used herein refers to an oligonucleotide which is capable of acting as a point of initiation of synthesis when placed under conditions in which primer extension is initiated. An oligonucleotide "primer" may occur naturally, as in a purified restriction digest or be produced synthetically. Synthesis of a primer extension product which is complementary to a nucleic acid strand is initiated in the presence of four different nucleoside triphosphates and a thermostable polymerase enzyme in an appropriate buffer at a suitable temperature. A "buffer" includes cofactors (such as divalent metal ions) and salt (to provide the appropriate ionic strength), adjusted to the desired pH.

A primer is single-stranded for maximum efficiency in amplification, but may alternatively be double-stranded. If double-stranded, the primer is first treated to separate its strands before being used to prepare extension products. The primer is usually an oligodeoxyribonucleotide. The primer must be sufficiently long to prime the synthesis of extension products in the presence of the polymerase enzyme. The exact length of a primer will depend on many factors, such as source of primer and result desired, and the reaction temperature must be adjusted depending on primer length and nucleotide sequence to ensure proper annealing of primer to template. Depending on the complexity of the target sequence, an oligonucleotide primer typically contains 15 to 35 nucleotides. Short primer molecules generally require lower temperatures to form sufficiently stable complexes with template.

A primer is selected to be "substantially" complementary to a strand of specific sequence of the template. A primer must be sufficiently complementary to hybridize with a template strand for primer elongation to occur. A primer sequence need not reflect the exact sequence of the template. For example, a non-complementary nucleotide fragment may be attached to the 5' end of the primer, with the remainder of the primer sequence being substantially complementary to the strand. Non-complementary bases or longer sequences can be interspersed into the primer, provided that the primer sequence has sufficient complementarity with the sequence of the template to hybridize and thereby form a template primer complex for synthesis of the extension product of the primer.

The terms "restriction endonucleases" and "restriction enzymes" refer to bacterial enzymes which cut double-stranded DNA at or near a specific nucleotide sequence.

The term "thermostable polymerase enzyme" refers to an enzyme which is stable to heat and is heat resistant and catalyzes (facilitates) combination of the nucleotides in the proper manner to form primer extension products that are complementary to a template nucleic acid strand. Generally, synthesis of a primer extension product begins at the 3' end of the primer and proceeds in the 5' direction along the template strand, until synthesis terminates.

In order to further facilitate understanding of the invention, specific thermostable DNA polymerase enzymes are referred to throughout the specification to exemplify the broad concepts of the invention, and these references are not intended to limit the scope of the invention. The specific enzymes which are frequently referenced are set forth below with a common abbreviation which will be used in the specification and their respective nucleotide and amino acid Sequence ID numbers.

| Thermostable DNA Polymerase | Common Abbr. | SEQ. ID NO: |
|---|---|---|
| Thermus aquaticus | Taq | SEQ ID NO:1 (nuc) |
| | | SEQ ID NO:2 (a.a.) |
| Thermotoga maritima | Tma | SEQ ID NO:3 (nuc) |
| | | SEQ ID NO:4 (a.a.) |
| Thermus species sps17 | Tsps17 | SEQ ID NO:5 (nuc) |
| | | SEQ ID NO:6 (a.a.) |
| Thermus species Z05 | TZ05 | SEQ ID NO:7 (nuc) |
| | | SEQ ID NO:8 (a.a.) |
| Thermus thermophilus | Tth | SEQ ID NO:9 (nuc) |
| | | SEQ ID NO:10 (a.a.) |
| Thermosipho africanus | Taf | SEQ ID No:11 (nuc) |
| | | SEQ ID NO:12 (a.a.) |

As summarized above, the present invention relates to thermostable DNA polymerases which exhibit altered 5' to 3' exonuclease activity from that of the native polymerase. Thus, the polymerases of the invention exhibit either an enhanced 5' to 3' exonuclease activity or an attenuated 5' to 3' exonuclease activity from that of the native polymerase.

### Thermostable DNA Polymerases With Attenuated 5' to 3' Exonuclease Activity

DNA polymerases often possess multiple functions. In addition to the polymerization of nucleotides E. coli DNA polymerase I (pol I), for example, catalyzes the pyrophosphorolysis of DNA as well as the hydrolysis of phosphodiester bonds. Two such hydrolytic activities have been characterized for pol I; one is a 3' to 5' exonuclease activity and the other a 5' to 3' exonuclease activity. The two exonuclease activities are associated with two different domains of the pol I molecule. However, the 5' to 3' exonuclease activity of pol I differs from that of thermostable DNA polymerases in that the 5' to 3' exonuclease activity of thermostable DNA polymerases has stricter structural requirements for the substrate on which it acts.

An appropriate and sensitive assay for the 5' to 3' exonuclease activity of thermostable DNA polymerases takes advantage of the discovery of the structural requirement of the activity. An important feature of the design of the assay is an upstream oligonucleoside primer which positions the polymerase appropriately for exonuclease cleavage of a labeled downstream oligonucleotide probe. For an assay of polymerization-independent exonuclease activity (i.e., an assay performed in the absence of deoxynucleoside triphosphates) the probe must be positioned such that the region of probe complementary to the template is immediately adjacent to the 3'-end of the primer. Additionally, the probe should contain at least one, but preferably 2-10, or most preferably 3-5 nucleotides at the 5'-end of the probe which are not complementary to the template. The combination of the primer and probe when annealed to the template creates a double stranded structure containing a nick with a 3'-hydroxyl 5' of the nick, and a displaced single strand 3' of the nick. Alternatively, the assay can be performed as a polymerization-dependent reaction, in which case each deoxynucleoside triphosphate should be included at a concentration of between 1 µM and 2 mM, preferably between 10 µM and 200 µM, although limited dNTP addition (and thus limited dNTP inclusion) may be involved as dictated by the template sequence. When the assay is performed in the presence of dNTPs, the necessary structural requirements are an upstream oligonucleotide primer to direct the synthesis of the complementary strand of the template by the polymerase, and a labeled downstream oligonucleotide probe which will be contacted by the polymerase in the process of extending the upstream primer. An example of a polymerization-independent thermostable DNA polymerase 5' to 3' exonuclease assay follows.

The synthetic 3' phosphorylated oligonucleotide probe (phosphorylated to preclude polymerase extension) BW33 (GATCGCTGCGCGTAACCACCACACCCGCCGCGCp) (SEQ ID NO:13) (100 pmol) was ³²P-labeled at the 5' end with gamma-[³²P] ATP (3000 Ci/mmol) and T4 polynucleotide kinase. The reaction mixture was extracted with phenol:chloroform:isoamyl alcohol, followed by ethanol precipitation. The ³²P-labeled oligonucleotide probe was redissolved in 100 µl of TE buffer, and unincorporated ATP was removed by gel filtration chromatography on a Sephadex G-50 spin column. Five pmol of ³²P-labeled BW33 probe, was annealed to 5 pmol of single-strand M13mp10w DNA, in the presence of 5 pmol of the synthetic oligonucleotide primer BW37 (GCGCTAGGGCGCTGGCAAGTGTAGCGGTCA) (SEQ ID NO:14) in a 100 µl reaction containing 10 mM Tris-HCl (pH 8.3), 50 mM KCl, and 3 mM MgCl₂. The annealing mixture was heated to 95°C for 5 minutes, cooled to 70°C over 10 minutes, incubated at 70°C for an additional 10 minutes, and then cooled to 25°C over a 30 minute period in a Perkin-Elmer Cetus DNA Thermal Cycler. Exonuclease reactions containing 10 µl of the annealing mixture were pre-incubated at 70°C for 1 minute. Thermostable DNA polymerase enzyme (approximately 0.01 to 1 unit of DNA polymerase activity, or 0.0005 to 0.05 pmol of enzyme) was added in a 2.5 µl volume to the pre-incubation reaction, and the reaction mixture was incubated at 70°C. Aliquots (5 µl) were removed after 1 minute and 5 minutes, and stopped by the addition of 1 µl of 60 mM EDTA. The reaction products were analyzed by homochromatography and exonuclease activity was quantified following autoradiography. Chromatography was carried out in a homochromatography mix containing 2% partially hydrolyzed yeast RNA in 7M urea on Polygram CEL 300 DEAE cellulose thin layer chromatography plates. The presence of 5' to 3' exonuclease activity results in the generation of small ³²P-labeled oligomers, which migrate up the TLC plate, and are easily differentiated on the autoradiogram from undegraded probe, which remains at the origin.

The 5' to 3' exonuclease activity of the thermostable DNA polymerases excises 5' terminal regions of double-stranded DNA releasing 5'-mono- and oligonucleotides in a sequential manner. The preferred substrate for the exonuclease is displaced single-stranded DNA, with hydrolysis of the phosphodiester bond occurring between the displaced single-stranded DNA and the double-helical DNA. The preferred exonuclease cleavage site is a phosphodiester bond in the double helical region. Thus, the exonuclease activity can be better described as a structure-dependent single-stranded endonuclease (SDSSE).

Many thermostable polymerases exhibit this 5' to 3' exonuclease activity, including the DNA polymerases of Taq, Tma, Tsps17, TZ05, Tth and Taf. When thermostable polymerases which have 5' to 3' exonuclease activity are utilized in the PCR process, a variety of undesirable results have been observed including a limitation of the amount of product produced, an impaired ability to generate long PCR products or amplify regions containing significant secondary structure, the production of shadow bands or the attenuation in signal strength of desired termination bands during DNA sequencing, the degradation of the 5'-end of oligonucleotide primers in the context of double-stranded primer-template complex, nick-translation synthesis during oligonucleotide-directed mutagenesis and the degradation of the RNA component of RNA:DNA hybrids.

The limitation of the amount of PCR product produced is attributable to a plateau phenomenon in the otherwise exponential accumulation of product. Such a plateau phenomenon occurs in part because 5' to 3' exonuclease activity causes the hydrolysis or cleavage of phosphodiester bonds when a polymerase with 5' to 3' exonuclease activity encounters a forked structure on a PCR substrate.

Such forked structures commonly exist in certain G-and C-rich DNA templates. The cleavage of these phosphodiester bonds under these circumstances is undesirable as it precludes the amplification of certain G- and C-rich targets by the PCR process. Furthermore, the phosphodiester bond cleavage also contributes to the plateau phenomenon in the generation of the later cycles of PCR when product strand concentration and renaturation kinetics result in forked structure substrates.

In the context of DNA sequencing, the 5' to 3' exonuclease activity of DNA polymerases is again a hinderance with forked structure templates because the phosphodiester bond cleavage during the DNA extension reactions results in "false stops". These "false stops" in turn contribute to shadow bands, and in extreme circumstances may result. in the absence of accurate and interpretable sequence data.

When utilized in a PCR process with double-stranded primer-template complex, the 5' to 3' exonuclease activity of a DNA polymerase may result in the degradation of the 5'-end of the oligonucleotide primers. This activity is not only undesirable in PCR, but also in second-strand cDNA synthesis and sequencing processes.

During optimally efficient oligonucleotide-directed mutagenesis processes, the DNA polymerase which is utilized must not have strand-displacement synthesis and/or nick-translation capability. Thus, the presence of 5' to 3' exonuclease activity in a polymerase used for oligonucleotide-directed mutagenesis is also undesirable.

Finally, the 5' to 3' exonuclease activity of polymerases generally also contains an inherent RNase H activity. However, when the polymerase is also to be used as a reverse transcriptase, as in a PCR process including an RNA:DNA hybrid, such an inherent RNase H activity may be disadvantageous.

Thus, one aspect of this invention involves the generation of thermostable DNA polymerase mutants displaying greatly reduced, attenuated or completely eliminated 5' to 3' exonuclease activity. Such mutant thermostable DNA polymerases will be more suitable and desirable for use in processes such as PCR, second-strand cDNA synthesis, sequencing and oligonucleotide-directed mutagenesis.

The production of thermostable DNA polymerase mutants with attenuated or eliminated 5' to 3' exonuclease activity may be accomplished by processes such as site-directed mutagenesis and deletion mutagenesis.

For example, a site-directed mutation of G to A in the second position of the codon for Gly at residue 46 in the Taq DNA polymerase amino acid sequence (i.e. mutation of G(137) to (A) in the DNA sequence has been found to result in an approximately 1000-fold reduction of 5' to 3' exonuclease activity with no apparent change in polymerase activity, processivity or extension rate. This site-directed mutation of the Taq DNA polymerase nucleotide sequence results in an amino acid change of Gly (46) to Asp.

Glycine 46 of Taq DNA polymerase is conserved in Thermus species spsl7 DNA polymerase, but is located at residue 43, and the same Gly to Asp mutation has a similar effect on the 5' to 3' exonuclease activity of Tsps17 DNA polymerase. Such a mutation of the conserved Gly of Tth (Gly 46), TZ05 (Gly 46), Tma (Gly 37) and Taf (Gly 37) DNA polymerases to Asp also has a similar attenuating effect on the 5' to 3' exonuclease activities of those polymerases.

Tsps17 Gly 43, Tth Gly 46, TZ05 Gly 46, Tma Gly 37 and Taf Gly 37 are also found in a conserved A(V/T)YG (SEQ ID NO:15) sequence domain, and changing the glycine to aspartic acid within this conserved sequence domain of any polymerase is also expected to attenuate 5' to 3' exonuclease activity. Specifically, Tsps17 Gly 43, Tth Gly 46, TZ05 Gly 46, and Taf Gly 37 share the AVYG sequence domain, and Tma Gly 37 is found in the ATYG domain. Mutations of glycine to aspartic acid in other thermostable DNA polymerases containing the conserved A(V/T)YG (SEQ ID NO:15) domain can be accomplished utilizing the same principles and techniques used for the site-directed mutagenesis of Taq polymerase. Exemplary of such site-directed mutagenesis techniques are Examples 5 and 8 of PCT Application No. 91/05753, filed August 13, 1991.

Such site-directed mutagenesis is generally accomplished by site-specific primer-directed mutagenesis. This technique is now standard in the art, and is conducted using a synthetic oligonucleotide primer complementary to a single-stranded phage DNA to be mutagenized except for limited mismatching, representing the desired mutation. Briefly, the synthetic oligonucleotide is used as a primer to direct synthesis of a strand complementary to the phasmid or phage, and the resulting double-stranded DNA is transformed into a phage-supporting host bacterium. Cultures of the transformed bacteria are plated in top agar, permitting plaque formation from single cells that harbor the phage or plated on drug selective media for phasmid vectors.

Theoretically, 50% of the new plaques will contain the phage having, as a single strand, the mutated form; 50% will have the original sequence. The plaques are tranferred to nitrocellulose filters and the "lifts" hybridized with kinased synthetic primer at a temperature that permits hybridization of an exact match, but at which the mismatches with the original strand are sufficient to prevent hybridization. Plaques that hybridize with the probe are then picked and cultured, and the DNA is recovered.

In the constructions set forth below, correct ligations for plasmid construction are confirmed by first transforming E. coli strains DG98, DG101, DG116, or other suitable hosts, with the ligation mixture. Successful transformants are selected by ampicillin, tetracycline or other antibiotic resistance or using other markers, depending on the mode of plasmid construction, as is understood in the art. Plasmids from the transformants are then prepared according to the method of Clewell, D.B., et al., Proc. Natl. Acad. Sci. (USA) (1969) 62:1159, optionally following chloramphenicol amplification (Clewell, D.B., J. Bacteriol. (1972) 110: 667). The isolated DNA is analyzed by restriction and/or sequenced by the dideoxy method of Sanger, F., et al., Proc. Natl. Acad. Sci. (USA) (1977) 74:5463 as further described by Messing, et al., Nucleic Acids Res. (1981) 9:309, or by the method of Maxam, et al., Methods in Enzymology (1980) 65:499.

For cloning and sequencing, and for expression of constructions under control of most lac or P_{L} promoters, E. coli strains DG98, DG101, DG116 were used as the host. For expression under control of the P_{L}N_{RBS} promoter, E. coli strain K12 MC1000 lambda lysogen, N₇N₅₃cI857 SusP₈₀, ATCC 39531 may be used. Exemplary hosts used herein for expression of the thermostable DNA polymerases with altered 5' to 3' exonuclease activity are E. coli DG116, which was deposited with ATCC (ATCC 53606) on April 7, 1987 and E. coli KB2, which was deposited with ATCC (ATCC 53075) on March 29, 1985.

For M13 phage recombinants, E. coli strains susceptible to phage infection, such as E. coli K12 strain DG98, are employed. The DG98 strain has been deposited with ATCC July 13, 1984 and has accession number 39768.

Mammalian expression can be accomplished in COS-7 COS-A2, CV-1, and murine cells, and insect cell-based expression in Spodoptera frugipeida.

The thermostable DNA polymerases of the present invention are generally purified from E. coli strain DG116 containing the features of plasmid pLSG33. The primary features are a temperature regulated promoter (λ P_{L} promoter), a temperature regulated plasmid vector, a positive retro-regulatory element (PRE) (see U.S. 4,666,848, issued May 19, 1987), and a modified form of a thermostable DNA polymerase gene. As described at page 46 of the specification of U.S patent application Serial No. 455,967, pLSG33 was prepared by ligating the NdeI-BamHI restriction fragment of pLSG24 into expression vector pDG178. The resulting plasmids are ampicillin resistant and capable of expressing 5' to 3' exonuclease deficient forms of the thermostable DNA polymerases of the present invention. The seed flask for a 10 liter fermentation contains tryptone (20 g/l), yeast extract (10 g/l), NaCl (10 g/l) and 0.005% ampicillin. The seed flask is inoculated from colonies from an agar plate, or a frozen glycerol culture stock can be used. The seed is grown to between 0.5 and 1.0 O.D. (A₆₈₀). The volume of seed culture inoculated into the fermentation is calculated such that the final concentration of bacteria will be 1 mg dry weight/liter. The 10 liter growth medium contained 25 mM KH₂PO₄, 10 mM (NH₄)₂ SO₄, 4 mM sodium citrate, 0.4 mM FeCl₂, 0.04 mM ZnCl₂, 0.03 mM CoCl₂, 0.03 mM CuCl₂, and 0.03 mM H₃BO₃. The following sterile components are added: 4 mM MgSO₄, 20 g/l glucose, 20 mg/l thiamine-HCl and 50 mg/l ampicillin. The pH was adjusted to 6.8 with NaOH and controlled during the fermentation by added NH₄OH. Glucose is continually added during the fermentation by coupling to NH₄OH addition. Foaming is controlled by the addition of polypropylene glycol as necessary, as an anti-foaming agent. Dissolved oxygen concentration is maintained at 40%.

The fermentation is inoculated as described above and the culture is grown at 30°C until an optical density of 21 (A₆₈₀) is reached. The temperature is then raised to 37°C to induce synthesis of the desired polymerase. Growth continues for eight hours after induction, and the cells are then harvested by concentration using cross flow filtration followed by centrifugation. The resulting cell paste is frozen at -70°C and yields about 500 grams of cell paste. Unless otherwise indicated, all purification steps are conducted at 4°C.

A portion of the frozen (-70°C) E. coli K12 strain DG116 harboring plasmid pLSG33 or other suitable host as described above is warmed overnight to -20°C. To the cell pellet the following reagents are added: 1 volume of 2X TE (100 mM Tris-HCl, pH 7.5, 20 mM EDTA), 1 mg/ml leupeptin and 144 mM PMSF (in dimethyl formamide). The final concentration of leupeptin was 1 µg/ml and for PMSF, 2.4 mM. Preferably, dithiothreitol (DTT) is included in TE to provide a final concentration of 1 mM DTT. The mixture is homogenized at low speed in a blender. All glassware is baked prior to use, and solutions used in the purification are autoclaved, if possible, prior to use. The cells are lysed by passage twice through a Microfluidizer at 10,000 psi.

The lysate is diluted with 1X TE containing 1 mM DTT to a final volume of 5.5X cell wet weight. Leupeptin is added to 1 µg/ml and PMSF is added to 2.4 mM. The final volume (Fraction I) is approximately 1540 ml.

Ammonium sulfate is gradually added to 0.2 M (26.4 g/l) and the lysate stirred. Upon addition of ammonium sulfate, a precipitate forms which is removed prior to the polyethylenimine (PEI) precipitation step, described below. The ammonium sulfate precipitate is removed by centrifugation of the suspension at 15,000 - 20,000 xg in a JA-14 rotor for 20 minutes. The supernatant is decanted and retained. The ammonium sulfate supernatant is then stirred on a heating plate until the supernatant reaches 75°C and then is placed in a 77°C bath and held there for 15 minutes with occasional stirring. The supernatant is then cooled in an ice bath to 20°C and a 10 ml aliquot is removed for PEI titration.

PEI titration and agarose gel electrophoresis are used to determine that 0.3% PEI (commercially available from BDH as PolyminP) precipitates ∼90% of the macromolecular DNA and RNA, i.e., no DNA band is visible on an ethidium bromide stained agarose gel after treatment with PEI. PEI is added slowly with stirring to 0.3% from a 10% stock solution. The PEI treated supernatant is centrifuged at 10,000 RPM (17,000 xg) for 20 minutes in a JA-14 rotor. The supernatant is decanted and retained. The volume (Fraction II) is approximately 1340 ml.

Fraction II is loaded onto a 2.6 x 13.3 cm (71 ml) phenyl sepharose CL-4B (Pharmacia-LKB) column following equilibration with 6 to 10 column volumes of TE containing 0.2 M ammonium sulfate. Fraction II is then loaded at a linear flow rate of 10 cm/hr. The flow rate is 0.9 ml/min. The column is washed with 3 column volumes of the equilibration buffer and then with 2 column volumes of TE to remove contaminating non-DNA polymerase proteins. The recombinant thermostable DNA polymerase is eluted with 4 column volumes of 2.5 M urea in TE containing 20% ethylene glycol. The DNA polymerase containing fractions are identified by optical absorption (A₂₈₀), DNA polymerase activity assay and SDS-PAGE according to standard procedures. Peak fractions are pooled and filtered through a 0.2 micron sterile vacuum filtration apparatus. The volume (Fraction III) is approximately 195 ml. The resin is equilibrated and recycled according to the manufacturer's recommendations.

A 2.6 x 1.75 cm (93 ml) heparin sepharose C1-6B column (Pharmacia-LKB) is equilibrated with 6-10 column volumes of 0.05 M KCl, 50 mM Tris-HCl, pH 7.5, 0.1 mM EDTA and 0.2% Tween 20 , at 1 column volume/hour. Preferably, the buffer contains 1 mM DTT. The column is washed with 3 column volumes of the equilibration buffer. The desired thermostable DNA polymerase of the invention is eluted with a 10 column volume linear gradient of 50-750 mM KCl gradient in the same buffer. Fractions (one-tenth column volume) are collected in sterile tubes and the fractions containing the desired thermostable DNA polymerase are pooled (Fraction IV, volume 177 ml).

Fraction IV is concentrated to 10 ml on an Amicon YM30 membrane. For buffer exchange, diafiltration is done 5 times with 2.5X storage buffer (50 mM Tris-HCl, pH 7.5, 250 mM KCl, 0.25 mM EDTA 2.5 mM DTT and 0.5% Tween-20 ) by filling the concentrator to 20 ml and concentrating the volumes to 10 ml each time. The concentrator is emptied and rinsed with 10 ml 2.5X storage buffer which is combined with the concentrate to provide Fraction V.

Anion exchange chromatography is used to remove residual DNA. The procedure is conducted in a biological safety hood and sterile techniques are used. A Waters Sep-Pak plus QMA cartridge with a 0.2 micron sterile disposable syringe tip filter unit is equilibrated with 30 ml of 2.5X storage buffer using a syringe at a rate of about 5 drops per second. Using a disposable syringe, Fraction V is passed through the cartridge at about 1 drop/second and collected in a sterile tube. The cartridge is flushed with 5 ml of 2.5 ml storage buffer and pushed dry with air. The eluant is diluted 1.5 X with 80% glycerol and stored at -20°C. The resulting final Fraction IV pool contains active thermostable DNA polymerase with altered 5' to 3' exonuclease activity.

In addition to site-directed mutagenesis of a nucleotide sequence, deletion mutagenesis techniques may also be used to attenuate the 5' to 3' exonuclease activity of a thermostable DNA polymerase. One example of such a deletion mutation is the deletion of all amino terminal amino acids up to and including the glycine in the conserved A(V/T)YG (SEQ ID NO:15) domain of thermostable DNA polymerases.

Those of skill in the art recognize that when such a deletion mutant is to be expressed in recombinant host cells, a methionine codon is usually placed at the 5' end of the coding sequence, so that the amino terminal sequence of the deletion mutant protein would be MET-ALA in the Thermus genus examples above.

The preferred techniques for performing deletion mutations involve utilization of known restriction sites on the nucleotide sequence of the thermostable DNA polymerase. Following identification of the particular amino acid or amino acids which are to be deleted, a restriction site is identified which when cleaved will cause the cleavage of the target DNA sequence at a position or slightly 3' distal to the position corresponding to the amino acid or domain to be deleted, but retains domains which code for other properties of the polymerase which are desired.

Alternatively, restriction sites on either side (5' or 3') of the sequence coding for the target amino acid or domain may be utilized to cleave the sequence. However, a ligation of the two desired portions of the sequence will then be necessary. This ligation may be performed using techniques which are standard in the art and exemplified in Example 7 of PCT Application No. 91/05753, filed August 13, 1991.

Another technique for achieving a deletion mutation of the thermostable DNA polymerase is by utilizing the PCR mutagenesis process. In this process, primers are prepared which incorporate a restriction site domain and optionally a methionine codon if such a codon is not already present. Thus, the product of the PCR with this primer may be digested with an appropriate restriction enzyme to remove the domain which codes for 5' to 3' exonuclease activity of the enzyme. Then, the two remaining sections of the product are ligated to form the coding sequence for a thermostable DNA polymerase lacking 5' to 3' exonuclease activity. Such coding sequences can be utilized as expression vectors in appropriate host cells to produce the desired thermostable DNA polymerase lacking 5' to 3' exonuclease activity.

In addition to the Taq DNA polymerase mutants with reduced 5' to 3' exonuclease activity, it has also been found that a truncated Tma DNA polymerase with reduced 5' to 3' exonuclease activity may be produced by recombinant techniques even when the complete coding sequence of the Tma DNA polymerase gene is present in an expression vector in E. coli. Such a truncated Tma DNA polymerase is formed by translation starting with the methionine codon at position 140. Furthermore, recombinant means may be used to produce a truncated polymerase corresponding to the protein produced by initiating translation at the methionine codon at position 284 of the Tma coding sequence.

The Tma DNA polymerase lacking amino acids 1 though 139 (about 86 kDa), and the Tma DNA polymerase lacking amino acids 1 through 283 (about 70 kDa) retain polymerase activity but have attenuated 5' to 3' exonuclease activity. An additional advantage of the 70 kDa Tma DNA polymerase is that it is significantly more thermostable than native Tma polymerase.

Thus, it has been found that the entire sequence of the intact Tma DNA polymerase I enzyme is not required for activity. Portions of the Tma DNA polymerase I coding sequence can be used in recombinant DNA techniques to produce a biologically active gene product with DNA polymerase activity.

Furthermore, the availability of DNA encoding the Tma DNA polymerase sequence provides the opportunity to modify the coding sequence so as to generate mutein (mutant protein) forms also having DNA polymerase activity but with attenuated 5' to 3' exonuclease activity. The amino(N)-terminal portion of the Tma DNA polymerase is not necessary for polymerase activity but rather encodes the 5' to 3' exonuclease activity of the protein.

Thus, using recombinant DNA methodology, one can delete approximately up to one-third of the N-terminal coding sequence of the Tma gene, clone, and express a gene product that is quite active in polymerase assays but, depending on the extent of the deletion, has no 5' to 3' exonuclease activity. Because certain N-terminal shortened forms of the polymerase are active, the gene constructs used for expression of these polymerases can include the corresponding shortened forms of the coding sequence.

In addition to the N-terminal deletions, individual amino acid residues in the peptide chain of Tma DNA polymerase or other thermostable DNA polymerases may be modified by oxidation, reduction, or other derivation, and the protein may be cleaved to obtain fragments that retain polymerase activity but have attenuated 5' to 3' exonuclease activity. Modifications to the primary structure of the Tma DNA polymerase coding sequence or the coding sequences of other thermostable DNA polymerases by deletion, addition, or alteration so as to change the amino acids incorporated into the thermostable DNA polymerase during translation of the mRNA produced from that coding sequence can be made without destroying the high temperature DNA polymerase activity of the protein.

Another technique for preparing thermostable DNA polymerases containing novel properties such as reduced or enhanced 5' to 3' exonuclease activity is a "domain shuffling" technique for the construction of "thermostable chimeric DNA polymerases". For example, substitution of the Tma DNA polymerase coding sequence comprising codons about 291 through about 484 for the Taq DNA polymerase I codons 289-422 would yield a novel thermostable DNA polymerase containing the 5' to 3' exonuclease domain of Taq DNA polymerase (1-289), the 3' to 5' exonuclease domain of Tma DNA polymerase (291-484), and the DNA polymerase domain of Taq DNA polymerase (423-832) . Alternatively, the 5' to 3' exonuclease domain and the 3' to 5' exonuclease domains of Tma DNA polymerase (ca. codons 1-484) may be fused to the DNA polymerase (dNTP binding and primer/template binding domains) portions of Taq DNA polymerase (ca. codons 423-832).

As is apparent, the donors and recipients for the creation of "thermostable chimeric DNA polymerase" by "domain shuffling" need not be limited to Taq and Tma DNA polymerases. Other thermostable polymerases provide analogous domains as Taq and Tma DNA polymerases. Furthermore, the 5' to 3' exonuclease domain may derive from a thermostable DNA polymerase with altered 5' to 3' nuclease activity. For example, the 1 to 289 5' to 3' nuclease domain of Taq DNA polymerase may derive from a Gly (46) to Asp mutant form of the Taq polymerase gene. Similarly, the 5' to 3' nuclease and 3' to 5' nuclease domains of Tma DNA polymerase may encode a 5' to 3' exonuclease deficient domain, and be retrieved as a Tma Gly (37) to Asp amino acid 1 to 484 encoding DNA fragment or alternatively a truncated Met 140 to amino acid 484 encoding DNA fragment.

While any of a variety of means may be used to generate chimeric DNA polymerase coding sequences (possessing novel properties), a preferred method employs "overlap" PCR. In this method, the intended junction sequence is designed into the PCR primers (at their 5'-ends). Following the initial amplification of the individual domains, the various products are diluted (ca. 100 to 1000-fold) and combined, denatured, annealed, extended, and then the final forward and reverse primers are added for an otherwise standard PCR.

Those of skill in the art recognize that the above thermostable DNA polymerases with attenuated 5' to 3' exonuclease activity are most easily constructed by recombinant DNA techniques. When one desires to produce one of the mutant enzymes of the present invention, with attenuated 5' to 3' exonuclease activity or a derivative or homologue of those enzymes, the production of a recombinant form of the enzyme typically involves the construction of an expression vector, the transformation of a host cell with the vector, and culture of the transformed host cell under conditions such that expression will occur.

To construct the expression vector, a DNA is obtained that encodes the mature (used here to include all chimeras or muteins) enzyme or a fusion of the mutant polymerase to an additional sequence that does not destroy activity or to an additional sequence cleavable under controlled conditions (such as treatment with peptidase) to give an active protein. The coding sequence is then placed in operable linkage with suitable control sequences in an expression vector. The vector can be designed to replicate autonomously in the host cell or to integrate into the chromosomal DNA of the host cell. The vector is used to transform a suitable host, and the transformed host is cultured under conditions suitable for expression of the recombinant polymerase.

Each of the foregoing steps can be done in a variety of ways. For example, the desired coding sequence may be obtained from genomic fragments and used directly in appropriate hosts. The construction for expression vectors operable in a variety of hosts is made using appropriate replicons and control sequences, as set forth generally below. Construction of suitable vectors containing the desired coding and control sequences employs standard ligation and restriction techniques that are well understood in the art. Isolated plasmids, DNA sequences, or synthesized oligonucleotides are cleaved, modified, and religated in the form desired. Suitable restriction sites can, if not normally available, be added to the ends of the coding sequence so as to facilitate construction of an expression vector, as exemplified below.

Site-specific DNA cleavage is performed by treating with suitable restriction enzyme (or enzymes) under conditions that are generally understood in the art and specified by the manufacturers of commercially available restriction enzymes. See, e.g., New England Biolabs, Product Catalog. In general, about 1 pg of plasmid or other DNA is cleaved by one unit of enzyme in about 20 µl of buffer solution; in the examples below, an excess of restriction enzyme is generally used to ensure complete digestion of the DNA. Incubation times of about one to two hours at about 37°C are typical, although variations can be tolerated. After each incubation, protein is removed by extraction with phenol and chloroform; this extraction can be followed by ether extraction and recovery of the DNA from aqueous fractions by precipitation with ethanol. If desired, size separation of the cleaved fragments may be performed by polyacrylamide gel or agarose gel electrophoresis using standard techniques. See, e.g., Methods in Enzymology, 1980, 65:499-560.

Restriction-cleaved fragments with single-strand "overhanging" termini can be made blunt-ended (double-strand ends) by treating with the large fragment of E. coli DNA polymerase I (Klenow) in the presence of the four deoxynucleoside triphosphates (dNTPs) using incubation times of about 15 to 25 minutes at 20°C to 25°C in 50 mM Tris-Cl pH 7.6, 50 mM NaCl, 10 mM MgCl₂, 10 mM DTT, and 5 to 10 µM dNTPs. The Klenow fragment fills in at 5' protruding ends, but chews back protruding 3' single strands, even though the four dNTPs are present. If desired, selective repair can be performed by supplying only one of the, or selected, dNTPs within the limitations dictated by the nature of the protruding ends. After treatment with Klenow, the mixture is extracted with phenol/chloroform and ethanol precipitated. Similar results can be achieved using S1 nuclease, because treatment under appropriate conditions with S1 nuclease results in hydrolysis of any single-stranded portion of a nucleic acid.

Synthetic oligonucleotides can be prepared using the triester method of Matteucci et al., 1981, J. Am. Chem. Soc. 103:3185-3191, or automated synthesis methods. Kinasing of single strands prior to annealing or for labeling is achieved using an excess, e.g., approximately 10 units, of polynucleotide kinase to 0.5 µM substrate in the presence of 50 mM Tris, pH 7.6, 10 mM MgCl₂, 5 mM dithiothreitol (DTT), and 1 to 2 µM ATP. If kinasing is for labeling of probe, the ATP will contain high specific activity γ-³²P.

Ligations are performed in 15-30 µl volumes under the following standard conditions and temperatures: 20 mM Tris-Cl, pH 7.5, 10 mM MgCl₂, 10 mM DTT, 33 µg/ml BSA, 10 mM-50 mM NaCl, and either 40 µM ATP and 0.01-0.02 (Weiss) units T4 DNA ligase at 0°C (for ligation of fragments with complementary single-stranded ends) or 1 mM ATP and 0.3-0.6 units T4 DNA ligase at 14°C (for "blunt end" ligation). Intermolecular ligations of fragments with complementary ends are usually performed at 33-100 µg/ml total DNA concentrations (5 to 100 nM total ends concentration). Intermolecular blunt end ligations (usually employing a 20 to 30 fold molar excess of linkers, optionally) are performed at 1 µM total ends concentration.

In vector construction, the vector fragment is commonly treated with bacterial or calf intestinal alkaline phosphatase (BAP or CIAP) to remove the 5' phosphate and prevent religation and reconstruction of the vector. BAP and CIAP digestion conditions are well known in the art, and published protocols usually accompany the commercially available BAP and CIAP enzymes. To recover the nucleic acid fragments, the preparation is extracted with phenol-chloroform and ethanol precipitated to remove the phosphatase and purify the DNA. Alternatively, religation of unwanted vector fragments can be prevented by restriction enzyme digestion before or after ligation, if appropriate restriction sites are available.

For portions of vectors or coding sequences that require sequence modifications, a variety of site-specific primer-directed mutagenesis methods are available. The polymerase chain reaction (PCR) can be used to perform site-specific mutagenesis. In another technique now standard in the art, a synthetic oligonucleotide encoding the desired mutation is used as a primer to direct synthesis of a complementary nucleic acid sequence of a single-stranded vector, such as pBS13+, that serves as a template for construction of the extension product of the mutagenizing primer. The mutagenized DNA is transformed into a host bacterium, and cultures of the transformed bacteria are plated and identified. The identification of modified vectors may involve transfer of the DNA of selected transformants to a nitrocellulose filter or other membrane and the "lifts" hybridized with kinased synthetic primer at a temperature that permits hybridization of an exact match to the modified sequence but prevents hybridization with the original strand. Transformants that contain DNA that hybridizes with the probe are then cultured and serve as a reservoir of the modified DNA.

In the constructions set forth below, correct ligations for plasmid construction are confirmed by first transforming E. coli strain DG101 or another suitable host with the ligation mixture. Successful transformants are selected by ampicillin, tetracycline or other antibiotic resistance or sensitivity or by using other markers, depending on the mode of plasmid construction, as is understood in the art. Plasmids from the transformants are then prepared according to the method of Clewell et al., 1969, Proc. Natl. Acad. Sci. USA 62:1159, optionally following chloramphenicol amplification (Clewell, 1972, J. Bacteriol. 110:667). Another method for obtaining plasmid DNA is described as the "Base-Acid" extraction method at page 11 of the Bethesda Research Laboratories publication Focus, volume 5, number 2, and very pure plasmid DNA can be obtained by replacing steps 12 through 17 of the protocol with CsCl/ethidium bromide ultracentrifugation of the DNA. The isolated DNA is analyzed by restriction enzyme digestion and/or sequenced by the dideoxy method of Sanger et al., 1977, Proc. Natl. Acad. Sci. USA 74:5463, as further described by Messing et al., 1981, Nuc. Acids Res. 9:309, or by the method of Maxam et al., 1980, Methods in Enzymology 65:499.

The control sequences, expression vectors, and transformation methods are dependent on the type of host cell used to express the gene. Generally, procaryotic, yeast, insect, or mammalian cells are used as hosts. Procaryotic hosts are in general the most efficient and convenient for the production of recombinant proteins and are therefore preferred for the expression of the thermostable DNA polymerases of the present invention.

The procaryote most frequently used to express recombinant proteins is E. coli. For cloning and sequencing, and for expression of constructions under control of most bacterial promoters, E. coli K12 strain MM294, obtained from the E. coli Genetic Stock Center under GCSC #6135, can be used as the host. For expression vectors with the P_{L}N_{RBS} control sequence, E. coli K12 strain MC1000 lambda lysogen, N₇N₅₃cI₈₅₇ SusP₈₀, ATCC 39531, may be used. E. coli DG116, which was deposited with the ATCC (ATCC 53606) on April 7, 1987, and E. coli KB2, which was deposited with the ATCC (ATCC 53075) on March 29, 1985, are also useful host cells. For M13 phage recombinants, E. coli strains susceptible to phage infection, such as E. coli K12 strain DG98, are employed. The DG98 strain was deposited with the ATCC (ATCC 39768) on July 13, 1984.

However, microbial strains other than E. coli can also be used, such as bacilli, for example Bacillus subtilis, various species of Pseudomonas, and other bacterial strains, for recombinant expression of the thermostable DNA polymerases of the present invention. In such procaryotic systems, plasmid vectors that contain replication sites and control sequences derived from the host or a species compatible with the host are typically used.

For example, E. coli is typically transformed using derivatives of pBR322, described by Bolivar et al., 1977, Gene 2:95. Plasmid pBR322 contains genes for ampicillin and tetracycline resistance. These drug resistance markers can be either retained or destroyed in constructing the desired vector and so help to detect the presence of a desired recombinant. Commonly used procaryotic control sequences, i.e., a promoter for transcription initiation, optionally with an operator, along with a ribosome binding site sequence, include the β-lactamase (penicillinase) and lactose (lac) promoter systems (Chang et al., 1977, Nature 198:1056), the tryptophan (trp) promoter system (Goeddel et al., 1980, Nuc. Acids Res. 8:4057), and the lambda-derived P_{L} promoter (Shimatake et al., 1981, Nature 292:128) and N-gene ribosome binding site (N_{RBS}). A portable control system cassette is set forth in United States Patent No. 4,711,845, issued December 8, 1987. This cassette comprises a P_{L} promoter operably linked to the N_{RBS} in turn positioned upstream of a third DNA sequence having at least one restriction site that permits cleavage within six bp 3' of the N_{RBS} sequence. Also useful is the phosphatase A (phoA) system described by Chang et al. in European Patent Publication No. 196,864, published October 8, 1986. However, any available promoter system compatible with procaryotes can be used to construct a modified thermostable DNA polymerase expression vector of the invention.

In addition to bacteria, eucaryotic microbes, such as yeast, can also be used as recombinant host cells. Laboratory strains of Saccharomyces cerevisiae, Baker's yeast, are most often used, although a number of other strains are commonly available. While vectors employing the two micron origin of replication are common (Broach, 1983, Meth. Enz. 101:307), other plasmid vectors suitable for yeast expression are known (see, for example, Stinchcomb et al., 1979, Nature 282:39; Tschempe et al., 1980, Gene 10:157; and Clarke et al., 1983, Meth. Enz. 101:300). Control sequences for yeast vectors include promoters for the synthesis of glycolytic enzymes (Hess et al., 1968, J. Adv. Enzyme Reg. 7:149; Holland et al., 1978, Biotechnology 17:4900; and Holland et al., 1981, J. Biol. Chem. 256:1385). Additional promoters known in the art include the promoter for 3-phosphoglycerate kinase (Hitzeman et al., 1980, J. Biol. Chem. 255:2073) and those for other glycolytic enzymes, such as glyceraldehyde 3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase. Other promoters that have the additional advantage of transcription controlled by growth conditions are the promoter regions for alcohol dehydrogenase 2, isocytochrome C, acid phosphatase, degradative enzymes associated with nitrogen metabolism, and enzymes responsible for maltose and galactose utilization (Holland, supra).

Terminator sequences may also be used to enhance expression when placed at the 3' end of the coding sequence. Such terminators are found in the 3' untranslated region following the coding sequences in yeast-derived genes. Any vector containing a yeast-compatible promoter, origin of replication, and other control sequences is suitable for use in constructing yeast expression vectors for the thermostable DNA polymerases of the present invention.

The nucleotide sequences which code for the thermostable DNA polymerases of the present invention can also be expressed in eucaryotic host cell cultures derived from multicellular organisms. See, for example, Tissue Culture, Academic Press, Cruz and Patterson, editors (1973). Useful host cell lines include COS-7, COS-A2, CV-1, murine cells such as murine myelomas N51 and VERO, HeLa cells, and Chinese hamster ovary (CHO) cells. Expression vectors for such cells ordinarily include promoters and control sequences compatible with mammalian cells such as, for example, the commonly used early and late promoters from Simian Virus 40 (SV 40) (Fiers et al., 1978, Nature 273:113), or other viral promoters such as those derived from polyoma, adenovirus 2, bovine papilloma virus (BPV), or avian sarcoma viruses, or immunoglobulin promoters and heat shock promoters. A system for expressing DNA in mammalian systems using a BPV vector system is disclosed in U.S. Patent No. 4,419,446. A modification of this system is described in U.S. Patent No. 4,601,978. General aspects of mammalian cell host system transformations have been described by Axel, U.S. Patent No. 4,399,216. "Enhancer" regions are also important in optimizing expression; these are, generally, sequences found upstream of the promoter region. Origins of replication may be obtained, if needed, from viral sources. However, integration into the chromosome is a common mechanism for DNA replication in eucaryotes.

Plant cells can also be used as hosts, and control sequences compatible with plant cells, such as the nopaline synthase promoter and polyadenylation signal sequences (Depicker et al., 1982, J. Mol. Appl. Gen. 1:561) are available. Expression systems employing insect cells utilizing the control systems provided by baculovirus vectors have also been described (Miller et al., 1986, Genetic Engineering (Setlow et al., eds., Plenum Publishing) 8:277-297). Insect cell-based expression can be accomplished in Spodoptera frugipeida. These systems can also be used to produce recombinant thermostable polymerases of the present invention.

Depending on the host cell used, transformation is done using standard techniques appropriate to such cells. The calcium treatment employing calcium chloride, as described by Cohen, 1972, Proc. Natl. Acad. Sci. USA 69:2110 is used for procaryotes or other cells that contain substantial cell wall barriers. Infection with Agrobacterium tumefaciens (Shaw et al., 1983, Gene 23:315) is used for certain plant cells. For mammalian cells, the calcium phosphate precipitation method of Graham and van der Eb, 1978, Virology 52:546 is preferred. Transformations into yeast are carried out according to the method of Van Solingen et al., 1977, J. Bact. 130:946 and Hsiao et al., 1979, Proc. Natl. Acad. Sci. USA 76:3829.

Once the desired thermostable DNA polymerase with altered 5' to 3' exonuclease activity has been expressed in a recombinant host cell, purification of the protein may be desired. Although a variety of purification procedures can be used to purify the recombinant thermostable polymerases of the invention, fewer steps may be necessary to yield an enzyme preparation of equal purity. Because E. coli host proteins are heat-sensitive, the recombinant thermostable DNA polymerases of the invention can be substantially enriched by heat inactivating the crude lysate. This step is done in the presence of a sufficient amount of salt (typically 0.2-0.3 M ammonium sulfate) to ensure dissociation of the thermostable DNA polymerase from the host DNA and to reduce ionic interactions of thermostable DNA polymerase with other cell lysate proteins.

In addition, the presence of 0.3 M ammonium sulfate promotes hydrophobic interaction with a phenyl sepharose column. Hydrophobic interaction chromatography is a separation technique in which substances are separated on the basis of differing strengths of hydrophobic interaction with an uncharged bed material containing hydrophobic groups. Typically, the column is first equilibrated under conditions favorable to hydrophobic binding, such as high ionic strength. A descending salt gradient may then be used to elute the sample.

According to the invention, an aqueous mixture (containing the recombinant thermostable DNA polymerase with altered 5' to 3' exonuclease activity) is loaded onto a column containing a relatively strong hydrophobic gel such as phenyl sepharose (manufactured by Pharmacia) or Phenyl TSK (manufactured by Toyo Soda). To promote hydrophobic interaction with a phenyl sepharose column, a solvent is used that contains, for example, greater than or equal to 0.3 M ammonium sulfate, with 0.3 M being preferred, or greater than or equal to 0.5 M NaCl. The column and the sample are adjusted to 0.3 M ammonium sulfate in 50 mM Tris (pH 7.5) and 1.0 mM EDTA ("TE") buffer that also contains 0.5 mM DTT, and the sample is applied to the column. The column is washed with the 0.3 M ammonium sulfate buffer. The enzyme may then be eluted with solvents that attenuate hydrophobic interactions, such as decreasing salt gradients, ethylene or propylene glycol, or urea.

For long-term stability, the thermostable DNA polymerase enzymes of the present invention can be stored in a buffer that contains one or more non-ionic polymeric detergents. Such detergents are generally those that have a molecular weight in the range of approximately 100 to 250,000 daltons, preferably about 4,000 to 200,000 daltons, and stabilize the enzyme at a pH of from about 3.5 to about 9.5, preferably from about 4 to 8.5. Examples of such detergents include those specified on pages 295-298 of McCutcheon's Emulsifiers & Detergents, North American edition (1983), published by the McCutcheon Division of MC Publishing Co., 175 Rock Road, Glen Rock, NJ (USA).

Preferably, the detergents are selected from the group comprising ethoxylated fatty alcohol ethers and lauryl ethers, ethoxylated alkyl phenols, octylphenoxy polyethoxy ethanol compounds, modified oxyethylated and/or oxypropylated straight-chain alcohols, polyethylene glycol monooleate compounds, polysorbate compounds, and phenolic fatty alcohol ethers. More particularly preferred are Tween 20, a polyoxyethylated (20) sorbitan monolaurate from ICI Americas Inc., Wilmington, DE, and Iconol NP-40, an ethoxylated alkyl phenol (nonyl) from BASF Wyandotte Corp., Parsippany, NJ.

The thermostable enzymes of this invention may be used for any purpose in which such enzyme activity is necessary or desired.

DNA sequencing by the Sanger dideoxynucleotide method (Sanger et al., 1977, Proc. Natl. Acad. Sci. USA 74:5463-5467) has undergone significant refinement in recent years, including the development of novel vectors (Yanisch-Perron et al., 1985, Gene 33:103-119), base analogs (Mills et al., 1979, Proc. Natl. Acad. Sci. USA 76:2232-2235, and Barr et al., 1986, BioTechniques 4:428-432), enzymes (Tabor et al., 1987, Proc. Natl. Acad. Sci. USA 84:4763-4771, and Innis, M.A. et al., 1988, Proc. Natl. Acad. Sci. USA 85:9436:9440), and instruments for partial automation of DNA sequence analysis (Smith et al., 1986, Nature 321:674-679; Prober et al., 1987, Science 238:336-341; and Ansorge et al., 1987, Nuc. Acids Res. 15:4593-4602). The basic dideoxy sequencing procedure involves (i) annealing an oligonucleotide primer to a suitable single or denatured double stranded DNA template; (ii) extending the primer with DNA polymerase in four separate reactions, each containing one α-labeled dNTP or ddNTP (alternatively, a labeled primer can be used), a mixture of unlabeled dNTPs, and one chain-terminating dideoxynucleotide-5'-triphosphate (ddNTP); (iii) resolving the four sets of reaction products on a high-resolution polyacrylamide-urea gel; and (iv) producing an autoradiographic image of the gel that can be examined to infer the DNA sequence. Alternatively, fluorescently labeled primers or nucleotides can be used to identify the reaction products. Known dideoxy sequencing methods utilize a DNA polymerase such as the Klenow fragment of E. coli DNA polymerase I, reverse transcriptase, Taq DNA polymerase, or a modified T7 DNA polymerase.

The introduction of commercial kits has vastly simplified the art, making DNA sequencing a routine technique for any laboratory. However, there is still a need in the art for sequencing protocols-that work well with nucleic acids that contain secondary structure such as palindromic hairpin loops and with G+C-rich DNA. Single stranded DNAs can form secondary structure, such as a hairpin loop, that can seriously interfere with a dideoxy sequencing protocol, both through improper termination in the extension reaction, or in the case of an enzyme with 5' to 3' exonuclease activity, cleavage of the template strand at the juncture of the hairpin. Since high temperature destabilizes secondary structure, the ability to conduct the extension reaction at a high temperature, i.e., 70-75°C, with a thermostable DNA polymerase results in a significant improvement in the sequencing of DNA that contains such secondary structure. However, temperatures compatible with polymerase extension do not eliminate all secondary structure. A 5' to 3' exonuclease-deficient thermostable DNA polymerase would be a further improvement in the art, since the polymerase could synthesize through the hairpin in a strand displacement reaction, rather than cleaving the template, resulting in an improper termination, i.e., an extension run-off fragment.

As an alternative to basic dideoxy sequencing, cycle dideoxy sequencing is a linear, asymmetric amplification of target sequences in the presence of dideoxy chain terminators. A single cycle produces a family of extension products of all possible lengths. Following denaturation of the extension reaction product from the DNA template, multiple cycles of primer annealing and primer extension occur in the presence of dideoxy terminators. The process is distinct from PCR in that only one primer is used, the growth of the sequencing reaction products in each cycle is linear, and the amplification products are heterogeneous in length and do not serve as template for the next reaction. Cycle dideoxy sequencing is a technique providing advantages for laboratories using automated DNA sequencing instruments and for other high volume sequencing laboratories. It is possible to directly sequence genomic DNA, without cloning, due to the specificity of the technique and the increased amount of signal generated. Cycle sequencing protocols accommodate single and double stranded templates, including genomic, cloned, and PCR-amplified templates.

Thermostable DNA polymerases have several advantages in cycle sequencing: they tolerate the stringent annealing temperatures which are required for specific hybridization of primer to genomic targets as well as tolerating the multiple cycles of high temperature denaturation which occur in each cycle. Performing the extension reaction at high temperatures, i.e., 70-75°C, results in a significant improvement in sequencing results with DNA that contains secondary structure, due to the destabilization of secondary structure. However, such temperatures will not eliminate all secondary structure. A 5' to 3' exonuclease-deficient thermostable DNA polymerase would be a further improvement in the art, since the polymerase could synthesize through the hairpin in a strand displacement reaction, rather than cleaving the template and creating an improper termination. Additionally, like PCR, cycle sequencing suffers from the phenomenon of product strand renaturation. In the case of a thermostable DNA polymerase possessing 5' to 3' exonuclease activity, extension of a primer into a double stranded region created by product strand renaturation will result in cleavage of the renatured complementary product strand. The cleaved strand will be shorter and thus appear as an improper termination. In addition, the correct, previously synthesized termination signal will be attenuated. A thermostable DNA polymerase deficient in 5' to 3' exonuclease activity will improve the art, in that such extension product fragments will not be formed. A variation of cycle sequencing, involves the simultaneous generation of sequencing ladders for each strand of a double stranded template while sustaining some degree of amplification (Ruano and Kidd, Proc. Natl. Acad. Sci. USA, 1991 88:2815-2819). This method of coupled amplification and sequencing would benefit in a similar fashion as stranded cycle sequencing from the use of a thermostable DNA polymerase deficient in 5' to 3' exonuclease activity.

In a particularly preferred embodiment, the enzymes in which the 5' to 3' exonuclease activity has been reduced or eliminated catalyze the nucleic acid amplification reaction known as PCR, and as stated above, with the resultant effect of producing a better yield of desired product than is achieved with the respective native enzymes which have greater amounts of the 5' to 3' exonuclease activity. Improved yields are the result of the inability to degrade previously synthesized product caused by 5' to 3' exonuclease activity. This process for amplifying nucleic acid sequences is disclosed and claimed in U.S. Patent Nos. 4,683,202 and 4,865,188. The PCR nucleic acid amplification method involves amplifying at least one specific nucleic acid sequence contained in a nucleic acid or a mixture of nucleic acids and in the most common embodiment, produces double-stranded DNA. Aside from improved yields, thermostable DNA polymerases with attenuated 5' to 3' exonuclease activity exhibit an improved ability to generate longer PCR products, an improved ability to produce products from G+C-rich templates and an improved ability to generate PCR products and DNA sequencing ladders from templates with a high degree of secondary structure.

For ease of discussion, the protocol set forth below assumes that the specific sequence to be amplified is contained in a double-stranded nucleic acid. However, the process is equally useful in amplifying single-stranded nucleic acid, such as mRNA, although in the preferred embodiment the ultimate product is still double-stranded DNA. In the amplification of a single-stranded nucleic acid, the first step involves the synthesis of a complementary strand (one of the two amplification primers can be used for this purpose), and the succeeding steps proceed as in the double-stranded amplification process described below.

This amplification process comprises the steps of:
(a) contacting each nucleic acid strand with four different nucleoside triphosphates and two oligonucleotide primers for each specific sequence being amplified, wherein each primer is selected to be substantially complementary to the different strands of the specific sequence, such that the extension product synthesized from one primer, when separated from its complement, can serve as a template for synthesis of the extension product of the other primer, said contacting being at a temperature that allows hybridization of each primer to a complementary nucleic acid strand;
(b) contacting each nucleic acid strand, at the same time as or after step (a), with a thermostable DNA polymerase of the present invention that enables combination of the nucleoside triphosphates to form primer extension products complementary to each strand of the specific nucleic acid sequence;
(c) maintaining the mixture from step (b) at an effective temperature for an effective time to promote the activity of the enzyme and to synthesize, for each different sequence being amplified, an extension product of each primer that is complementary to each nucleic acid strand template, but not so high as to separate each extension product from the complementary strand template;
(d) heating the mixture from step (c) for an effective time and at an effective temperature to separate the primer extension products from the templates on which they were synthesized to produce single-stranded molecules but not so high as to denature irreversibly the enzyme;
(e) cooling the mixture from step (d) for an effective time and to an effective temperature to promote hybridization of a primer to each of the single-stranded molecules produced in step (d); and
(f) maintaining the mixture from step (e) at an effective temperature for an effective time to promote the activity of the enzyme and to synthesize, for each different sequence being amplified, an extension product of each primer that is complementary to each nucleic acid template produced in step (d) but not so high as to separate each extension product from the complementary strand template. The effective times and temperatures in steps (e) and (f) may coincide, so that steps (e) and (f) can be carried out simultaneously. Steps (d)-(f) are repeated until the desired level of amplification is obtained.

The amplification method is useful not only for producing large amounts of a specific nucleic acid sequence of known sequence but also for producing nucleic acid sequences that are known to exist but are not completely specified. One need know only a sufficient number of bases at both ends of the sequence in sufficient detail so that two oligonucleotide primers can be prepared that will hybridize to different strands of the desired sequence at relative positions along the sequence such that an extension product synthesized from one primer, when separated from the template (complement), can serve as a template for extension of the other primer into a nucleic acid sequence of defined length. The greater the knowledge about the bases at both ends of the sequence, the greater can be the specificity of the primers for the target nucleic acid sequence and the efficiency of the process and specificity of the reaction.

In any case, an initial copy of the sequence to be amplified must be available, although the sequence need not be pure or a discrete molecule. In general, the amplification process involves a chain reaction for producing, in exponential quantities relative to the number of reaction steps involved, at least one specific nucleic acid sequence given that (a) the ends of the required sequence are known in sufficient detail that oligonucleotides can be synthesized that will hybridize to them and (b) that a small amount of the sequence is available to initiate the chain reaction. The product of the chain reaction will be a discrete nucleic acid duplex with termini corresponding to the 5' ends of the specific primers employed.

Any nucleic acid sequence, in purified or nonpurified form, can be utilized as the starting nucleic acid(s), provided it contains or is suspected to contain the specific nucleic acid sequence one desires to amplify. The nucleic acid to be amplified can be obtained from any source, for example, from plasmids such as pBR322, from cloned DNA or RNA, or from natural DNA or RNA from any source, including bacteria, yeast, viruses, organelles, and higher organisms such as plants and animals. DNA or RNA may be extracted from blood, tissue material such as chorionic villi, or amniotic cells by a variety of techniques. See, e.g., Maniatis et al., 1982, Molecular Cloning: A Laboratory Manual (Cold Spring Harbor Laboratory, Cold Spring Harbor, NY) pp. 280-281. Thus, the process may employ, for example, DNA or RNA, including messenger RNA, which DNA or RNA may be single-stranded or double-stranded. In addition, a DNA-RNA hybrid that contains one strand of each may be utilized. A mixture of any of these nucleic acids can also be employed as can nucleic acids produced from a previous amplification reaction (using the same or different primers). The specific nucleic acid sequence to be amplified can be only a fraction of a large molecule or can be present initially as a discrete molecule, so that the specific sequence constitutes the entire nucleic acid.

The sequence to be amplified need not be present initially in a pure form; the sequence can be a minor fraction of a complex mixture, such as a portion of the β-globin gene contained in whole human DNA (as exemplified in Saiki et al., 1985, Science 230:1530-1534) or a portion of a nucleic acid sequence due to a particular microorganism, which organism might constitute only a very minor fraction of a particular biological sample. The cells can be directly used in the amplification process after suspension in hypotonic buffer and heat treatment at about 90°C-100°C until cell lysis and dispersion of intracellular components occur (generally 1 to 15 minutes). After the heating step, the amplification reagents may be added directly to the lysed cells. The starting nucleic acid sequence can contain more than one desired specific nucleic acid sequence. The amplification process is useful not only for producing large amounts of one specific nucleic acid sequence but also for amplifying simultaneously more than one different specific nucleic acid sequence located on the same or different nucleic acid molecules.

Primers play a key role in the PCR process. The word "primer" as used in describing the amplification process can refer to more than one primer, particularly in the case where there is some ambiguity in the information regarding the terminal sequence(s) of the fragment to be amplified or where one employs the degenerate primer process described in PCT Application No. 91/05753, filed August 13, 1991. For instance, in the case where a nucleic acid sequence is inferred from protein sequence information, a collection of primers containing sequences representing all possible codon variations based on degeneracy of the genetic code can be used for each strand. One primer from this collection will be sufficiently homologous with a portion of the desired sequence to be amplified so as to be useful for amplification.

In addition, more than one specific nucleic acid sequence can be amplified from the first nucleic acid or mixture of nucleic acids, so long as the appropriate number of different oligonucleotide primers are utilized. For example, if two different specific nucleic acid sequences are to be produced, four primers are utilized. Two of the primers are specific for one of the specific nucleic acid sequences, and the other two primers are specific for the second specific nucleic acid sequence. In this manner, each of the two different specific sequences can be produced exponentially by the present process.

A sequence within a given sequence can be amplified after a given number of amplification cycles to obtain greater specificity in the reaction by adding, after at least one cycle of amplification, a set of primers that are complementary to internal sequences (i.e., sequences that are not on the ends) of the sequence to be amplified. Such primers can be added at any stage and will provide a shorter amplified fragment. Alternatively, a longer fragment can be prepared by using primers with non-complementary ends but having some overlap with the primers previously utilized in the amplification.

Primers also play a key role when the amplification process is used for in vitro mutagenesis. The product of an amplification reaction where the primers employed are not exactly complementary to the original template will contain the sequence of the primer rather than the template, so introducing an in vitro mutation. In further cycles, this mutation will be amplified with an undiminished efficiency because no further mispaired priming is required. The process of making an altered DNA sequence as described above could be repeated on the altered DNA using different primers to induce further sequence changes. In this way, a series of mutated sequences can gradually be produced wherein each new addition to the series differs from the last in a minor way, but from the original DNA source sequence in an increasingly major way.

Because the primer can contain as part of its sequence a non-complementary sequence, provided that a sufficient amount of the primer contains a sequence that is complementary to the strand to be amplified, many other advantages can be realized. For example, a nucleotide sequence that is not complementary to the template sequence (such as, e.g., a promoter, linker, coding sequence, etc.) may be attached at the 5' end of one or both of the primers and so appended to the product of the amplification process. After the extension primer is added, sufficient cycles are run to achieve the desired amount of new template containing the non-complementary nucleotide insert. This allows production of large quantities of the combined fragments in a relatively short period of time (e.g., two hours or less) using a simple technique.

Oligonucleotide primers can be prepared using any suitable method, such as, for example, the phosphotriester and phosphodiester methods described above, or automated embodiments thereof. In one such automated embodiment, diethylphosphoramidites are used as starting materials and can be synthesized as described by Beaucage et al., 1981, Tetrahedron Letters 22:1859-1862. One method for synthesizing oligonucleotides on a modified solid support is described in U.S. Patent No. 4,458,066. One can also use a primer that has been isolated from a biological source (such as a restriction endonuclease digest).

No matter what primers are used, however, the reaction mixture must contain a template for PCR to occur, because the specific nucleic acid sequence is produced by using a nucleic acid containing that sequence as a template. The first step involves contacting each nucleic acid strand with four different nucleoside triphosphates and two oligonucleotide primers for each specific nucleic acid sequence being amplified or detected. If the nucleic acids to be amplified or detected are DNA, then the nucleoside triphosphates are usually dATP, dCTP, dGTP, and dTTP, although various nucleotide derivatives can also be used in the process. For example, when using PCR for the detection of a known sequence in a sample of unknown sequences, dTTP is often replaced by dUTP in order to reduce contamination between samples as taught in PCT Application No. 91/05210 filed July 23, 1991.

The concentration of nucleoside triphosphates can vary widely. Typically, the concentration is 50 to 200 µM in each dNTP in the buffer for amplification, and MgCl₂ is present in the buffer in an amount of 1 to 3 mM to activate the polymerase and increase the specificity of the reaction. However, dNTP concentrations of 1 to 20 pM may be preferred for some applications, such as DNA sequencing or generating radiolabeled probes at high specific activity.

The nucleic acid strands of the target nucleic acid serve as templates for the synthesis of additional nucleic acid strands, which are extension products of the primers. This synthesis can be performed using any suitable method, but generally occurs in a buffered aqueous solution, preferably at a pH of 7 to 9, most preferably about 8. To facilitate synthesis, a molar excess of the two oligonucleotide primers is added to the buffer containing the template strands. As a practical matter, the amount of primer added will generally be in molar excess over the amount of complementary strand (template) when the sequence to be amplified is contained in a mixture of complicated long-chain nucleic acid strands. A large molar excess is preferred to improve the efficiency of the process. Accordingly, primer:template ratios of at least 1000:1 or higher are generally employed for cloned DNA templates, and primer: template ratios of about 10⁸:1 or higher are generally employed for amplification from complex genomic samples.

The mixture of template, primers, and nucleoside triphosphates is then treated according to whether the nucleic acids being amplified or detected are double-or single-stranded. If the nucleic acids are single-stranded, then no denaturation step need be employed prior to the first extension cycle, and the reaction mixture is held at a temperature that promotes hybridization of the primer to its complementary target (template) sequence. Such temperature is generally from about 35°C to 65°C or more, preferably about 37°C to 60°C for an effective time, generally from a few seconds to five minutes, preferably from 30 seconds to one minute. A hybridization temperature of 35°C to 70°C may be used for 5' to 3' exonuclease mutant thermostable DNA polymerases. Primers that are 15 nucleotides or longer in length are used to increase the specificity of primer hybridization. Shorter primers require lower hybridization temperatures.

The complement to the original single-stranded nucleic acids can be synthesized by adding the thermostable DNA polymerase of the present invention in the presence of the appropriate buffer, dNTPs, and one or more oligonucleotide primers. If an appropriate single primer is added, the primer extension product will be complementary to the single-stranded nucleic acid and will be hybridized with the nucleic acid strand in a duplex of strands of equal or unequal length (depending on where the primer hybridizes to the template), which may then be separated into single strands as described above to produce two single, separated, complementary strands. A second primer would then be added so that subsequent cycles of primer extension would occur using both the original single-stranded nucleic acid and the extension product of the first primer as templates. Alternatively, two or more appropriate primers (one of which will prime synthesis using the extension product of the other primer as a template) can be added to the single-stranded nucleic acid and the reaction carried out.

If the nucleic acid contains two strands, as in the case of amplification of a double-stranded target or second-cycle amplification of a single-stranded target, the strands of nucleic acid must be separated before the primers are hybridized. This strand separation can be accomplished by any suitable denaturing method, including physical, chemical or enzymatic means. One preferred physical method of separating the strands of the nucleic acid involves heating the nucleic acid until complete (>99%) denaturation occurs. Typical heat denaturation involves temperatures ranging from about 80°C to 105°C for times generally ranging from about a few seconds to minutes, depending on the composition and size of the nucleic acid. Preferably, the effective denaturing temperature is 90°C-100°C for a few seconds to 1 minute. Strand separation may also be induced by an enzyme from the class of enzymes known as helicases or the enzyme RecA, which has helicase activity and in the presence of ATP is known to denature DNA. The reaction conditions suitable for separating the strands of nucleic acids with helicases are described by Kuhn Hoffmann-Berling, 1978, CSH-Quantitative Biology 43:63, and techniques for using RecA are reviewed in Radding, 1982, Ann. Rev. Genetics 16:405-437. The denaturation produces two separated complementary strands of equal or unequal length.

If the double-stranded nucleic acid is denatured by heat, the reaction mixture is allowed to cool to a temperature that promotes hybridization of each primer to the complementary target (template) sequence. This temperature is usually from about 35°C to 65°C or more, depending on reagents, preferably 37°C to 60°C. The hybridization temperature is maintained for an effective time, generally a few seconds to minutes, and preferably 10 seconds to 1 minute. In practical terms, the temperature is simply lowered from about 95°C to as low as 37°C, and hybridization occurs at a temperature within this range.

Whether the nucleic acid is single- or double-stranded, the thermostable DNA polymerase of the present invention can be added prior to or during the denaturation step or when the temperature is being reduced to or is in the range for promoting hybridization. Although the thermostability of the polymerases of the invention allows one to add such polymerases to the reaction mixture at any time, one can substantially inhibit non-specific amplification by adding the polymerase to the reaction mixture at a point in time when the mixture will not be cooled below the stringent hybridization temperature. After hybridization, the reaction mixture is then heated to or maintained at a temperature at which the activity of the enzyme is promoted or optimized, i.e., a temperature sufficient to increase the activity of the enzyme in facilitating synthesis of the primer extension products from the hybridized primer and template. The temperature must actually be sufficient to synthesize an extension product of each primer that is complementary to each nucleic acid template, but must not be so high as to denature each extension product from its complementary template (i.e., the temperature is generally less than about 80°C to 90°C).

Depending on the nucleic acid(s) employed, the typical temperature effective for this synthesis reaction generally ranges from about 40°C to 80°C, preferably 50°C to 75°C. The temperature more preferably ranges from about 65°C to 75°C for the thermostable DNA polymerases of the present invention. The period of time required for this synthesis may range from about 10 seconds to several minutes or more, depending mainly on the temperature, the length of the nucleic acid, the enzyme, and the complexity of the nucleic acid mixture. The extension time is usually about 30 seconds to a few minutes. If the nucleic acid is longer, a longer time period is generally required for complementary strand synthesis.

The newly synthesized strand and the complement nucleic acid strand form a double-stranded molecule that is used in the succeeding steps of the amplification process. In the next step, the strands of the double-stranded molecule are separated by heat denaturation at a temperature and for a time effective to denature the molecule, but not at a temperature and for a period so long that the thermostable enzyme is completely and irreversibly denatured or inactivated. After this denaturation of template, the temperature is decreased to a level that promotes hybridization of the primer to the complementary single-stranded molecule (template) produced from the previous step, as described above.

After this hybridization step, or concurrently with the hybridization step, the temperature is adjusted to a temperature that is effective to promote the activity of the thermostable enzyme to enable synthesis of a primer extension product using as a template both the newly synthesized and the original strands. The temperature again must not be so high as to separate (denature) the extension product from its template, as described above. Hybridization may occur during this step, so that the previous step of cooling after denaturation is not required. In such a case, using simultaneous steps, the preferred temperature range is 50°C to 70°C.

The heating and cooling steps involved in one cycle of strand separation, hybridization, and extension product synthesis can be repeated as many times as needed to produce the desired quantity of the specific nucleic acid sequence. The only limitation is the amount of the primers, thermostable enzyme, and nucleoside triphosphates present. Usually, from 15 to 30 cycles are completed. For diagnostic detection of amplified DNA, the number of cycles will depend on the nature of the sample, the initial target concentration in the sample and the sensitivity of the detection process used after amplification. For a given sensitivity of detection, fewer cycles will be required if the sample being amplified is pure and the initial target concentration is high. If the sample is a complex mixture of nucleic acids and the initial target concentration is low, more cycles will be required to amplify the signal sufficiently for detection. For general amplification and detection, the process is repeated about 15 times. When amplification is used to generate sequences to be detected with labeled sequence-specific probes and when human genomic DNA is the target of amplification, the process is repeated 15 to 30 times to amplify the sequence sufficiently so that a clearly detectable signal is produced, i.e., so that background noise does not interfere with detection.

No additional nucleotides, primers, or thermostable enzyme need be added after the initial addition, provided that no key reagent has been exhausted and that the enzyme has not become denatured or irreversibly inactivated, in which case additional polymerase or other reagent would have to be added for the reaction to continue. After the appropriate number of cycles has been completed to produce the desired amount of the specific nucleic acid sequence, the reaction can be halted in the usual manner, e.g., by inactivating the enzyme by adding EDTA, phenol, SDS, or CHCl₃ or by separating the components of the reaction.

The amplification process can be conducted continuously. In one embodiment of an automated process, the reaction mixture can be temperature cycled such that the temperature is programmed to be controlled at a certain level for a certain time. One such instrument for this purpose is the automated machine for handling the amplification reaction developed and marketed by Perkin-Elmer Cetus Instruments. Detailed instructions for carrying out PCR with the instrument are available upon purchase of the instrument.

The thermostable DNA polymerases of the present invention with altered 5' to 3' exonuclease activity are very useful in the diverse processes in which amplification of a nucleic acid sequence by PCR is useful. The amplification method may be utilized to clone a particular nucleic acid sequence for insertion into a suitable expression vector, as described in U.S. Patent No. 4,800,159. The vector may be used to transform an appropriate host organism to produce the gene product of the sequence by standard methods of recombinant DNA technology. Such cloning may involve direct ligation into a vector using blunt-end ligation, or use of restriction enzymes to cleave at sites contained within the primers. Other processes suitable for the thermostable DNA polymerases of the present invention include those described in U.S. Patent Nos. 4,683,195 and 4,683,202 and European Patent Publication Nos. 229,701; 237,362; and 258,017. In addition, the present enzyme is useful in asymmetric PCR (see Gyllensten and Erlich, 1988, Proc. Natl. Acad. Sci. USA 85:7652-7656); inverse PCR (Ochman et al., 1988, Genetics 120:621); and for DNA sequencing (see Innis et al., 1988, Proc. Natl. Acad. Sci. USA 85:9436-9440, and McConlogue et al., 1988, Nuc. Acids Res. 16(20):9869), random amplification of cDNA ends (RACE), random priming PCR which is used to amplify a series of DNA fragments, and PCR processes with single sided specificity such as anchor PCR and ligation-mediated anchor PCR as described by Loh, E. in METHODS: A Companion to Methods in Enzymology (1991) 2: pp. 11-19.

An additional process in which a 5' to 3' exonuclease deficient thermostable DNA polymerase would be useful is a process referred to as polymerase ligase chain reaction (PLCR). As its name suggests, this process combines features of PCR with features of ligase chain reaction (LCR).

PLCR was developed in part as a technique to increase the specificity of allele-specific PCR in which the low concentrations of dNTPs utilized (∼1 µM) limited the extent of amplification. In PLCR, DNA is denatured and four complementary, but not adjacent, oligonucleotide primers are added with dNTPs, a thermostable DNA polymerase and a thermostable ligase.

The primers anneal to target DNA in a non-adjacent fashion and the thermostable DNA polymerase causes the addition of appropriate dNTPs to the 3' end of the downstream primer to fill the gap between the non-adjacent primers and thus render the primers adjacent. The thermostable ligase will then ligate the two adjacent oligonucleotide primers.

However, the presence of 5' to 3' exonuclease activity in the thermostable DNA polymerase significantly decreases the probability of closing the gap between the two primers because such activity causes the excision of nucleotides or small oligonucleotides from the 5' end of the downstream primer thus preventing ligation of the primers. Therefore, a thermostable DNA polymerase with attenuated or eliminated 5' to 3' exonuclease activity would be particularly useful in PLCR.

Briefly, the thermostable DNA polymerases of the present invention which have been mutated to have reduced, attenuated or eliminated 5' to 3' exonuclease activity are useful for the same procedures and techniques as their respective non-mutated polymerases except for procedures and techniques which require 5' to 3' exonuclease activity such as the homogeneous assay technique discussed below. Moreover, the mutated DNA polymerases of the present invention will oftentimes result in more efficient performance of the procedures and techniques due to the reduction or elimination of the inherent 5' to 3' exonuclease activity.

Specific thermostable DNA polymerases with attenuated 5' to 3' exonuclease activity include the following mutated forms of Taq, Tma, Tsps17, TZ05, Tth and Taf DNA polymerases. In the table below, and throughout the specification, deletion mutations are inclusive of the numbered nucleotides or amino acids which define the deletion.

| DNA Polymerase | Mutation | Mutant Designation |
|---|---|---|
| Taq | G(137) to A in nucleotide SED ID NO:1 | pRDA3-2 |
| | | |
| | Gly (46) to Asp in amino acid SEQ ID NO:2 | ASP46 Taq |
| | | |
| | Deletion of nucleotides 4-228 of nucleotide SEQ ID NO:1 | pTAQd2-76 |
| | | |
| | Deletion of amino acids 2-76 of amino acid SEQ ID NO:2 | MET-ALA 77 Taq |
| | | |
| | Deletion of nucleotides 4-138 of nucleotide SEQ ID NO:1 | pTAQd2-46 |
| | | |
| | Deletion of amino acids 2-46 of amino acid SEQ ID NO:2 | MET-PHE 47 Taq |
| | | |
| | Deletion of nucleotides 4-462 of nucleotide SEQ ID NO:1 | pTAQd2-155 |
| | | |
| | Deletion of amino acids 2-154 of amino acid SEQ ID NO:2 | MET-VAL 155 Taq |
| | | |
| | Deletion of nucleotides 4-606 of nucleotide SEQ ID NO:1 | pTAQd2-202 |
| | | |
| | Deletion of amino acids 2-202 of amino acid SEQ ID NO:2 | MET-THR 203 Taq |
| | | |
| | Deletion of nucleotides 4-867 of nucleotide SEQ ID NO:1 | pLSG8 |
| | | |
| | Deletion of amino acids 2-289 of amino acid SEQ ID NO:2 | MET-SER 290 Taq (Stoffel fragment) |
| | | |
| Tma | G(110) to A in nucleotide SEQ ID NO:3 | |
| | | |
| | Gly (37) to Asp in amino acid SEQ ID NO:4 | ASP37 Tma |
| | | |
| | Deletion of nucleotides 4-131 of nucleotide SEQ ID NO:3 | pTMAd2-37 |
| | | |
| | Deletion of amino acids 2-37 of amino acid SEQ ID NO:4 | MET-VAL 38 Tma |
| | | |
| | Deletion of nucleotides 4-60 of nucleotide SEQ ID NO:3 | pTMAd2-20 |
| | | |
| | Deletion of amino acids 2-20 of amino acid SEQ ID NO:4 | MET-ASP 21 Tma |
| | | |
| | Deletion of nucleotides 4-219 of nucleotide SEQ ID NO:3 | pTMAd2-73 |
| | | |
| | Deletion of amino acids 2-73 amino acid SEQ ID NO: 4 | MET-GLU 74 Tma |
| | | |
| | Deletion of nucleotides 1-417 of nucleotide SEQ ID NO:3 | pTMA16 |
| | | |
| | Deletion of amino acids 1-139 of amino acid SEQ ID NO:4 | MET 140 Tma |
| | | |
| | Deletion of nucleotides 1-849 of nucleotide SEQ ID NO:3 | pTMA15 |
| | | |
| | Deletion of amino acids 1-283 of amino acid SEQ ID NO:4 | MET 284 Tma |
| | | |
| Tsps17 | G(128) to A in nucleotide SEQ ID NO:5 | |
| | | |
| | Gly (43) to Asp in amino acid SEQ ID NO:6 | ASP43 Tsps17 |
| | | |
| | Deletion of nucleotides 4-129 of nucleotide SEQ ID NO:5 | pSPSd2-43 |
| | | |
| | Deletion of amino acids 2-43 of amino acid SEQ ID NO:6 | MET-PHE 44 Tsps17 |
| | | |
| | Deletion of nucleotides 4-219 of nucleotide SEQ ID NO:5 | pSPSd2-73 |
| | | |
| | Deletion of amino acids 2-73 of amino acid SEQ ID NO:6 | MET-ALA 74 Tsps17 |
| | | |
| | Deletion of nucleotides 4-453 of nucleotide SEQ ID NO:5 | pSPSd2-151 |
| | | |
| | Deletion of amino acids 2-151 of amino acid SEQ ID NO:6 | MET-LEU 152 Tsps17 |
| | | |
| | Deletion of nucleotides 4-597 of nucleotide SEQ ID NO:5 | pSPSd2-199 |
| | | |
| | Deletion of amino acids 2-199 of amino acid SEQ ID NO:6 | MET-THR 200 Tsps17 |
| | | |
| | Deletion of nucleotides 4-861 of nucleotide SEQ ID NO:5 | pSPSA288 |
| | | |
| | Deletion of amino acids 2-287 of amino acid SEQ ID NO:6 | MET-ALA 288 Tsps 17 |
| | | |
| TZ05 | G(137) to A in nucleotide SEQ ID NO:7 | |
| | | |
| | Gly (46) to Asp in amino acid SEQ ID NO:8 | ASP46 TZ05 |
| | | |
| | Deletion of nucleotides 4-138 of nucleotide SEQ ID NO:7 | pZ05d2-46 |
| | | |
| | Deletion of amino acids 2-46 of amino acid SEQ ID NO:8 | MET-PHE 47 TZ05 |
| | | |
| | Deletion of nucleotides 4-231 of nucleotide SEQ ID NO:7 | pZ05d2-77 |
| | | |
| | Deletion of amino acids 2-77 of amino acid SEQ ID NO:8 | MET-ALA 78 TZ05 |
| | | |
| | Deletion of nucleotides 4-475 of nucleotide SEQ ID NO:7 | pZ05d2-155 |
| | | |
| | Deletion of amino acids 2-155 of amino acid SEQ ID NO:8 | MET-VAL 156 TZ05 |
| | | |
| | Deletion of nucleotides 4-609 of nucleotide SEQ ID NO:7 | pZ05d2-203 |
| | | |
| | Deletion of amino acids 2-203 of amino acid SEQ ID NO:8 | MET-THR 204 TZ05 |
| | | |
| | Deletion of nucleotides 4-873 of nucleotide SEQ ID NO:7 | pZ05A292 |
| | | |
| | Deletion of amino acids 2-291 of amino acid SEQ ID NO:8 | MET-ALA 292 TZ05 |
| | | |
| Tth | G(137) to A in nucleotide SEQ ID NO:9 | |
| | | |
| | Gly (46) to Asp in amino acid SEQ ID NO:10 | ASP46 Tth |
| | | |
| | Deletion of nucleotides 4-138 of nucleotide SEQ ID NO:9 | pTTHd2-46 |
| | | |
| | Deletion of amino acids 2-46 of amino acid SEQ ID NO:10 | MET-PHE 47 Tth |
| | | |
| | Deletion of nucleotides 4-231 of nucleotide SEQ ID NO:9 | pTTHd2-77 |
| | | |
| | Deletion of amino acids 2-77 of amino acid SEQ ID NO:10 | MET-ALA 78 Tth |
| | | |
| | Deletion of nucleotides 4-465 of nucleotide SEQ ID NO:9 | pTTHd2-155 |
| | | |
| | Deletion of amino acids 2-155 of amino acid SEQ ID NO:10 | MET-VAL 156 Tth |
| | | |
| | Deletion of nucleotides 4-609 of nucleotide SEQ ID NO:9 | pTTHd2-203 |
| | | |
| | Deletion of amino acids 2-203 of amino acid SEQ ID NO:10 | MET-THR 204 Tth |
| | | |
| | Deletion of nucleotides 4-873 of nucleotide SEQ ID NO:9 | pTTHA292 |
| | | |
| | Deletion of amino acids 2-291 of amino acid SEQ ID NO:10 | MET-ALA 292 Tth |
| | | |
| Taf | G(110) to A and A(111) to T in nucleotide SEQ ID NO:11 | |
| | | |
| | Gly (37) to Asp in amino acid SEQ ID NO:12 | ASP37 Taf |
| | | |
| | Deletion of nucleotides 4-111 of nucleotide SEQ ID NO:11 | pTAFd2-37 |
| | | |
| | Deletion of amino acids 2-37 of amino acid SEQ ID NO:12 | MET-LEU 38 Taf |
| | | |
| | Deletion of nucleotides 4-279 of nucleotide SEQ ID NO:11 | pTAF09 |
| | | |
| | Deletion of amino acids 2-93 amino acid SEQ ID NO:12 | MET-TYR 94 Taf |
| | | |
| | Deletion of nucleotides 4-417 of nucleotide SEQ ID NO:11 | pTAF11 |
| | | |
| | Deletion of amino acids 2-139 of amino acid SEQ ID NO:12 | MET-GLU 140 Taf |
| | | |
| | Deletion of nucleotides 4-609 of nucleotide SEQ ID NO:11 | pTAFd2-203 |
| | | |
| | Deletion of amino acids 2-203 of amino acid SEQ ID NO:12 | MET-THR 204 Taf |
| | | |
| | Deletion of nucleotides 4-852 of nucleotide SEQ ID NO:11 | pTAFI285 |
| | | |
| | Deletion of amino acids 2-284 of amino acid SEQ ID NO:12 | MET-ILE 285 Taf |

### Example 1

### Preparation of a 5' to 3' Exonuclease Mutant of Taq DNA Polymerase by Random Mutagenesis PCR of the Known 5' to 3' Exonuclease Domain

### Preparation of Insert

Plasmid pLSG12 was used as a template for PCR. This plasmid is a HindIII minus version of pLSG5 in which the Taq polymerase gene nucleotides 616 - 621 of SEQ ID NO:1 were changed from AAGCTT to AAGCTG. This change eliminated the HindIII recognition sequence within the Taq polymerase gene without altering encoded protein sequence.

Using oligonucleotides MK61 (AGGACTACAACTGCCACACACC) (SEQ ID NO:21) and RA01 (CGAGGCGCGCCAGCCCCAGGAGATCTACCAGCTCCTTG) (SEQ ID NO:22) as primers and pLSG12 as the template, PCR was conducted to amplify a 384 bp fragment containing the ATG start of the Taq polymerase gene, as well as an additional 331 bp of coding sequence downstream of the ATG start codon.

A 100 µl PCR was conducted for 25 cycles utilizing the following amounts of the following agents and reactants:
50 pmol of primer MK61 (SEQ ID NO:21);
50 pmol of primer RA01 (SEQ ID NO:22);
50 µM of each dNTP;
10 mM Tris-HCl, pH 8.3;
50 mM KCl;
1.5 mM MgCl₂;
75.6 pg pLSG12;
2.5 units AmpliTaq DNA polymerase.

The PCR reaction mixture described was placed in a Perkin-Elmer Cetus Thermocycler and run through the following profile. The reaction mixture was first ramped up to 98°C over 1 minute and 45 seconds, and held at 98°C for 25 seconds. The reaction mixture was then ramped down to 55°C over 45 seconds and held at that temperature for 20 seconds. Finally, the mixture was ramped up to 72°C over 45 seconds, and held at 72°C for 30 seconds. A final 5 minute extension occurred at 72°C.

The PCR product was then extracted with chloroform and precipitated with isopropanol using techniques which are well known in the art.

A 300 ng sample of the PCR product was digested with 20 U of HindIII (in 30 µl reaction) for 2 hours at 37°C. Then, an additional digestion was made with 8 U of BssHII for an 2 hours at 50°C. This series of digestions yielded a 330 bp fragment for cloning.

A vector was prepared by digesting 5.3 pg of pLSG12 with 20 U HindIII (in 40 µl) for 2 hours at 37°C. This digestion was followed by addition of 12 U of BssHII and incubation for 2 hours at 50°C.

The vector was dephosphorylated by treatment with CIAP (calf intestinal alkaline phosphatase), specifically 0.04 U CIAP for 30 minutes at 30°C. Then, 4 µl of 500 mM EGTA was added to the vector preparation to stop the reaction, and the phosphatase was inactivated by incubation at 65°C for 45 minutes.

225 ng of the phosphatased vector described above was ligated at a 1:1 molar ratio with 10 ng of the PCR-derived insert.

Then, DG116 cells were transformed with one fifth of the ligation mixture, and ampicillin-resistant transformants were selected at 30°C.

Appropriate colonies were grown overnight at 30°C to OD₆₀₀ 0.7. Cells containing the P_{L} vectors were induced at 37°C in a shaking water bath for 4, 9, or 20 hours, and the preparations were sonicated and heat treated at 75°C in the presence of 0.2 M ammonium sulfate. Finally, the extracts were assayed for polymerase activity and 5' to 3' exonuclease activity.

The 5' to 3' exonuclease activity was quantified utilizing the 5' to 3' exonuclease assay described above. Specifically, the synthetic 3' phosphorylated oligonucleotide probe (phosphorylated to preclude polymerase extension) BW33 (GATCGCTGCGCGTAACCACCACACCCGCCGCGCp) (SEQ ID NO:13) (100 pmol) was ³²P-labeled at the 5' end with gamma-[³²P] ATP (3000 Ci/mmol) and T4 polynucleotide kinase. The reaction mixture was extracted with phenol:chloroform:isoamyl alcohol, followed by ethanol precipitation. The ³²P-labeled oligonucleotide probe was redissolved in 100 µl of TE buffer, and unincorporated ATP was removed by gel filtration chromatography on a Sephadex G-50 spin column. Five pmol of ³²P-labeled BW33 probe, was annealed to 5 pmol of single-strand M13mp10w DNA, in the presence of 5 pmol of the synthetic oligonucleotide primer BW37 (GCGCTAGGGCGCTGGCAAGTGTAGCGGTCA) (SEQ ID NO:14) in a 100 µl reaction containing 10 mM Tris-HCl (pH 8.3), 50 mM KCl, and 3 mM MgCl₂. The annealing mixture was heated to 95°C for 5 minutes, cooled to 70°C over 10 minutes, incubated at 70°C for an additional 10 minutes, and then cooled to 25°C over a 30 minute period in a Perkin-Elmer Cetus DNA thermal cycler. Exonuclease reactions containing 10 µl of the annealing mixture were pre-incubated at 70°C for 1 minute. The thermostable DNA polymerase preparations of the invention (approximately 0.3 U of enzyme activity) were added in a 2.5 µl volume to the pre-incubation reaction, and the reaction mixture was incubated at 70°C. Aliquots (5 µl) were removed after 1 minute and 5 minutes, and stopped by the addition of 1 µl of 60 mM EDTA. The reaction products were analyzed by homochromatography and exonuclease activity was quantified following autoradiography. Chromatography was carried out in a homochromatography mix containing 2% partially hydrolyzed yeast RNA in 7M urea on Polygram CEL 300 DEAE cellulose thin layer chromatography plates. The presence of 5' to 3' exonuclease activity resulted in the generation of small ³²P-labeled oligomers, which migrated up the TLC plate, and were easily differentiated on the autoradiogram from undegraded probe, which remained at the origin.

The clone 3-2 had an expected level of polymerase activity but barely detectable 5' to 3' exonuclease activity. This represented a greater than 1000-fold reduction in 5' to 3' exonuclease activity from that present in native Taq DNA polymerase.

This clone was then sequenced and it was found that G (137) was mutated to an A in the DNA sequence. This mutation results in a Gly (46) to Asp mutation in the amino acid sequence of the Taq DNA polymerase, thus yielding a thermostable DNA polymerase of the present invention with significantly attenuated 5' to 3' exonuclease activity.

The recovered protein was purified according to the Taq DNA polymerase protocol.

### Example 2

### Expression of Truncated Tma DNA Polymerases MET-ASP21 and MET-GLU74

To effect translation initiation at the aspartic acid codon at position 21 of the Tma DNA polymerase gene coding sequence, a methionine codon is introduced before the codon, and the region from the initial NcoI site to this introduced methionine codon is deleted. Similar to Example 4, the deletion process involved PCR with the same downstream primer described above (FL69) and an upstream primer (FL66) designed to incorporate an NcoI site and a methionine codon to yield a 570 base pair product.

The amplified product was concentrated with a Centricon-100 microconcentrator to eliminate excess primers and buffer. The product was concentrated in a Speed Vac concentrator and then resuspended in the digestion mix. The amplified product was digested with NcoI and XbaI. Likewise, pTmal2-1, pTma13, or pTmal9-RBS was digested with the same two restriction enzymes, and the digested, amplified fragment is ligated with the digested expression vector. The resulting construct has a deletion from the NcoI site upstream of the start codon of the native Tma coding sequence to the new methionine codon introduced upstream of the aspartic acid codon at position 21 of the native Tma coding sequence.

Similarly, a deletion mutant was created such that translation initiation begins at Glu74, the glutamic acid codon at position 74 of the native Tma coding sequence. An upstream primer (FL67) is designed to introduce a methionine codon and an NcoI site before Glu74. The downstream primer and cloning protocol used are as described above for the MET-ASP21 construct.

The upstream primer sequences used in this example are listed below and in the Sequence Listing section.

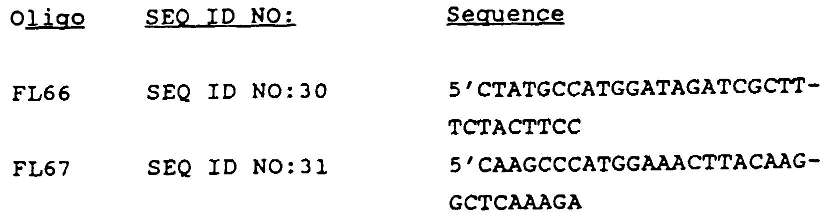

## Claims

1. A thermostable DNA polymerase mutant which has attenuated 5' to 3' exonuclease activity compared to that of its native thermostable DNA polymerase, which native thermostable DNA polymerase comprises the conserved amino acid sequence domain A(X)YG, wherein X is V or T (SEQ ID NO:15), characterized in that the glycine residue (G) in the conserved amino acid sequence A(X)YG of SEQ ID NO:15 is replaced by another amino acid residue.

2. The thermostable DNA polymerase mutant as claimed in claim 1, characterized in that the glycine residue (G) in the conserved amino acid sequence A(X)YG of SEQ ID NO:15 is replaced by the amino acid residue aspartic acid (D).

3. The thermostable DNA polymerase mutant as claimed in claim 1 or 2, wherein said naturally occurring thermostable DNA polymerase is a polymerase selected from the group consisting of Thermus aquaticus polymerase, Thermotoga maritima polymerase, Thermus thermophilus polymerase and Thermosipho africanus polymerase.

4. The thermostable DNA polymerase mutant having the sequence of amino acid residues 1-832 of SEQ ID NO:2, but wherein the glycine residue (G) at position 46 is replaced by another amino acid residue, preferably by the amino acid residue aspartic acid (D).

5. The thermostable DNA polymerase mutant having the sequence of amino acid residues 1-893 of SEQ ID NO:4, but wherein the glycine residue (G) at position 37 is replaced by another amino acid residue, preferably by the amino acid residue aspartic acid (D).

6. The thermostable DNA polymerase mutant having the sequence of amino acid residues 1-830 of SEQ ID NO:6, but wherein the glycine residue (G) at position 43 is replaced by another amino acid residue, preferably by the amino acid residue aspartic acid (D).

7. The thermostable DNA polymerase mutant having the sequence of amino acid residues 1-834 of SEQ ID NO:8, but wherein the glycine residue (G) at position 46 is replaced by another amino acid residue, preferably by the amino acid residue aspartic acid (D).

8. The thermostable DNA polymerase mutant having the sequence of amino acid residues 1-834 of SEQ ID NO:10, but wherein the glycine residue (G) at position 46 is replaced by another amino acid residue, preferably by the amino acid residue aspartic acid (D).

9. The thermostable DNA polymerase mutant having the sequence of amino acid residues 1-892 of SEQ ID NO:12, but wherein the glycine residue (G) at position 37 is replaced by another amino acid residue, preferably by the amino acid residue aspartic acid (D).

10. The thermostable DNA polymerase mutant as claimed in any one of claims 1 to 9, wherein additionally the 3' to 5' exonuclease domain or the polymerase (dNTP binding and primer/template binding) domain is substituted by the corresponding domain of another thermostable DNA polymerase.

11. A DNA encoding a thermostable DNA polymerase mutant as claimed in any one of claims 1 to 10.

12. The DNA as claimed in claim 11 which is obtained by site directed mutagenesis from a DNA encoding a naturally occurring thermostable DNA polymerase.

13. A recombinant DNA vector comprising a DNA as claimed in claim 11 or 12.

14. A recombinant host cell transformed with a recombinant DNA vector as claimed in claim 13.

15. A process for the preparation of a thermostable DNA polymerase mutant as claimed in any one of claims 1 to 10, which process comprises the steps of:
(a) culturing a host cell transformed with a recombinant DNA vector that comprises a DNA sequence encoding said thermostable DNA polymerase mutant; and
(b) isolating the thermostable DNA polymerase mutant produced in the host cell from the culture.

16. A thermostable DNA polymerase mutant prepared by a process as claimed in claim 15.

17. A thermostable DNA polymerase mutant as claimed in any one of claims 1 to 10 or 16, which is modified by oxidation or reduction.

18. A stabilized preparation comprising a thermostable DNA polymerase mutant as claimed in any one of claims 1 to 10 or 16, or a modified thermostable DNA polymerase mutant as claimed in claim 17, in a buffer containing one or more non-ionic polymeric detergents.

19. Use of a thermostable DNA polymerase mutant as claimed in any one of claims 1 to 10 or 16, or a modified thermostable DNA polymerase mutant as claimed in claim 17, or a stabilized preparation as claimed in claim 18 for amplifying or sequencing a nucleic acid.

20. A kit for DNA sequencing comprising a thermostable DNA polymerase mutant as claimed in any one of claims 1 to 10 or 16, or a modified thermostable DNA polymerase mutant as claimed in claim 17, or a stabilized preparation as claimed in claim 18 and one or more further reagents useful for sequencing a nucleic acid selected from the group of a primer, nucleotide triphosphate precursors (dNTPs) and a chain-terminating nucleotide triphosphate precursor, such as a dideoxynucleotide-5'-triphosphate (ddNTP).

21. A kit for amplifying a nucleic acid sequence comprising a thermostable DNA polymerase mutant as claimed in any one of claims 1 to 10 or 16, or a modified thermostable DNA polymerase mutant as claimed in claim 17, or a stabilized preparation as claimed in claim 18 and one or more further reagents useful for performing an amplification reaction selected from the group of oligonucleotide primers, a set of nucleoside triphosphate precursors and an oligonucleotide probe.

## Patentansprüche

1. Thermostabile DNA-Polymerase-Mutante, die eine abgeschwächte 5' nach 3'-Exonucleaseaktivität im Vergleich zu der Aktivität ihrer nativen thermostabilen DNA-Polymerase besitzt, wobei die native thermostabile DNA-Polymerase die konservierte Aminosäuresequenzdomäne A(X)YG besitzt, in der X der Rest V oder T ist (SEQ ID No. 15), dadurch gekennzeichnet, daß der Glycinrest (G) in der konservierten Aminosäuresequenz A(X)YG von SEQ ID No. 15 durch einen anderen Aminosäurerest ersetzt ist.

2. Thermostabile DNA-Polymerase-Mutante nach Anspruch 1, dadurch gekennzeichnet, daß der Glycinrest (G) in der konservierten Aminosäuresequenz A(X)YG von SEQ ID NO. 15 durch den Aminosäurerest Asparaginsäure (D) ersetzt ist.

3. Thermostabile DNA-Polymerase-Mutante nach Anspruch 1 oder 2, wobei die natürlich vorkommende thermostabile DNA-Polymerase eine Thermus aquaticus-Polymerase, Thermotoga maritima-Polymerase, Thermus thermophilus-Polymerase oder Thermosipho africanus-Polymerase ist.

4. Thermostabile DNA-Polymerase-Mutante mit der Sequenz der Aminosäurereste 1-832 von SEQ ID No. 2, worin jedoch der Glycinrest (G) an der Position 46 durch einen anderen Aminosäurerest ersetzt ist, vorzugsweise durch den Aminosäurerest Asparaginsäure (D).

5. Thermostabile DNA-Polymerase-Mutante mit der Sequenz der Aminosäurereste 1-893 von SEQ ID No. 4, worin jedoch der Glycinrest (G) an der Position 37 durch einen anderen Aminosäurerest ersetzt ist, vorzugsweise durch den Aminosäurerest Asparaginsäure (D).

6. Thermostabile DNA-Polymerase-Mutante mit der Sequenz der Aminosäurereste 1-830 von SEQ ID No. 6, worin jedoch der Glycinrest (G) an der Position 43 durch einen anderen Aminosäurerest ersetzt ist, vorzugsweise durch den Aminosäurerest Asparaginsäure (D).

7. Thermostabile DNA-Polymerase-Mutante mit der Sequenz der Aminosäurereste 1-834 von SEQ ID No. 8, worin jedoch der Glycinrest (G) an der Position 46 durch einen anderen Aminosäurerest ersetzt ist, vorzugsweise durch den Aminosäurerest Asparaginsäure (D).

8. Thermostabile DNA-Polymerase-Mutante mit der Sequenz der Aminosäurereste 1-834 von SEQ ID No. 10, worin jedoch der Glycinrest (G) an der Position 46 durch einen anderen Aminosäurerest ersetzt ist, vorzugsweise durch den Aminosäurerest Asparaginsäure (D).

9. Thermostabile DNA-Polymerase-Mutante mit der Sequenz der Aminosäurereste 1-892 von SEQ ID No. 12, worin jedoch der Glycinrest (G) an der Position 37 durch einen anderen Aminosäurerest ersetzt ist, vorzugsweise durch den Aminosäurerest Asparaginsäure (D).

10. Thermostabile DNA-Polymerase-Mutante nach einem der Ansprüche 1 bis 9, in der zusätzlich die 3' nach 5'-Exonucleasedomäne oder die Polymerase (dNTP-Bindungs- und Primer/Matrizenbindungs)-Domäne durch die entsprechende Domäne einer anderen thermostabilen DNA-Polymerase ersetzt ist.

11. DNA, die eine thermostabile DNA-Polymerase-Mutante nach einem der Ansprüche 1 bis 10 codiert.

12. DNA nach Anspruch 11, die durch ortsspezifische Mutagenese einer DNA erhalten wird, die eine natürlich vorkommende thermostabile DNA-Polymerase codiert.

13. Rekombinanter DNA-Vektor, der eine DNA nach Anspruch 11 oder 12 umfaßt.

14. Rekombinante Wirtszelle, die mit einem rekombinanten DNA-Vektor nach Anspruch 13 transformiert ist.

15. Verfahren zur Herstellung einer thermostabilen DNA-Polymerase-Mutante nach einem der Ansprüche 1 bis 10, wobei das Verfahren die folgenden Schritte umfaßt:
(a) Züchtung einer Wirtszelle, die mit einem rekombinanten DNA-Vektor transformiert ist, der eine die thermostabile DNA-Polymerase-Mutante codierende DNA-Sequenz umfaßt, und
(b) Isolierung der in der Wirtszelle produzierten thermostabilen DNA-Polymerase-Mutante aus der Kultur.

16. Thermostabile DNA-Polymerase-Mutante, die durch ein Verfahren nach Anspruch 15 hergestellt wird.

17. Thermostabile DNA-Polymerase-Mutante nach einem der Ansprüche 1 bis 10 oder 16, die durch Oxidation oder Reduktion modifiziert ist.

18. Stabilisiertes Präparat, das eine thermostabile DNA-Polymerase-Mutante nach einem der Ansprüche 1 bis 10 oder 16 oder eine modifizierte thermostabile DNA-Polymerase-Mutante nach Anspruch 17 in einem ein oder mehrere nichtionische polymere Detergentien enthaltenden Puffer umfaßt.

19. Verwendung einer thermostabilen DNA-Polymerase-Mutante nach einem der Ansprüche 1 bis 10 oder 16 oder einer modifizierten thermostabilen DNA-Polymerase-Mutante nach Anspruch 17 oder einem stabilisierten Präparat nach Anspruch 18 zur Amplifikation oder Sequenzierung einer Nucleinsäure.

20. Kit zur DNA-Sequenzierung, umfassend eine thermostabile DNA-Polymerase-Mutante nach einem der Ansprüche 1 bis 10 oder 16 oder eine modifizierte thermostabile DNA-Polymerase-Mutante nach Anspruch 17 oder ein stabilisiertes Präparat nach Anspruch 18 und ein oder mehrere weitere zur Sequenzierung einer Nucleinsäure nützliche Reagentien, ausgewählt aus einem Primer, Nucleotidtriphosphatvorläufern (dNTPs) und einem Nucleotidtriphosphatvorläufer zur Kettentermination, wie einem Didesoxynucleotid-5'-triphosphat (ddNTP).

21. Kit zur Amplifikation einer Nucleinsäuresequenz, umfassend eine thermostabile DNA-Polymerase-Mutante nach einem der Ansprüche 1 bis 10 oder 16 oder eine modifizierte thermostabile DNA-Polymerase-Mutante nach Anspruch 17 oder ein stabilisiertes Präparat nach Anspruch 18 und ein oder mehrere weitere zur Durchführung einer Amplifikationsreaktion nützliche Reagentien, ausgewählt aus Oligonucleotidprimern, einem Satz von Nucleosidtriphosphatvorläufern und einer Oligonucleotidsonde.

## Revendications

1. Mutant d'ADN-polymérase thermostable, qui présente une activité atténuée d'exonucléase 5'-3' par comparaison à celle de son ADN-polymérase thermostable native, laquelle ADN-polymérase thermostable native comprend le domaine conservé A(X)YG de la séquence d'acides aminés, où X est V ou T (SEQ ID N° 15), caractérisé en ce que le résidu de glycine (G) dans le domaine conservé A(X)YG de la séquence d'acides aminés SEQ ID N° 15 est remplacé par un autre résidu d'acide aminé.

2. Mutant d'ADN-polymérase thermostable selon la revendication 1, caractérisé en ce que le résidu de glycine (G) dans le domaine conservé A(X)YG de la séquence d'acides aminés SEQ ID N° 15 est remplacé par le résidu d'acide aminé acide aspartique (D).

3. Mutant d'ADN-polymérase thermostable selon la revendication 1 ou 2, dans lequel ladite ADN-polymérase thermostable naturelle est une polymérase choisie dans le groupe comprenant la polymérase de Thermus aquaticus, la polymérase de Thermotoga maritima, la polymérase de Thermus thermophilus et la polymérase de Thermosipho africanus.

4. Mutant d'ADN-polymérase thermostable ayant la séquence des résidus d'acides aminés 1-832 de la séquence SEQ ID N° 2, mais dans lequel le résidu de glycine (G) en position 46 est remplacé par un autre résidu d'acide aminé, de préférence par le résidu d'acide aminé acide aspartique (D).

5. Mutant d'ADN-polymérase thermostable ayant la séquence des résidus d'acides aminés 1-893 de la séquence SEQ ID N° 4, mais dans lequel le résidu de glycine (G) en position 37 est remplacé par un autre résidu d'acide aminé, de préférence par le résidu d'acide aminé acide aspartique (D).

6. Mutant d'ADN-polymérase thermostable ayant la séquence des résidus d'acides aminés 1-830 de la séquence SEQ ID N° 6, mais dans lequel le résidu de glycine (G) en position 43 est remplacé par un autre résidu d'acide aminé, de préférence par le résidu d'acide aminé acide aspartique (D).

7. Mutant d'ADN-polymérase thermostable ayant la séquence des résidus d'acides aminés 1-834 de la séquence SEQ ID N° 8, mais dans lequel le résidu de glycine (G) en position 46 est remplacé par un autre résidu d'acide aminé, de préférence par le résidu d'acide aminé acide aspartique (D).

8. Mutant d'ADN-polymérase thermostable ayant la séquence des résidus d'acides aminés 1-834 de la séquence SEQ ID N° 10, mais dans lequel le résidu de glycine (G) en position 46 est remplacé par un autre résidu d'acide aminé, de préférence par le résidu d'acide aminé acide aspartique (D).

9. Mutant d'ADN-polymérase thermostable ayant la séquence des résidus d'acides aminés 1-892 de la séquence SEQ ID N° 12, mais dans lequel le résidu de glycine (G) en position 37 est remplacé par un autre résidu d'acide aminé, de préférence par le résidu d'acide aminé acide aspartique (D).

10. Mutant d'ADN-polymérase thermostable selon l'une quelconque des revendications 1 à 9, dans lequel en outre le domaine exonucléase 3'-5' ou le domaine polymérase (liaison au dNTP ou liaison à l'amorce/matrice) est remplacé par le domaine correspondant d'une autre ADN-polymérase thermostable.

11. ADN codant un mutant d'ADN-polymérase thermostable selon l'une quelconque des revendications 1 à 10.

12. ADN selon la revendication 11, que l'on obtient par mutagenèse dirigée vers un site à partir d'un ADN codant une ADN-polymérase thermostable naturelle.

13. Vecteur à ADN recombinant comprenant un ADN selon la revendication 11 ou 12.

14. Cellule hôte recombinante transformée par un vecteur à ADN recombinant selon la revendication 13.

15. Procédé pour préparer un mutant d'ADN polymérase thermostable selon l'une quelconque des revendications 1 à 10, lequel procédé comprend les étapes :
(a) de culture d'une cellule hôte transformée par un vecteur à ADN recombinant, qui comprend une séquence d'ADN codant ledit mutant d'ADN-polymérase thermostable, et
(b) d'isolement, à partir de la culture, du mutant d'ADN-polymérase thermostable produit dans la cellule hôte.

16. Mutant d'ADN-polymérase thermostable préparé par un procédé selon la revendication 15.

17. Mutant d'ADN-polymérase thermostable selon l'une quelconque des revendications 1 à 10 ou 16, qui est modifié par oxydation ou par réduction.

18. Préparation stabilisée comprenant un mutant d'ADN-polymérase thermostable selon l'une quelconque des revendications 1 à 10 ou 16, ou un mutant d'ADN-polymérase thermostable modifié selon la revendication 17, dans un tampon contenant un ou plusieurs détergents polymères non-ioniques.

19. Utilisation d'un mutant d'ADN-polymérase thermostable selon l'une quelconque des revendications 1 à 10 ou 16, ou d'un mutant d'ADN-polymérase thermostable modifié selon la revendication 17, ou d'une préparation stabilisée selon la revendication 18, pour amplifier ou séquencer un acide nucléique.

20. Coffret pour le séquençage de l'ADN, qui comprend un mutant d'ADN-polymérase thermostable selon l'une quelconque des revendications 1 à 10 ou 16, ou un mutant d'ADN-polymérase thermostable modifié selon la revendication 17, ou une préparation stabilisée selon la revendication 18, et un ou plusieurs réactifs pouvant être utilisés pour séquencer un acide nucléique, choisis dans le groupe comprenant les amorces, les précurseurs de nucléotide-triphosphates (dNTP) et les précurseurs de nucléotide-triphosphates à terminaison de chaîne, tels qu'un didésoxynucléotide-5'-triphosphate (ddNTP).

21. Coffret pour amplifier une séquence d'acide nucléique, comprenant un mutant d'ADN-polymérase thermostable selon l'une quelconque des revendications 1 à 10 ou 16, ou un mutant d'ADN-polymérase thermostable modifié selon la revendication 17, ou une préparation stabilisée selon la revendication 18, et un ou plusieurs autres réactifs pouvant être utilisés pour mettre en oeuvre une réaction d'amplification, choisis dans le groupe comprenant les amorces oligonucléotidiques, un ensemble de précurseurs de nucléosides triphosphates, et les sondes oligonucléotidiques.
